(19) Europäisches Patentamt
European Patent Office
Office européen des brevets

(11) EP 4 692 347 A1

(12) **EUROPEAN PATENT APPLICATION**
published in accordance with Art. 153(4) EPC

(43) Date of publication:
**11.02.2026 Bulletin 2026/07**

(21) Application number: **24784438.4**

(22) Date of filing: **07.04.2024**

(51) International Patent Classification (IPC):
***C12N 15/11*** (2006.01) ***A61K 31/713*** (2006.01)

(52) Cooperative Patent Classification (CPC):
**A61K 31/713; C12N 15/11**

(86) International application number:
**PCT/CN2024/086382**

(87) International publication number:
**WO 2024/208357 (10.10.2024 Gazette 2024/41)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.04.2023 CN 202310369024**
**23.01.2024 CN 202410096499**

(71) Applicants:
- **Rona Bioscience, Limited**
  **Admiralty, Hong Kong (HK)**
- **Shanghai Ennovabio Pharmaceuticals Co., Ltd.**
  **Shanghai 201203 (CN)**

(72) Inventors:
- **HUANG, Jinyu**
  **Shanghai 201315 (CN)**
- **FANG, Jianwu**
  **Shanghai 201315 (CN)**
- **MENG, Guofeng**
  **Shanghai 201315 (CN)**
- **YUE, Ming**
  **Shanghai 201315 (CN)**

(74) Representative: **Cabinet Becker et Associés**
**25, rue Louis le Grand**
**75002 Paris (FR)**

Remarks:
The complete document including Reference Table(s) and the Sequence Listing(s) can be downloaded from the EPO website

# (54) DSRNA MOLECULE FOR REGULATING EXPRESSION OF CIDEB MRNA

(57) Provided are a double-stranded RNA for inhibiting expression of cell-death-inducing DFFA-like effector B (CIDEB) in cells, a cell comprising the double-stranded RNA, and a method of using the dsRNA or the cell to treat diseases or symptoms mediated by or associated with CIDEB expression in a subject.



Processed by Luminess, 75001 PARIS (FR)

## Description

### Technical Field

The present disclosure relates to the field of RNA interference.

### Background

Cell death-inducing DFFA-like effector B (CIDEB) is a member of the cell death-inducing DFFA-like effector (CIDE) protein family. CIDEB is highly expressed in the liver and kidney, with low expression in the small intestine, white adipose tissue, and colon (Li, J.Z. et al. 2007. Diabetes. 56: 2523-2532). CIDEB is located in lipid droplets and the smooth endoplasmic reticulum and is capable of directly interacting with apoB100 and apoB48 (Ye et al 2009 Cell Metab.9: 177-190). Overexpression of the CIDEB protein, as a member of the CIDE family, induces cell death.

The physiological function of CIDEB is associated with various lipid metabolism pathways, especially the VLDL pathway. For example, CIDEB mediates VLDL lipidation and maturation via interaction with ApoB. CIDEB is also required for the biosynthesis of VLDL transport vesicles as well as for the lipidation of small intestinal chylomicrons. The transcription of CIDEB is regulated by the transport of nuclear factors in stem cells. It is reported by Li et al. (Ye et al 2009 Cell Metab.9: 177-190; Li J.W. et al. 2010. Biochim. Biophys. Acta. 1801 : 577-586) that CideB knockout mice exhibit different levels of lipid metabolism than wild-type mice. In addition, previous studies have demonstrated that CIDEB is also required for the assembly of hepatitis C virus particles.

Non-alcoholic fatty liver disease (NAFLD)/nonalcoholic steatohepatitis (NASH) is a hepatic manifestation of metabolic syndrome. Changes in diet as well as lifestyle have led to the prevalence of obesity and metabolic syndrome in many countries, resulting in a significant increase in the incidence of NAFLD. NASH is the result of further development of simple fatty liver, with pathological manifestations of lipid deposition, inflammatory cell infiltration, necrosis of liver tissue, and fibrotic lesions, which further progress to a more severe cirrhosis and hepatocellular carcinoma (HCC). NAFLD not only affects the hepatobiliary system of patients, but is also closely related to insulin resistance, dyslipidemia, atherosclerosis, fat embolism, hematological diseases, etc. (Friedman SL et al., Nat Med, 2018, 24: 908-22). NAFLD has a high prevalence in developed countries and regions, approximately 15% to 40%, in which 10% to 20% of the NAFLD patients will progress to non-alcoholic steatohepatitis (NASH). It is estimated that the incidence of NASH worldwide is 5% to 7%, which rises to 22% in diabetic people, and about 15% to 25% of NASH patients will develop cirrhosis.

Accordingly, there is a need in the art for compositions and methods for treating diseases, disorders, and conditions associated with the activity of cell death-inducing DFFA-like effector B (CIDEB).

Reduction of CIDEB expression by double-stranded RNA (dsRNA), in particular small interfering RNA (siRNA) based on RNA interference mechanisms, represents a novel approach for treating diseases, disorders and conditions associated with the activity of cell death-inducing DFFA-like effector B (CIDEB).

### Summary of the Invention

The present disclosure provides a novel double strand RNA (or abbreviated as dsRNA) and a cell for inhibiting the expression of cell death-inducing DFFA-like effector B (CIDEB) in the cell, and a pharmaceutical composition and a kit comprising the same, and a method of inhibiting or reducing cell death-inducing DFFA-like effector B (CIDEB) gene expression or treating a disease or a disorder benefiting from reduced expression of cell death-inducing DFFA-like effector B (CIDEB) using the dsRNA, the cell, as well as the pharmaceutical composition and the kit.

In a first aspect, the present disclosure provides a double-stranded RNA (dsRNA) for inhibiting the expression of cell death-inducing DFFA-like effector B (CIDEB) in a cell, comprising a sense strand and an antisense strand forming a double-stranded region, wherein the sense strand and the antisense strand are each independently 15-30 nucleotides in length, and wherein the antisense strand comprises a nucleotide sequence of at least 15 contiguous nucleotides from the nucleotide sequence set forth in any one of SEQ ID NOs: 62-122. In some embodiments, the sense strand comprises a nucleotide sequence of at least 15 contiguous nucleotides from the nucleotide sequence set forth in any one of SEQ ID NOs: 1-61.

In some embodiments, the sense strand and the antisense strand are each independently 15-27 nucleotides in length, preferably 18-25 nucleotides, more preferably 19-21 nucleotides. In some embodiments, the sense strand is 15-27 nucleotides in length, preferably 17-25 nucleotides, more preferably 18-23 nucleotides, more preferably 19-21 nucleotides, and most preferably 19 amino acids. In some embodiments, the antisense strand is 15-27 nucleotides in length, preferably 17-25 nucleotides, more preferably 18-23 nucleotides, more preferably 19-22 nucleotides, and most preferably 21 amino acids.

In some embodiments, a hairpin loop is formed between the sense strand and the antisense strand in the dsRNA. In other embodiments, the dsRNA is an siRNA.

In some embodiments, the length of the double-stranded region is 15-25 nucleotide pairs, preferably 16-23 nucleotide pairs, more preferably 18-20 nucleotide pairs.

In some embodiments, one or both of the sense strand and the antisense strand comprise a 3' overhang and/or a 5' overhang having at least 1 nucleotide. For example, one or both of the sense strand and the antisense strand comprise a 3' overhang and/or a 5' overhang having at least 2 nucleotides. In some embodiments, the antisense strand has a 3' overhang and/or a 5' overhang of at least 1 nucleotide, and preferably the antisense strand comprises a 3' overhang and/or a 5' overhang of 2 nucleotides. In some specific embodiments, the dsRNA has a two-nucleotide overhang at the 3' end and a blunt end at the 5' end of the antisense strand.

In some embodiments, the antisense strand comprises a nucleotide sequence of at least 16 contiguous nucleotides, a nucleotide sequence of at least 17 contiguous nucleotides, a nucleotide sequence of at least 18 contiguous nucleotides, a nucleotide sequence of at least 19 contiguous nucleotides, or a nucleotide sequence of at least 20 contiguous nucleotides from the nucleotide sequence set forth in any one of SEQ ID NOs: 62-122, and preferably the antisense strand comprises the nucleotide sequence set forth in any one of SEQ ID NOs: 62-122. In some embodiments, the sense strand comprises a nucleotide sequence of at least 16 contiguous nucleotides, a nucleotide sequence of at least 17 contiguous nucleotides, or a nucleotide sequence of at least 18 contiguous nucleotides from the nucleotide sequence set forth in any one of SEQ ID NOs: 1-61, and preferably the sense strand comprises the nucleotide sequence set forth in any one of SEQ ID NOs: 1-61.

In some embodiments, the dsRNA comprises any pair of the paired sense strand sequences and antisense strand sequences as shown in Table 3.

In some embodiments, substantially all nucleotides of the sense strand and substantially all nucleotides of the antisense strand are modified nucleotides, or all nucleotides of the sense strand and all nucleotides of the antisense strand are modified nucleotides.

In some embodiments, the sense strand and the antisense strand each independently comprise one or more modified nucleotides selected from the group consisting of an SCP modified nucleotide, a 2'-O-alkyl modified nucleotide (e.g., a 2'-O-methyl modified nucleotide), a 2'-methoxyethyl modified nucleotide, a 2'-fluoro modified nucleotide, a 2'-deoxy modified nucleotide, an inosine ribonucleotide, an abasic nucleotide, an inverted abasic deoxyribonucleotide, a phosphorothioate internucleotide linkage modification, a vinylphosphonate modified nucleotide, a locked nucleotide, an unlocked nucleotide, a 2'-amino modified nucleotide, a 2'-C-alkyl modified nucleotide, a 2'-O-allyl modified nucleotide, a morpholino nucleotide, a phosphoramidate, a nucleotide comprising a non-natural base, a terminal nucleotide linked to a cholesteryl derivative or a dodecanoic acid bisdecylamide group, a deoxyribonucleotides, a 3'-terminal deoxythymine (dT) nucleotide, a conformationally restricted nucleotide, a constrained ethyl nucleotide, a 2'-hydroxy modified nucleotide, a nucleotide comprising methylphosphonate group, a nucleotide comprising 5'-phosphate, a nucleotide comprising 5'-phosphate mimic, a glycol modified nucleotide (GNA), and a 2-O-(N-methylacetamide) modified nucleotide. In some embodiments, the sense strand and the antisense strand each independently comprise one or more nucleotide modifications selected from the group consisting of a 2'-O-methyl modified nucleotide, a 2'-fluoro modified nucleotide, a 2'-deoxy modified nucleotide, an SCP modified nucleotide, and a phosphorothioate internucleotide linkage modification.

In some embodiments, the sense strand and/or the antisense strand comprises at least two 2'-fluoro modified nucleotides. In some embodiments, the sense strand and/or the antisense strand comprises at least eight 2'-O-methyl modified nucleotides. In some embodiments, the 3' end and/or 5' end of the sense strand and/or the antisense strand comprises 1-5 phosphorothioate internucleotide bondings, preferably 2-3 phosphorothioate internucleotide bondings.

In some embodiments, the 3' end and/or 5' end of the sense strand and/or the antisense strand comprises 1-5 phosphorothioate internucleotide bondings, preferably 2-3 phosphorothioate internucleotide bondings.

In some embodiments, the antisense strand of the dsRNA has a length of 21 nucleotides and has

(i) 2'-O-methyl modified nucleotides at positions 1, 3, 5, 7, 9, 11, 13, 15, 17, 19, and 21, and 2'-fluoro modified nucleotides at positions 2, 4, 6, 8, 10, 12, 14, 16, 18, and 20 (counting from the 5' end); and/or
(ii) phosphorothioate internucleotide bondings between nucleotide positions 1 and 2, between nucleotide positions 2 and 3, between nucleotide positions 19 and 20, and between nucleotide positions 20 and 21 (counting from the 5' end).

In some embodiments, the antisense strand of the dsRNA has a length of 21 nucleotides and has

(i) 2'-O-methyl modified nucleotides at positions 1, 3, 5, 7, 9, 11, 13, 15, 17, 19, 20, and 21, and 2'-fluoro modified nucleotides at positions 2, 4, 6, 8, 10, 12, 14, 16, and 18 (counting from the 5' end); and/or
(ii) phosphorothioate internucleotide bondings between nucleotide positions 1 and 2, between nucleotide positions 2 and 3, between nucleotide positions 19 and 20, and between nucleotide positions 20 and 21 (counting from the 5' end).

In some embodiments, the antisense strand of the dsRNA has a length of 21 nucleotides and has

(i) a 2'-deoxy modification at one or more nucleotide positions;
(ii) an SCP modification at one or more nucleotide positions;
(iii) a 2'-fluoro modification at one or more nucleotide positions;
(iv) a 2'-O-methyl modification at a position other than the position having the 2'-deoxy modification, SCP modification and 2'-fluoro modification described in (i), (ii) and (iii); and/or
(v) phosphorothioate internucleotide bondings between nucleotide positions 1 and 2, between nucleotide positions 2 and 3, between nucleotide positions 19 and 20, and between nucleotide positions 20 and 21 (counting from the 5' end).

In some embodiments, the antisense strand of the dsRNA has a length of 21 nucleotides and has

(i) 2'-deoxy modifications at positions 2, 5, 7, and 12 (counting from the 5' end);
(ii) an SCP modification at position 1 (counting from the 5' end);
(iii) a 2'-fluoro modification at position 14 (counting from the 5' end);
(iv) 2'-O-methyl modifications at positions 3, 4, 6, 8, 9, 10, 11, 13, 15, 16, 17, 18, 19, 20, and 21 (counting from the 5' end); and/or
(v) phosphorothioate internucleotide bondings between nucleotide positions 1 and 2, between nucleotide positions 2 and 3, between nucleotide positions 19 and 20, and between nucleotide positions 20 and 21 (counting from the 5' end).

In some embodiments, the antisense strand of the dsRNA has a length of 23 nucleotides and has

(i) an SCP modification at position 1 (counting from the 5' end);
(ii) 2'-fluoro modifications at positions 2, 4, 6, 8, 10, 12, 14, 16, and 18 (counting from the 5' end);
(iii) 2'-O-methyl modifications at positions 3, 5, 7, 9, 11, 13, 15, 17, 19, 20, 21, 22, and 23 (counting from the 5' end); and/or
(iv) phosphorothioate internucleotide bondings between nucleotide positions 1 and 2, between nucleotide positions 2 and 3, between nucleotide positions 21 and 22, and between nucleotide positions 22 and 23 (counting from the 5' end).

In some embodiments, the sense strand of the dsRNA has a length of 19 nucleotides and has:

(i) 2'-O-methyl modified nucleotides at positions 1 to 6, 10 to 19, and 2'-fluoro modified nucleotides at positions 7 to 9 (counting from the 5' end); and/or
(ii) phosphorothioate internucleotide bondings between nucleotide positions 1 and 2 and between nucleotide positions 2 and 3 (counting from the 5' end).

In some embodiments, the sense strand of the dsRNA has a length of 19 nucleotides and has:

(i) 2'-O-methyl modified nucleotides at positions 1 to 6, 10 to 19, and 2'-fluoro modified nucleotides at positions 7 to 9 (counting from the 5' end); and/or
(ii) phosphorothioate internucleotide bondings between nucleotide positions 1 and 2, between nucleotide positions 2 and 3, between positions 17 and 18, and between positions 18 and 19 (counting from the 5' end).

In some embodiments, the sense strand of the dsRNA has a length of 21 nucleotides and has

(i) 2'-fluoro modifications at positions 9, 10, and 11 (counting from the 5' end);
(ii) 2'-O-methyl modifications at positions 1, 2, 3, 4, 5, 6, 7, 8, 12, 13, 14, 15, 16, 17, 18, 19, 20, and 21 (counting from the 5' end); and/or
(iii) phosphorothioate internucleotide bondings between nucleotide positions 1 and 2, between nucleotide positions 2 and 3, and between nucleotide positions 20 and 21 (counting from the 5' end).

In some embodiments, the dsRNA is further conjugated to a ligand moiety comprising N-acetylgalactosamine, and preferably the sense strand of the dsRNA is conjugated to the ligand moiety. In some embodiments, the 3' end of the sense strand is conjugated to the ligand moiety. In other embodiments, the 5' end of the sense strand is conjugated to the ligand moiety. In some specific embodiments, the sense strand of the dsRNA has a phosphorothioate internucleotide bonding between nucleotide positions 1 and 2 (counting from the 3' end), and is conjugated at the 3' end of the sense strand to the ligand moiety via a phosphorothioate.
In some embodiments, the ligand moiety comprises a conjugation group of formula (X'):

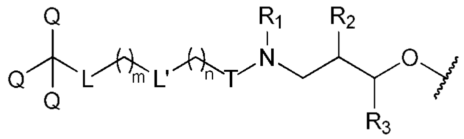

(X')

wherein,

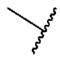

represents the point of attachment to the dsRNA;
Q is independently H,

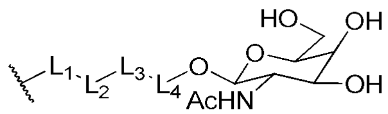

,

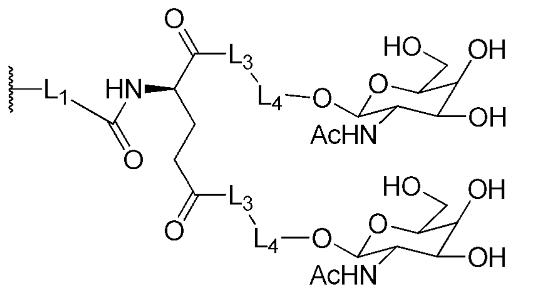

,

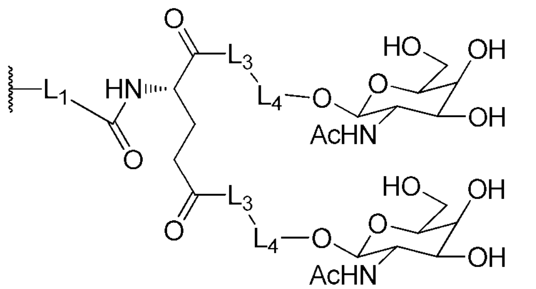

,

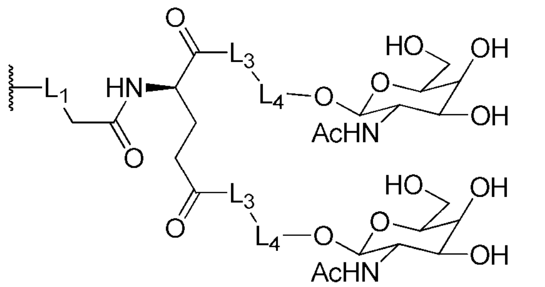

,

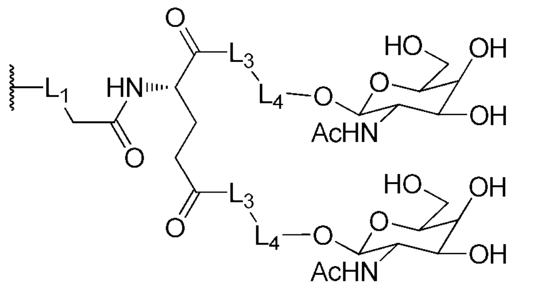

,

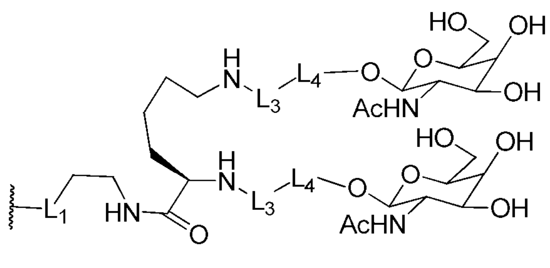

, or

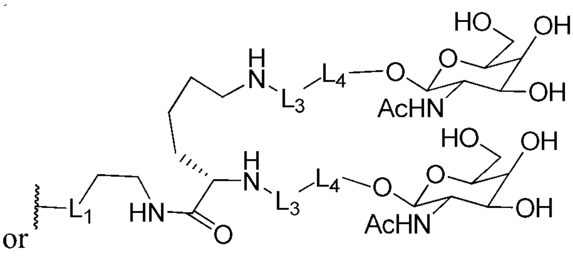

;

wherein $L_1$ is a bond, $-CH_2-$, $-CH_2CH_2-$, -C(O)-, $-CH_2O-$, $-CH_2O-CH_2CH_2O-$, or $-NHC(O)-(CH_2NHC(O))_a-$;
$L_2$ is a bond or $-CH_2CH_2C(O)-$;
$L_3$ is a bond, $-(NHCH_2CH_2)_b-$, $-(NHCH_2CH_2CH_2)_b-$, or $-C(O)CH_2-$;
$L_4$ is $-(OCH_2CH_2)_c-$, $-(OCH_2CH_2CH_2)_c-$, $-(OCH_2CH_2CH_2CH_2)_c-$, - $(OCH_2CH_2CH_2CH_2CH_2)_c-$, or $-NHC(O)-(CH_2)_d-$;
wherein a = 0, 1, 2, or 3;
b 1, 2, 3, 4 or 5;
c 1, 2, 3, 4 or 5;
d = 1, 2, 3, 4, 5, 6, 7, or 8;

L is a bond, $-CH_2O-$, or -NHC(O)-;
L' is a bond, -C(O)NH-, -NHC(O)-, or $-O(CH_2CH_2O)_e-$;
wherein e is 1, 2, 3, 4, or 5;
T is a bond, $-CH_2-$, -C(O)-, -M-, $-CH_2-M-$, or -C(O)-M-;
wherein M is

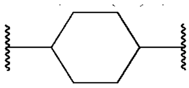, or 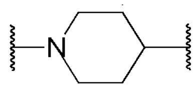;

$R_1$ and $R_2$ together form $-CH_2CH_2O-$ or $-CH_2CH(R)-O-$, and $R_3$ is H;
or $R_1$ and $R_3$ together form $-C_{1-2}$ alkylene-, and $R_2$ is H;
wherein R is -OR', $-CH_2OR'$, or $-CH_2CH_2OR'$; wherein R' is H, a hydroxy-protecting group, or a solid support, and the hydroxy-protecting group is preferably $-C(O)CH_2CH_2C(O)OH$ or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

In some embodiments, the conjugation ligand targets an asialoglycoprotein receptor (ASGPR).
In some preferred embodiments, the conjugation group is selected from Table 1:

Table 1. Structure of Conjugation Groups

| No. | Structure |
|---|---|
| 1 | 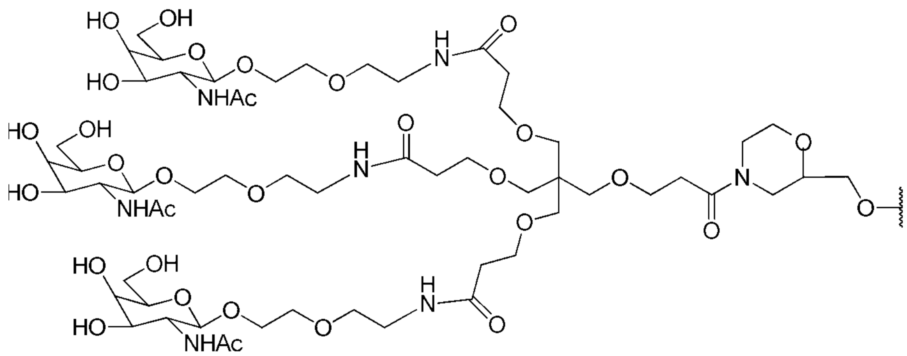 |
| 2 | 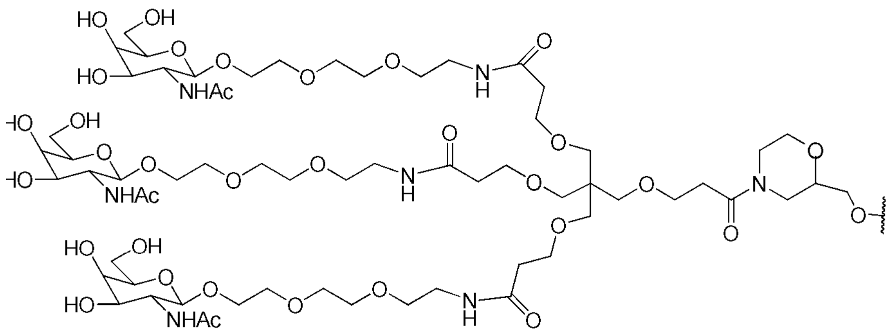 |
| 3 | 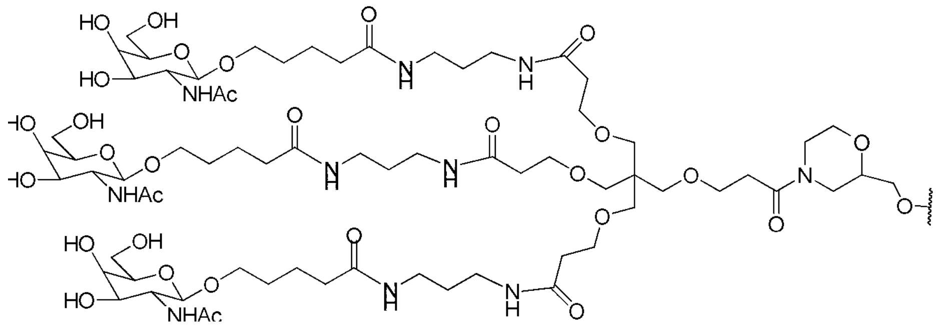 |

(continued)

| No. | Structure |
| --- | --- |
| 4 | 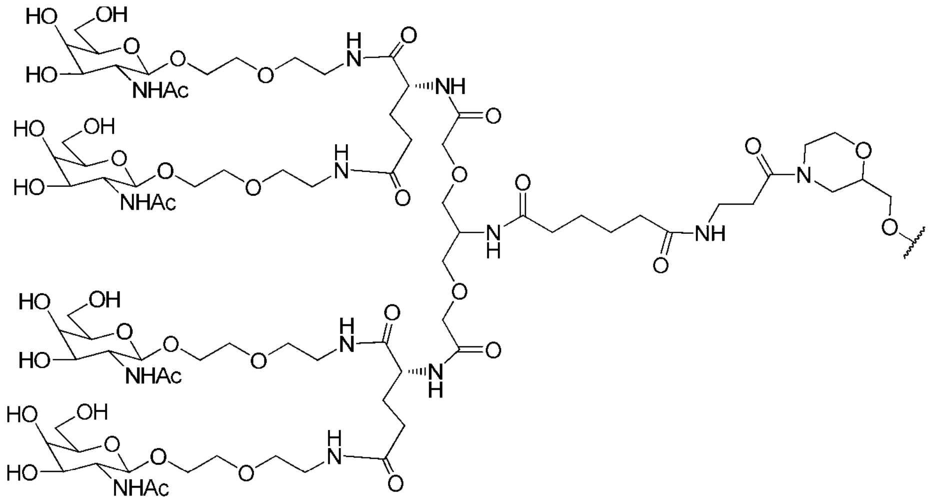 |
| 5 | 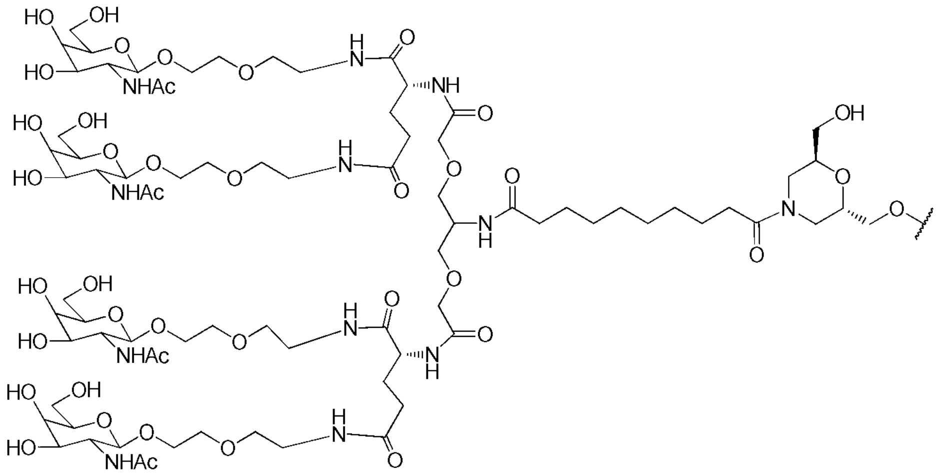 |
| 6 | 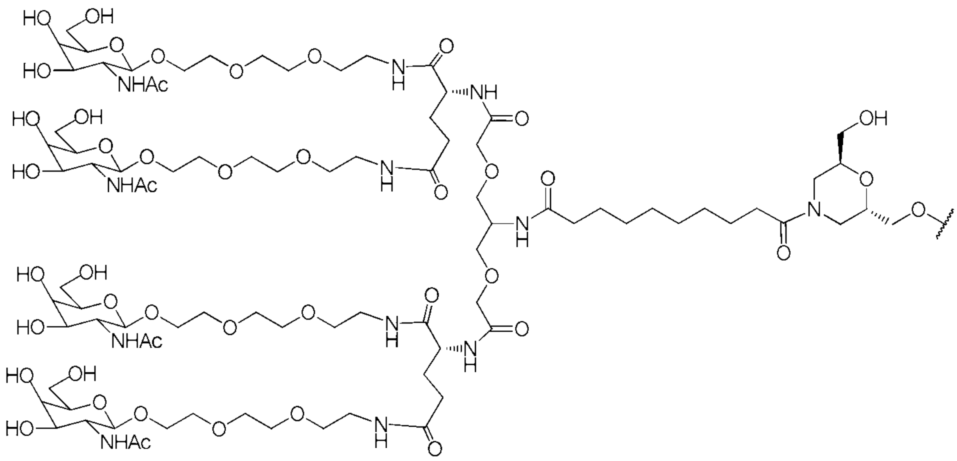 |

(continued)

| No. | Structure |
| --- | --- |
| 7 | 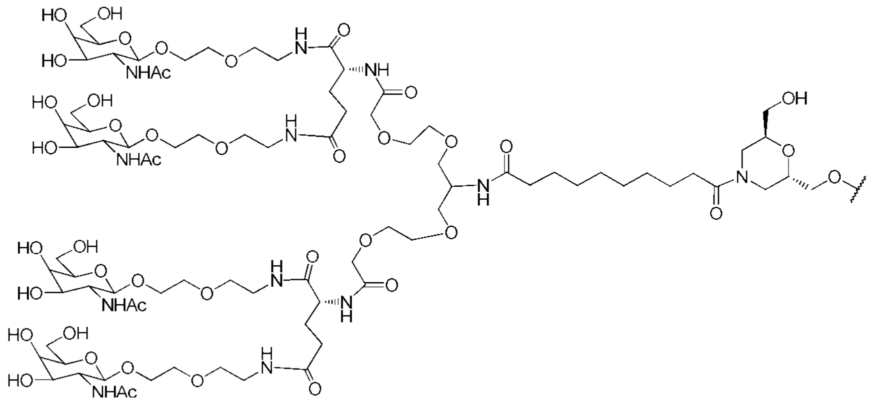 |
| 8 | 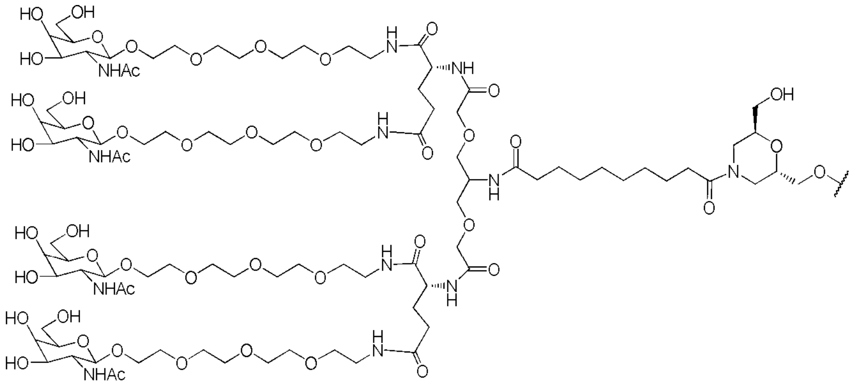 |
| 9 | 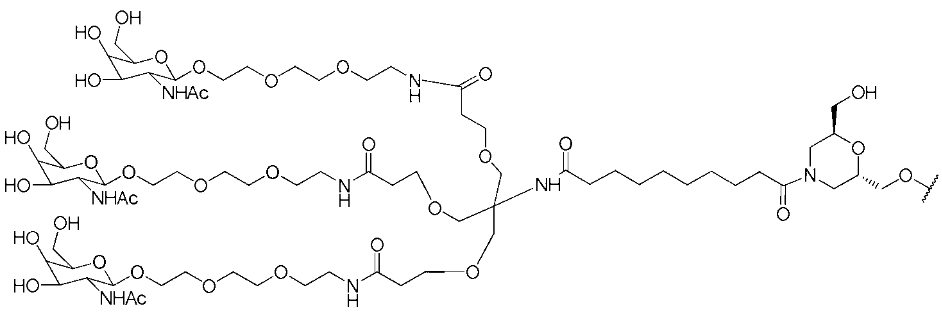 |
| 10 | 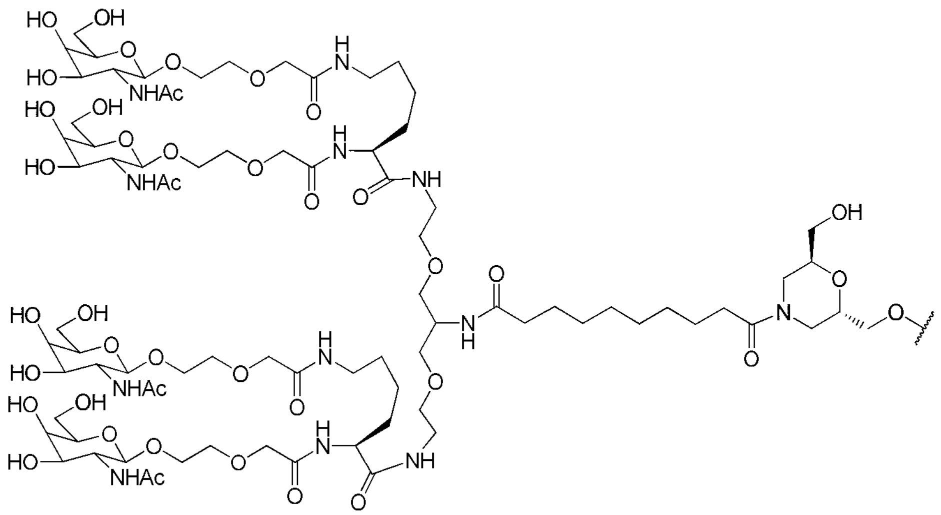 |

(continued)

| No. | Structure |
|---|---|
| 11 | 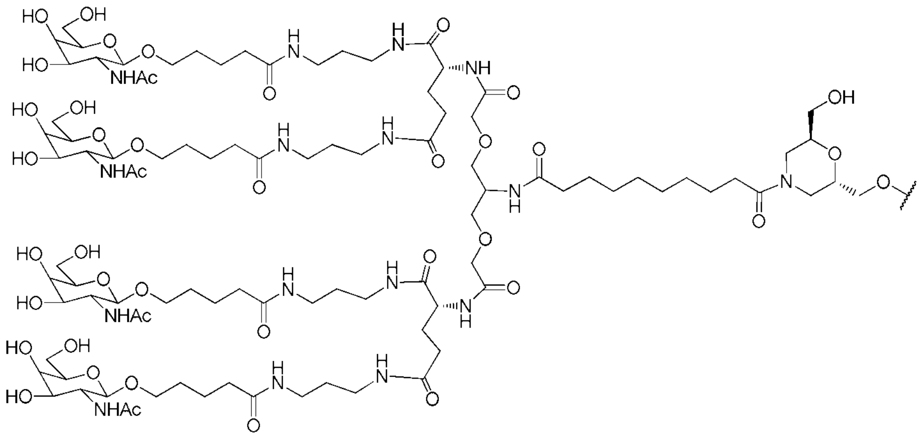 |
| 12 | 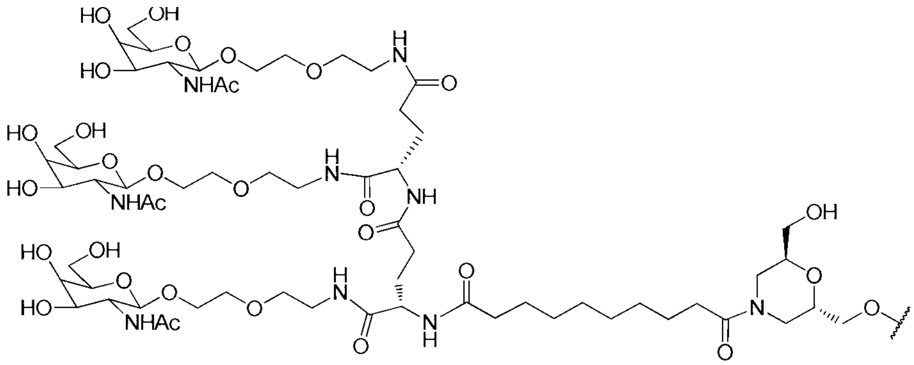 |
| 13 | 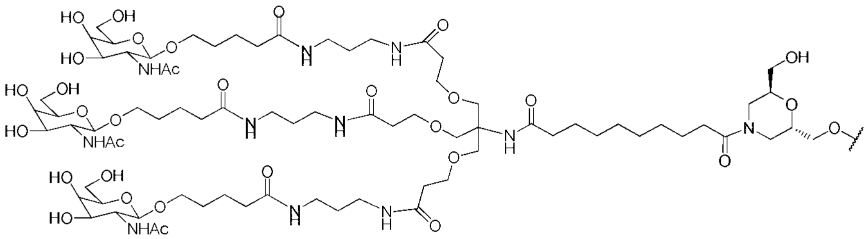 |
| 14 | 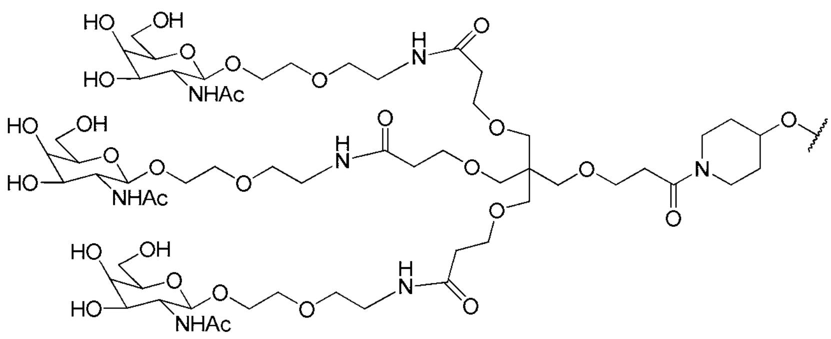 |

(continued)

| No. | Structure |
|---|---|
| 15 | 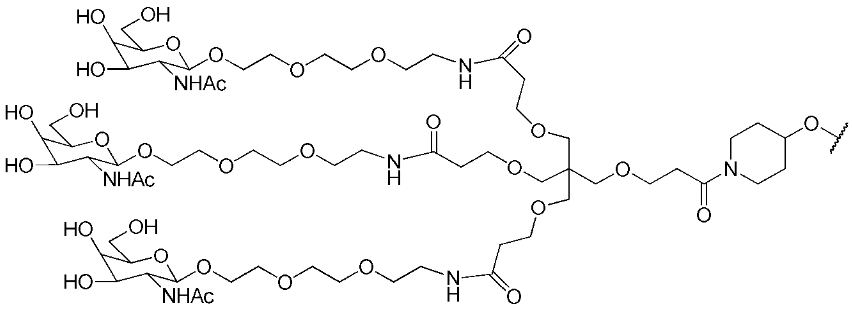 |
| 16 | 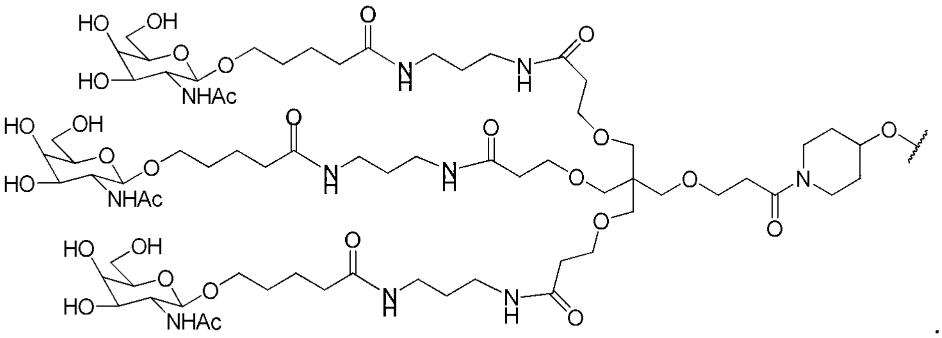 |

In some preferred embodiments, the conjugation group is selected from Table 2:

Table 2. Structure of Conjugation Groups

| No. | Structure |
|---|---|
| 17 | 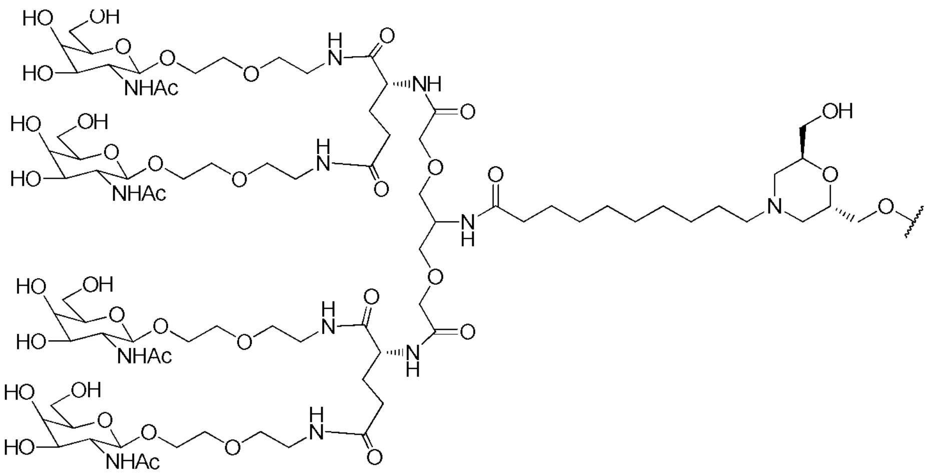 |

(continued)

| No. | Structure |
|---|---|
| 18 | 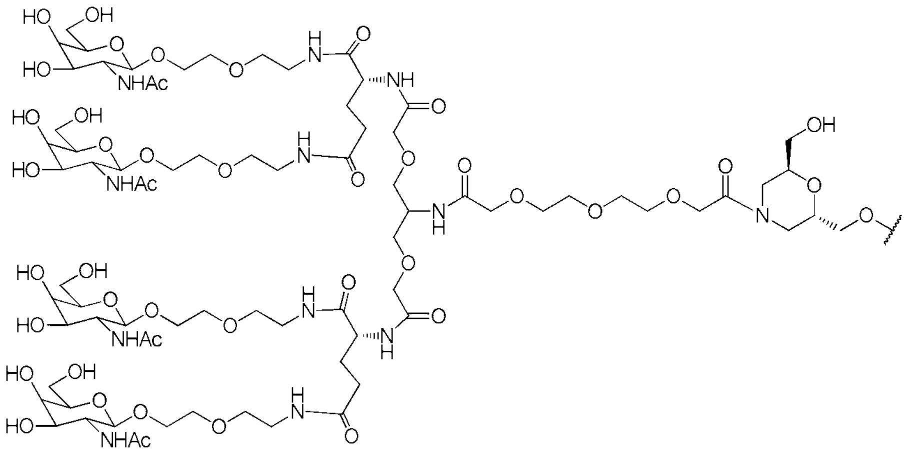 |
| 19 | 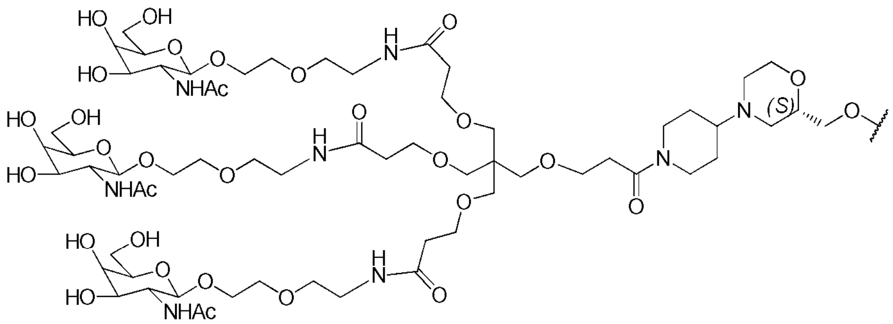 |
| 20 | 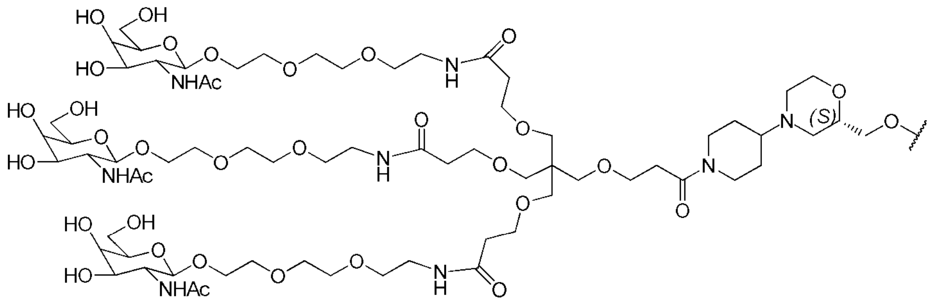 |
| 21 | 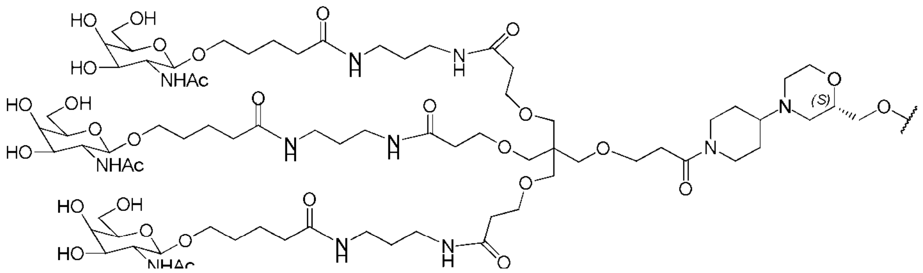 |

(continued)

| No. | Structure |
|---|---|
| 22 | 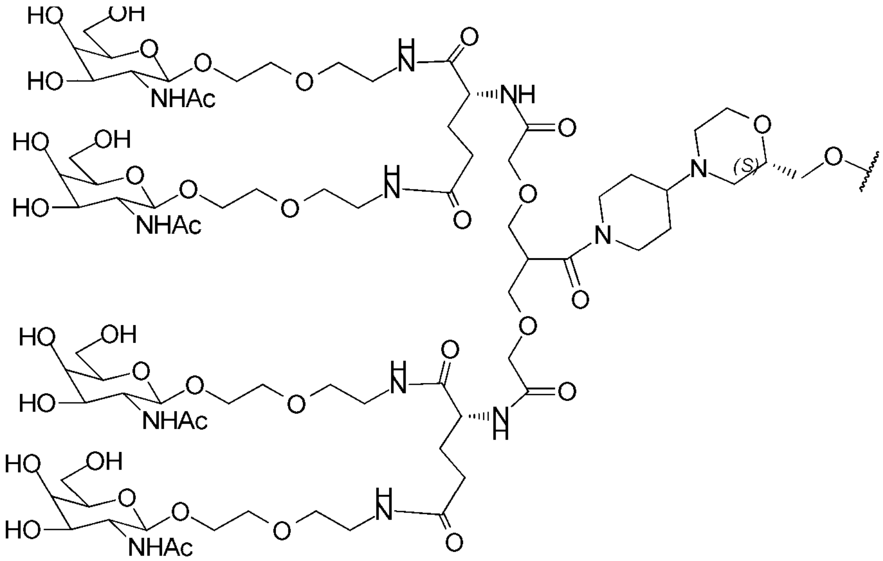 |
| 23-1 | 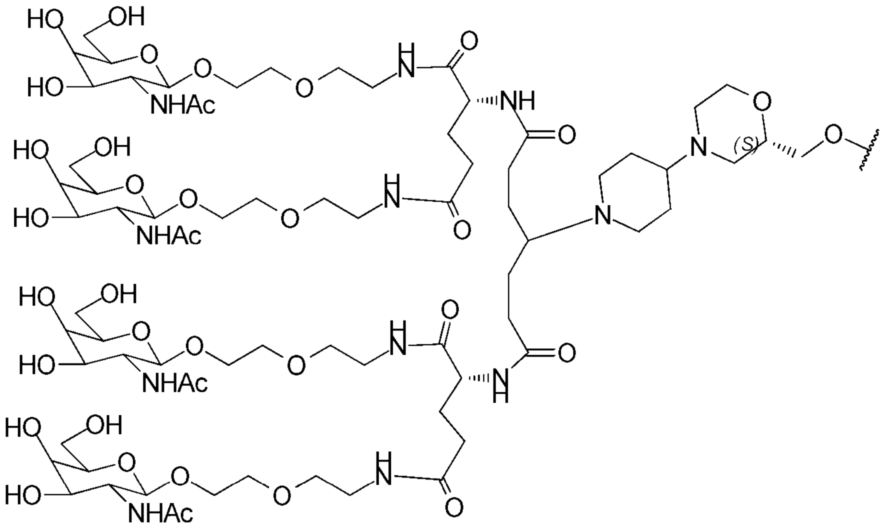 |
| 23-2 | 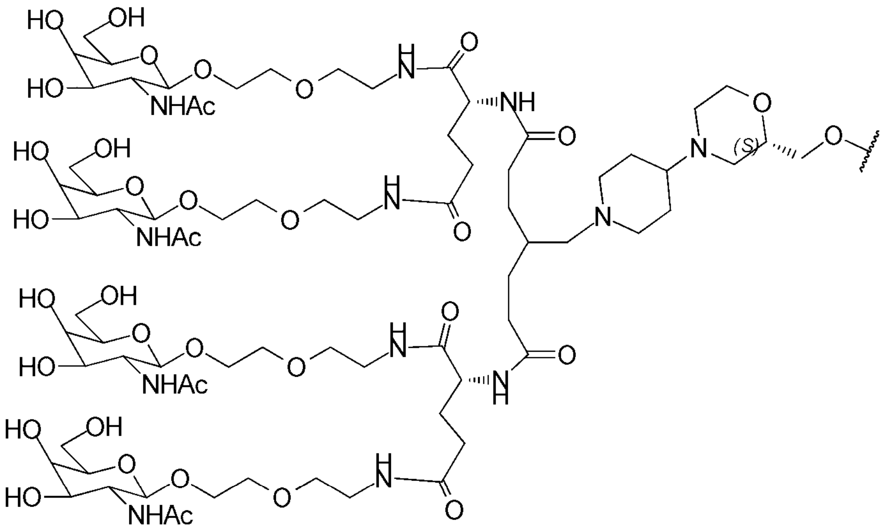 |

(continued)

| No. | Structure |
|---|---|
| 24 | 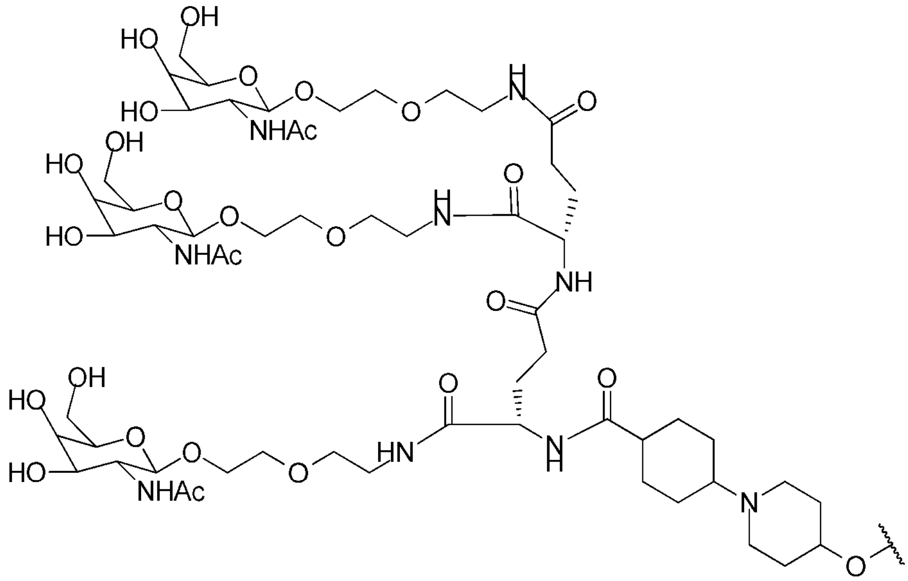 |
| 25 | 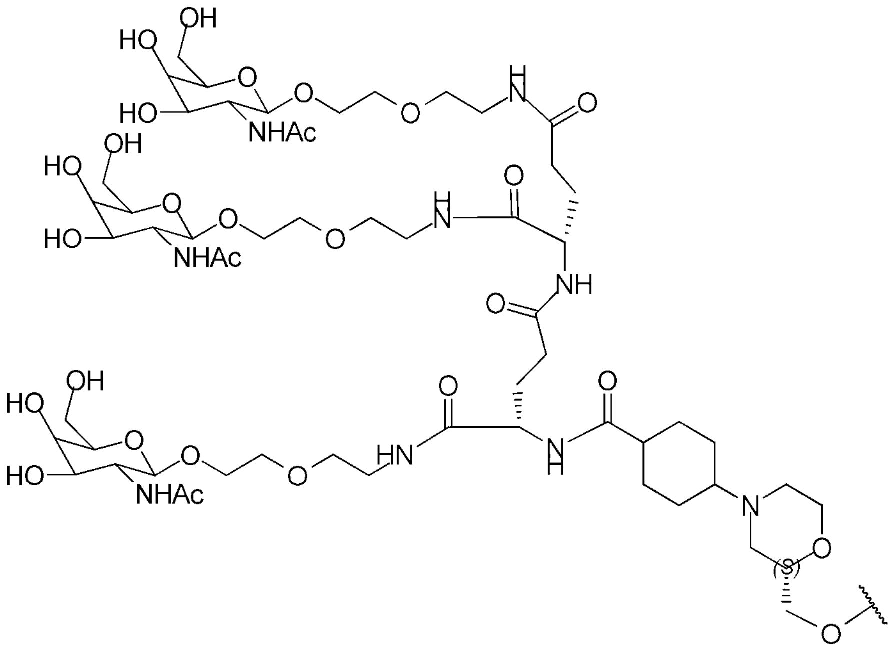 |

(continued)

| No. | Structure |
|---|---|
| 26 | 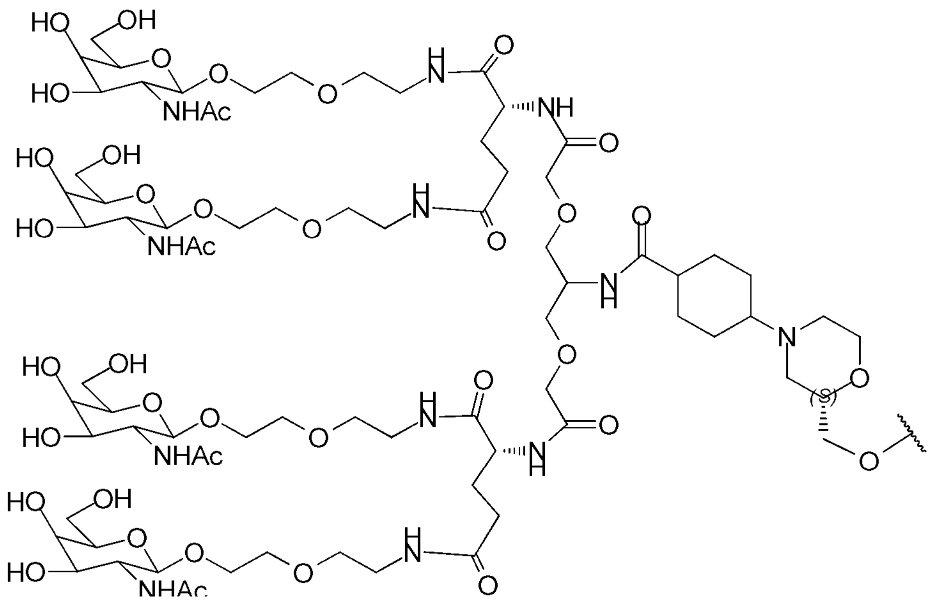 |
| 27 | 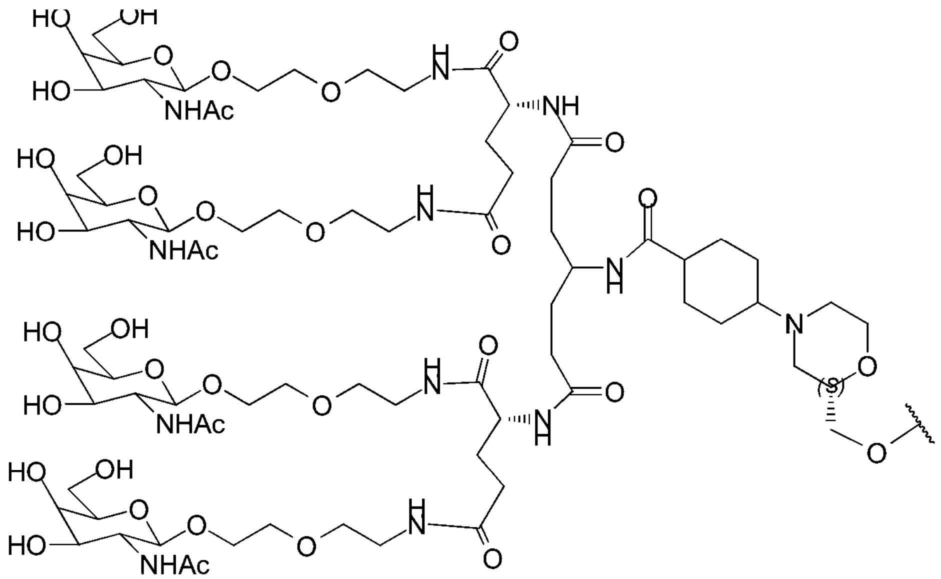 |
| 28 | 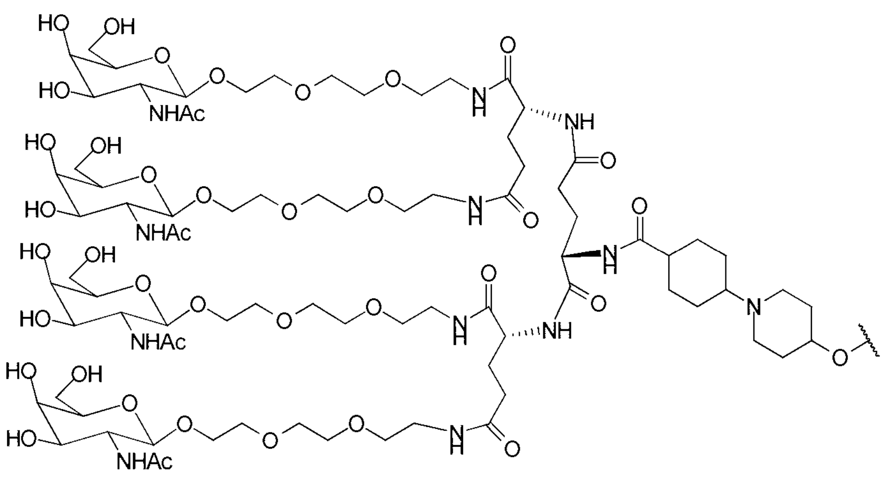 |

(continued)

| No. | Structure |
|---|---|
| 29 | 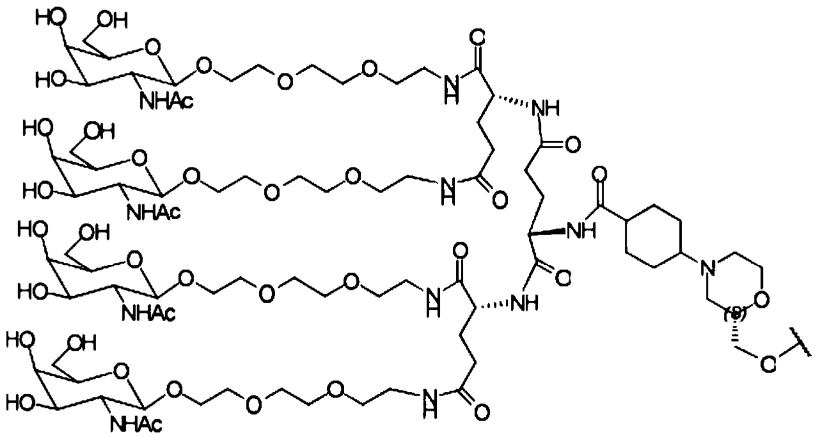 |

In some embodiments, the ligand moiety in the dsRNA has the following structure:

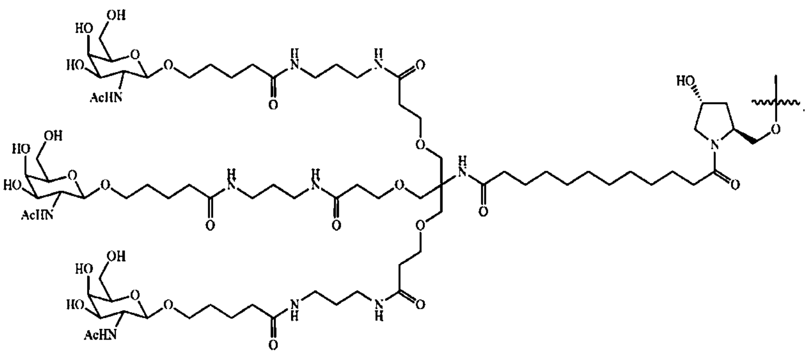

,

wherein

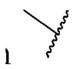

represents the point of attachment to the sense strand (preferably, the 3' end of the sense strand) of the dsRNA via a phosphate group or a phosphorothioate group.

In some embodiments, the ligand moiety in the dsRNA has the following structure:

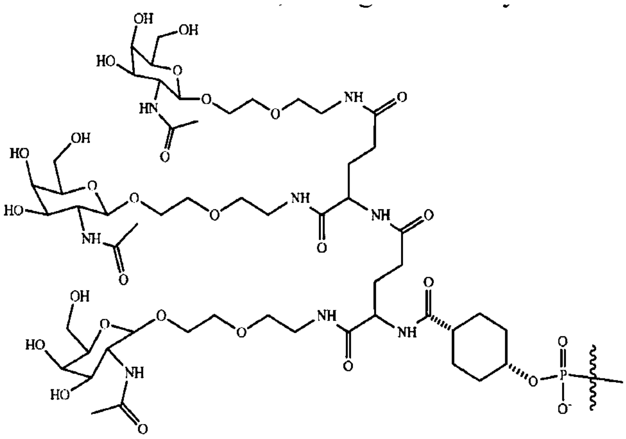

.

wherein

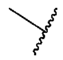

represents the point of attachment to the sense strand (preferably, the 3' end of the sense strand) of the dsRNA via a phosphate group or a phosphorothioate group.

In some embodiments, the ligand moiety in the dsRNA of the present disclosure has the following structure:

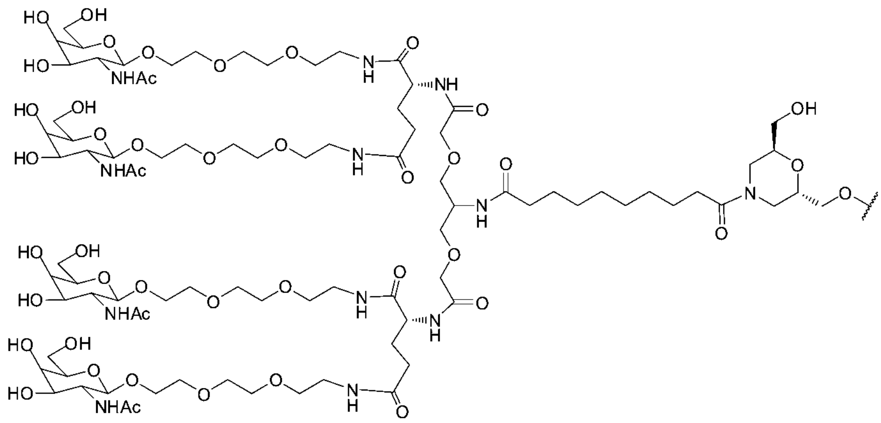

,

wherein

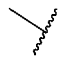

represents the point of attachment to the dsRNA via a phosphate group or a phosphorothioate group.

In some embodiments, the ligand moiety in the dsRNA of the present disclosure has the following structure:

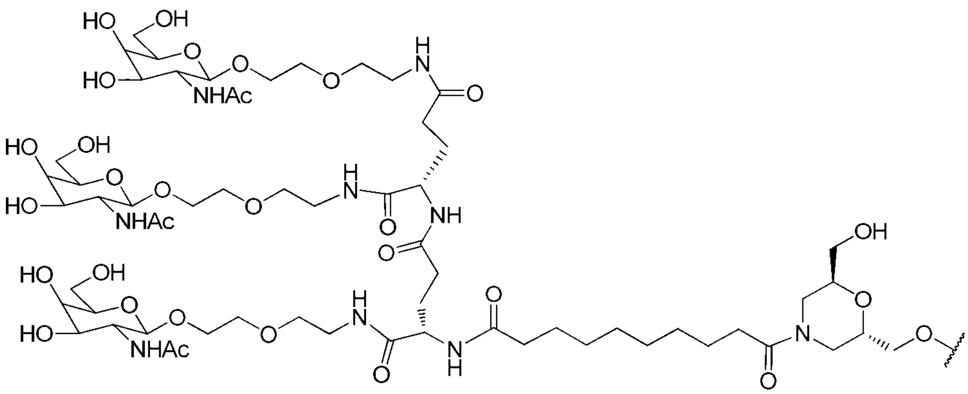

;

wherein

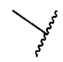

represents the point of attachment to the sense strand (preferably, the 3' end of the sense strand) of the dsRNA via a phosphate group or a phosphorothioate group.

In some embodiments, the antisense strand comprises any one of the modified nucleotide sequences shown in Table 5, and/or the sense strand comprises any one of the modified nucleotide sequences shown in Table 4. In some embodiments, the dsRNA comprises any pair of the paired modified sense strand sequences and modified antisense strand sequences shown in Table 6.

In some embodiments, in the dsRNA of the present disclosure,

(a) the sense strand comprises UmsAmsCmUmCmAmGfGfUfCmAmGmUmAmUmCmUmAmsAms-GL6 (SEQ ID NO: 149), and the antisense strand comprises UmsUfsAmGfAmUfAmCfUmGfAmCfCmUfGmAfGmUfAmsAfsGm (SEQ ID NO: 259);

(b) the sense strand comprises CmsAmsCmCmAmUmGfGfAfGmUmAmCmCmUmCmUmCmsAms-GL6 (SEQ ID NO: 150), and the antisense strand comprises UmsGfsAmGfAmGfGmUfAmCfUmCfCmAfUmGfGmUfGmsGfsAm (SEQ ID NO: 257);

(c) the sense strand comprises AmsGmsGmUmCmAmGfUfAfUmCmUmAmAmUmAmUmAmsAms-GL6 (SEQ ID NO: 151), and the antisense strand comprises UmsUfsAmUfAmUfUmAfGmAfUmAfCmUfGmAfCmCfUmsGfsAm (SEQ ID NO: 260);

(d) the sense strand comprises CmsUmsCmUmAmUmGfAfGfUmUmGmUmGmAmCmUmUmsUms-GL6 (SEQ ID NO: 152), and the antisense strand comprises AmsAfsAmGfUmCfAmCfAmAfCmUfCmAfUmAfGmAfGmsUfsAm (SEQ ID NO: 263);

(e) the sense strand comprises AmsGmsAmCmUmAmUfGfAfCmAmGmCmAmUmCmAmAmsAms-GL6 (SEQ ID NO: 153), and the antisense strand comprises UmsUfsUmGfAmUfGmCfUmGfUmCfAmUfAmGfUmCfUmsUfsUm (SEQ ID NO: 267);

(f) the sense strand comprises GmsAmsCmUmAmUmGfAfCfAmGmCmAmUmCmAmAmAmsUms-GL6 (SEQ ID NO: 154), and the antisense strand comprises AmsUfsUmUfGmAfUmGfCmUfGmUfCmAfUmAfGmUfCmsUfsUm (SEQ ID NO: 268);

(g) the sense strand comprises AmsCmsUmAmUmGmAfCfAfGmCmAmUmCmAmAmAmUmsUms-GL6 (SEQ ID NO: 155), and the antisense strand comprises AmsAfsUmUfUmGfAmUfGmCfUmGfUmCfAmUfAmGfUmsCfsUm (SEQ ID NO: 269);

(h) the sense strand comprises CmsUmsAmUmGmAmCfAfGfCmAmUmCmAmAmAmUmUmsUms-GL6 (SEQ ID NO: 156), and the antisense strand comprises AmsAfsAmUfUmUfGmAfUmGfCmUfGmUfCmAfUmAfGmsUfsCm (SEQ ID NO: 270);

(i) the sense strand comprises AmsUmsGmAmCmAmGfCfAfUmCmAmAmAmUmUmUmCmsAms-GL6 (SEQ ID NO: 157), and the antisense strand comprises UmsGfsAmAfAmUfUmUfGmAfUmGfCmUfGmUfCmAfUmsAfsGm (SEQ ID NO: 271);

(j) the sense strand comprises UmsGmsAmCmAmGmCfAfUfCmAmAmAmUmUmUmCmAmsAms-GL6 (SEQ ID NO: 158), and the antisense strand comprises UmsUfsGmAfAmAfUmUfUmGfAmUfGmCfUmGfUmCfAmsUfsAm (SEQ ID NO: 272);

(k) the sense strand comprises CmsAmsGmAmCmAmGfUfAfCmAmGmGmCmUmAmGmAmsUms-GL6 (SEQ ID NO: 159), and the antisense strand comprises AmsUfsCmUfAmGfCmCfUmGfUmAfCmUfGmUfCmUfGmsCfsAm (SEQ ID NO: 273);

(l) the sense strand comprises AmsGmsAmCmAmGmUfAfCfAmGmGmCmUmAmGmAmUmsAms-GL6 (SEQ ID NO: 160), and the antisense strand comprises UmsAfsUmCfUmAfGmCfCmUfGmUfAmCfUmGfUmCfUmsGfsCm (SEQ ID NO: 274);

(m) the sense strand comprises GmsAmsCmAmGmUmAfCfAfGmGmCmUmAmGmAmUmAmsAms-GL6 (SEQ ID NO: 161), and the antisense strand comprises UmsUfsAmUfCmUfAmGfCmCfUmGfUmAfCmUfGmUfCmsUfsGm (SEQ ID NO: 275);

(n) the sense strand comprises AmsAmsAmCmAmUmUfUfCfCmAmAmUmAmAmAmAmAmsUms-GL6 (SEQ ID NO: 162), and the antisense strand comprises AmsUfsUmUfUmUfAmUfUmGfGmAfAmAfUmGfUmUfUmsUfsUm (SEQ ID NO: 276);

(o) the sense strand comprises AmsCmsAmUmUmUmCfCfAfAmUmAmAmAmAmAmUmAmsUms-GL6 (SEQ ID NO: 163), and the antisense strand comprises AmsUfsAmUfUmUfUmUfAmUfUmGfGmAfAmAfUmGfUmsUfsUm (SEQ ID NO: 277);

(p) the sense strand comprises CmsCmsCmUmAmAmAfCfUfCmCmCmCmAmGmCmAmUmsAms-GL6 (SEQ ID NO: 164), and the antisense strand comprises UmsAfsUmGfCmUfGmGfGmGfAmGfUmUfUmAfGmGfGmsAfsCm (SEQ ID NO: 281);

(q) the sense strand comprises UmsAmsCmUmCmAmGfGfUfCmAmGmUmAmUmCmUmAmsAms-GL6 (SEQ ID NO: 149); and the antisense strand comprises (SCP-U)sdTsAmGmdAUmdACmUmGmAmdCCmUfGmAmGmUmAmsAmsGm (SEQ ID NO: 283);

(r) the sense strand comprises AmsGmsGmUmCmAmGfUfAfUmCmUmAmAmUmAmUmAmsAms-GL6 (SEQ ID NO: 151), and the antisense strand comprises (SCP-U)sdTsAmUmdAUmdTAmGmAmUmdACmUfGmAmCmCmUmsGmsAm (SEQ ID NO: 284);

(s) the sense strand comprises AmsGmsAmCmUmAmUfGfAfCmAmGmCmAmUmCmAmAmsAms-GL6 (SEQ ID NO: 153), and the antisense strand comprises (SCP-U)sdTsUmGmdAUmdGCmUmGmUmdCAmUfAmGmUmCmUmsUmsUm (SEQ ID NO: 285);

(t) the sense strand comprises GmsAmsCmUmAmUmGfAfCfAmGmCmAmUmCmAmAmAmsAms-GL6 (SEQ ID

NO: 165), and the antisense strand comprises (SCP-U)sdTsUmUmdGAmdTGmCmUmGmdTCmAfUmAmGmUmCmsUmsUm (SEQ ID NO: 286);

(u) the sense strand comprises UmsGmsAmCmAmGmCfAtUfCmAmAmAmUmUmUmCmAmsAms-GL6 (SEQ ID NO: 158), and the antisense strand comprises (SCP-U)sdTsGmAmdAAmdTUmUmGmAmdTGmCfUmGmUmCmAmsUmsAm (SEQ ID NO: 288);

(v) the sense strand comprises AmsGmsAmCmAmGmUfAfCfAmGmGmCmUmAmGmAmUmsAms-GL6 (SEQ ID NO: 160), and the antisense strand comprises (SCP-U)sdAsUmCmdTAmdGCmCmUmGmdTAmCfUmGmUmCmUmsGmsCm (SEQ ID NO: 289);

(w) the sense strand comprises GmsAmsCmAmGmUmAfCfAfGmGmCmUmAmGmAmUmAmsAms-GL6 (SEQ ID NO: 161), and the antisense strand comprises (SCP-U)sdTsAmUmdCUmdAGmCmCmUmdGUmAfCmUmGmUmCmsUmsGm (SEQ ID NO: 290);

(x) the sense strand comprises AmsAmsAmCmAmUmUfUfCfCmAmAmUmAmAmAmAmAmsAms-GL6 (SEQ ID NO: 166), and the antisense strand comprises (SCP-U)sdTsUmUmdTUmdAUmUmGmGmdAAmAfUmGmUmUmUmsUmsUm (SEQ ID NO: 291);

(y) the sense strand comprises AmsCmsAmUmUmUmCfCfAfAmUmAmAmAmAmAmUmAmsAms-GL6 (SEQ ID NO: 167), and the antisense strand comprises (SCP-U)sdTsAmUmdTUmdTUmAmUmUmdGGmAfAmAmUmGmUmsUmsUm (SEQ ID NO: 292);

(z) the sense strand comprises GmsCmsUmCmUmGmAfAfCfCmCmCmAmGmUmGmAmCmsAms-GL6 (SEQ ID NO: 168), and the antisense strand comprises (SCP-U)sdGsUmCmdACmdTGmGmGmGmdTUmCfAmGmAmGmCmsUmsGm (SEQ ID NO: 293);

(ab) the sense strand comprises GmsCmsCmAmGmGmAfGfCfUmGmCmUmAmGmCmCmAmsAms-GL6 (SEQ ID NO: 169), and the antisense strand comprises (SCP-U)sdTsGmGmdCUmdAGmCmAmGmdCUmCfCmUmGmGmCmsUmsGm (SEQ ID NO: 294);

(ac) the sense strand comprises CmsCmsUmUmUmGmAfCfGfUmGmUmAmCmAmAmGmCmsAms-GL6 (SEQ ID NO: 170), and the antisense strand comprises (SCP-U)sdGsCmUmdTGmdTAmCmAmCmdGUmCfAmAmAmGmGmsUmsGm (SEQ ID NO: 295);

(ad) the sense strand comprises GmsCmsCmAmUmAmUfGfUfUmGmCmUmGmGmGmAmAmsAms-GL6 (SEQ ID NO: 171), and the antisense strand comprises (SCP-U)sdTsUmCmdCCmdAGmCmAmAmdCAmUfAmUmGmGmCmsUmsCm (SEQ ID NO: 296);

(ae) the sense strand comprises CmsCmsGmCmCmUmCfCfAfUmUmCmCmUmAmCmUmAmsAms-GL6 (SEQ ID NO: 172), and the antisense strand comprises (SCP-U)sdTsAmGmdTAmdGGmAmAmUmdGGmAfGmGmCmGmGmsUmsCm (SEQ ID NO: 297);

(af) the sense strand comprises GmsCmsAmAmAmGmAfCfUfAmUmGmAmCmAmGmCmAmsAms-GL6 (SEQ ID NO: 173), and the antisense strand comprises (SCP-U)sdTsGmCmdTGmdTCmAmUmAmdGUmCfUmUmUmGmCmsAmsGm (SEQ ID NO: 298);

(ag) the sense strand comprises CmsUmsCmUmGmAmAfCfCfCmCmAmGmUmGmAmCmUmsAms-GL6 (SEQ ID NO: 174), and the antisense strand comprises (SCP-U)sdAsGmUmdCAmdCUmGmGmGmdGUmUfCmAmGmAmGmsCmsUm (SEQ ID NO: 299);

(ah) the sense strand comprises CmsCmsUmCmAmCmAfUfCfCmCmAmAmGmUmCmUmAmsAms-GL6 (SEQ ID NO: 176), and the antisense strand comprises (SCP-U)sdTsAmGmdACmdTUmGmGmGmdAUmGfUmGmAmGmGmsCmsGm (SEQ ID NO: 301);

(ai) the sense strand comprises AmsUmsUmUmCmCmAfAfUfAmAmAmAmAmUmAmUmCmsAms-GL6 (SEQ ID NO: 177), and the antisense strand comprises (SCP-U)sdGsAmUmdAUmdTUmUmUmAmdTUmGfGmAmAmAmUmsGmsUm (SEQ ID NO: 302);

(aj) the sense strand comprises AmsAmsAmAmAmCmAmUmUfUfCfCmAmAmUmAmAmAmAmAmsAms-GL6 (SEQ ID NO: 178), and the antisense strand comprises (SCP-U)sUfsUmUfUmUfAmUfUmGfGmAfAmAfUmGfUmUfUmUmUmsGmsUm (SEQ ID NO: 303);

(ak) the sense strand comprises AmsAmsAmCmAmUmUmUmCfCfAfAmUmAmAmAmAmAmUmAmsAms-GL6 (SEQ ID NO: 179), and the antisense strand comprises (SCP-U)sUfsAmUfUmUfUmUfAmUfUmGfGmAfAmAfUmGfUmUmUmsUmsUm (SEQ ID NO: 304),

Wherein GL6 is

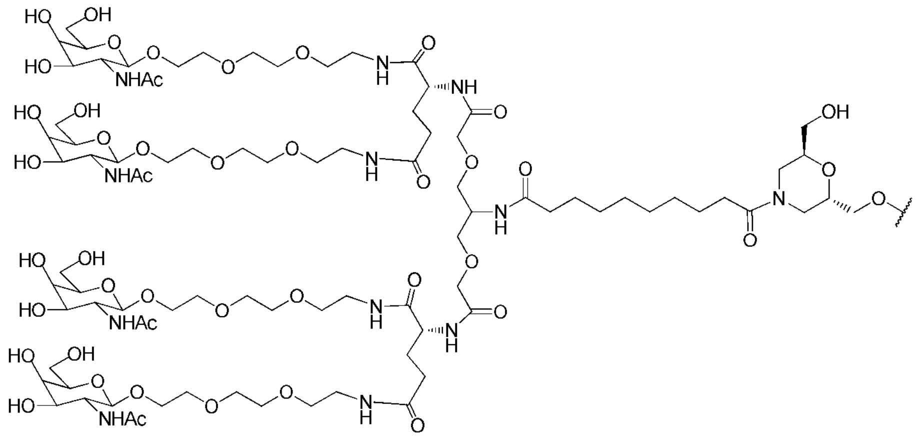

Wherein

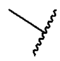

represents the point of attachment to the 3' end of the sense strand of the dsRNA via a phosphate group or a phosphorothioate group.

In a second aspect, the present disclosure provides a cell comprising the dsRNA of the present disclosure.

In a third aspect, the present disclosure provides a pharmaceutical composition comprising the dsRNA or the cell of the present disclosure, and optionally a pharmaceutically acceptable carrier or excipient.

In a fourth aspect, the present disclosure provides a kit comprising the dsRNA, the cell, or the pharmaceutical composition of the present disclosure.

In a fifth aspect, the present disclosure provides a method of reducing cell death-inducing DFFA-like effector B (CIDEB) in a subject, comprising a step of administering to the subject the dsRNA, the cell, or the pharmaceutical composition of the present disclosure. The present disclosure also provides a method of treating a disease or disorder benefiting from reduced expression of cell death-inducing DFFA-like effector B (CIDEB) in a subject, comprising a step of administering to the subject the dsRNA, the cell, or the pharmaceutical composition of the present disclosure. The present disclosure also provides a method of preventing at least one symptom in a subject having a disease or disorder benefiting from reduced expression of cell death-inducing DFFA-like effector B (CIDEB), comprising a step of administering to the subject the dsRNA, the cell of claim 25, or the pharmaceutical composition of the present disclosure.

In some embodiments, the disease or disorder benefiting from reduced expression of cell death-inducing DFFA-like effector B (CIDEB) is a CIDEB-mediated or CIDEB-related disease.

In some embodiments, the CIDEB-mediated or CIDEB-related disease is selected from liver diseases (e.g. fatty liver, hepatitis (e.g. steatohepatitis, non-alcoholic steatohepatitis, alcoholic steatohepatitis, and viral hepatitis), non-alcoholic fatty liver disease, alcoholic fatty liver disease, hepatic fibrosis, cirrhosis and liver failure), cholangitis (primary biliary cholangitis, primary sclerosing cholangitis), endocrine disorders, urinary diseases, metabolic diseases (e.g. metabolic syndrome), hepatobiliary diseases, fibrotic diseases, cardiovascular diseases (e.g. hypertension, endothelial cell dysfunction, arteriosclerosis, atherosclerosis, coronary artery disease, myocardial infarction, ischemic stroke, and other forms of heart diseases), immunoinflammatory diseases, central nervous diseases, gastrointestinal diseases, hyperproliferative diseases (e.g. cancers), dyslipidemia (e.g. hyperlipidemia, hypercholesterolemia, hypertriglyceridemia, high LDL cholesterol, low HDL cholesterol, postprandial hypertriglyceridemia), glycemic control disorders (e.g. insulin resistance, type 2 diabetes), adipocyte dysfunction, visceral fat deposition, obesity, eating disorders, and excessive sugar cravings, and the like. In some embodiments, the CIDEB-mediated or CIDEB-related disease is selected from liver diseases (e.g. fatty liver, hepatitis (e.g. steatohepatitis, non-alcoholic steatohepatitis, alcoholic steatohepatitis, and viral hepatitis), non-alcoholic fatty liver disease, alcoholic fatty liver disease, hepatic fibrosis, cirrhosis, and liver failure), cholangitis (primary biliary cholangitis, primary sclerosing cholangitis), metabolic diseases (e.g. metabolic syndrome), autoimmune diseases, cardiovascular diseases (e.g. hypertension, endothelial cell dysfunction, arteriosclerosis, atherosclerosis, coronary artery disease, myocardial infarction, ischemic stroke, and other forms of cardiac diseases), and dyslipidemia (e.g. hyperlipidemia, hypercholesterolemia, hypertriglyceridemia, high LDL cholesterol, low HDL cholesterol, and postprandial hypertriglyceridemia).

In some embodiments, the inventive method of treating a disease or disorder benefiting from reduced expression of cell death-inducing DFFA-like effector B (CIDEB) in a subject, the inventive method of preventing at least one symptom

in a patient having a disease or disorder benefiting from reduced expression of cell death-inducing DFFA-like effector B (CIDEB), or the inventive method of reducing cell death-inducing DFFA-like effector B (CIDEB) in a subject, comprises administering the dsRNA, the cell, or the pharmaceutical composition to the subject subcutaneously, locally, or intravenously. In some embodiments, the subject is a human patient.

## Detailed Description

The following describes the embodiments of the present invention through specific examples. Those skilled in the art can easily understand other advantages and effects of the present invention from the disclosure of the present specification. The present invention can also be implemented or applied through other different specific embodiments. Various details in this specification can also be modified or changed in various ways based on different viewpoints and applications without departing from the spirit of the present invention.

It should be understood that the protection scope of the present invention is not limited to the following specific embodiments; it should also be understood that the terms used in the embodiments of the present invention are for describing specific embodiments, not for limiting the protection scope of the present invention.

In the specification and claims of the present invention, the singular forms "a", "an", and "the" include plural referents unless the context clearly dictates otherwise.

When the embodiments give numerical ranges, it should be understood that, unless otherwise stated in the present invention, both endpoints of each numerical range and any value between the two endpoints can be selected. Unless defined otherwise, all technical and scientific terms used herein have the same meaning as commonly understood by one of ordinary skill in the art. In addition to the specific methods, equipment, and materials used in the embodiments, those skilled in the art can also use methods, equipment, and materials described in the embodiments of the present invention based on their understanding of the prior art and the description of the present invention. Any methods, equipment, and materials that are similar or equivalent to the prior art to implement the present invention shall fall within the protection scope of the present invention. Embodiments of the present invention are described in greater detail below.

## Definitions

A "double-stranded region" refers herein to a region comprising two nucleic acid strands that are antiparallel and complementary or substantially complementary to each other.

As used herein, the term "double-stranded RNA" or "dsRNA" refers to a ribonucleic acid molecule or a complex of ribonucleic acid molecules comprising a double-stranded region as defined above. The two portions forming the double-stranded region may be two different moieties of a larger RNA molecule, or they may be separate RNA molecules.

When the two portions are separate RNA molecules, the dsRNA herein is referred to as a Small interfering RNA or a short interfering RNA, abbreviated as siRNA.

When the two portions are two different moieties of a larger molecule, that is, when the 3' end of one moiety is connected to the 5' end of the other moiety by one or more uninterrupted nucleotides therebetween to form a double-stranded region, the uninterrupted nucleotide(s) used for this connection is(are) referred to as a "hairpin loop". When the two moieties are covalently linked by means other than a hairpin loop to form a double-stranded region, the linking structure is referred to as a "linker". Such a dsRNA, when introduced into a cell, may be cleaved by an endoribonuclease called Dicer enzyme within the cell into siRNA.

The term "siRNA" herein is a class of dsRNA molecule comprising a sense strand and an antisense strand, which can mediate the silencing of target RNA (e.g., mRNA, e.g., transcript of a protein-encoding gene) complementary or substantially complementary to the antisense strand. A siRNA is generally double-stranded, including an antisense strand complementary to the target RNA thereof and a sense strand complementary or substantially complementary to this antisense strand. For convenience, such an mRNA is also referred to herein as an mRNA to be silenced, and such a gene is also called a target gene. Typically, the RNA to be silenced is an endogenous gene or a pathogen gene. In addition, RNAs other than mRNA (for example, tRNA) and viral RNA may also be targeted.

As used herein, the term "antisense strand" refers to a strand in a dsRNA (particularly an siRNA), wherein said strand contains a region that is completely or substantially complementary to the target sequence thereof.

As used herein, the term "complementary region" refers to a region on an antisense strand that is completely or substantially complementary to the target mRNA sequence thereof. In cases where a complementary region is incompletely complementary to the target sequence thereof, a mismatch may be located in an internal or terminal region of the molecule. Typically, the most tolerated mismatch is located in a terminal region, e.g., within 5, 4, 3, 2 or 1 nucleotide at the 5' and/or 3' end. The portion of an antisense strand most sensitive to a mismatch is called the "seed region". For example, in a siRNA containing a strand of 19 nt, position 19 (from the 5' to the 3') may tolerate some mismatches.

As used herein, the term "complementary" refers to the ability of a first polynucleotide to hybridize with a second polynucleotide under certain conditions, such as stringent conditions. For example, stringent conditions may include 400

mM NaCl, 40 mM PIPES, pH 6.4, 1 mM EDTA, and 50°C or 70°C for 12-16 hours.

As used herein, with respect to fulfilling the above required capabilities related to the hybridization ability thereof, the "complementary" sequences may also include or be entirely composed of non-Watson-Crick base pairs and/or base pairs formed from non-natural as well as modified nucleotides. Such non-Watson-Crick base pairs include, but are not limited to, G:U wobble base pairing or Hoogsteen base pairing.

As used herein, a polynucleotide that is "at least partially complementary" or "substantially complementary" to a messenger RNA (mRNA) refers to a polynucleotide that is substantially complementary to a contiguous portion of an mRNA of interest (e.g., an mRNA encoding CIDEB). For example, a polynucleotide is at least partially complementary to an mRNA encoding CIDEB, when the sequence thereof is substantially complementary to an uninterrupted portion of the *CIDEB* mRNA.

The terms "complementary," "completely complementary," and "substantially complementary" as used herein may be used with respect to base pairing between the sense strand and the antisense strand of a dsRNA, particularly an siRNA, or between the antisense strand of a dsRNA, particularly an siRNA, and a target sequence.

As used herein, the term "sense strand" refers to one strand of an siRNA that comprises a region substantially complementary to the region of an antisense strand as defined herein.

A "nucleoside" is a compound comprising two components: one is a purine base or a pyrimidine base, and the other is a ribose or a deoxyribose. A "nucleotide" is a compound comprising three components: one is a purine base or a pyrimidine base, another is a ribose or deoxyribose, and the third is a phosphoric acid. An "oligonucleotide" refers to, for example, a nucleic acid molecule (RNA or DNA) having a length of less than 100, 200, 300, or 400 nucleotides.

The term "base" is a fundamental building block of nucleosides, nucleotides, and nucleic acids; also referred to as "nitrogenous base," as it always contains nitrogen. Unless otherwise specified, the capital letters herein, i.e., A, U, T, G, and C, denote the bases of nucleotides, representing adenine, uracil, thymine, guanine and cytosine, respectively.

As used herein, the term "nucleotide overhang" refers to at least one unpaired nucleotide that protrudes from the double-stranded region of the siRNA. Nucleotide overhangs exist, for example, when the 3'-end of one strand of the siRNA extends beyond the 5'-end of the other strand, or vice versa. The siRNA may comprise an overhang having at least one nucleotide, an overhang having at least two nucleotides, an overhang having at least three nucleotides, an overhang having at least four nucleotides, or an overhang having at least five nucleotides or more. A nucleotide overhang may comprise or consist of a nucleotide/modified nucleotide (including a deoxynucleotide/nucleoside). One or more overhangs can be on the sense strand or the antisense strand, or any combination thereof. An overhang having one or more nucleotides may be present at the 5'-end, 3'-end, or both ends of the antisense or sense strand of the siRNA.

"Blunt end" means that there are no unpaired nucleotides, i.e., no nucleotide overhang at that end of the double-stranded siRNA. A "blunt-ended siRNA" is a siRNA that is double-stranded throughout its length, i.e., there is no nucleotide overhang at either end of the molecule.

Substantially all nucleotides of the dsRNA, particularly siRNA, of the present invention are modified. For example, substantially all nucleotides of the sense strand are modified nucleotides, or substantially all nucleotides of the antisense strand are modified nucleotides, or substantially all nucleotides of both the sense and antisense strands are modified nucleotides. In other embodiments of the present invention, all nucleotides of the dsRNA, particularly siRNA, of the present invention are modified nucleotides. For example, all nucleotides of the sense strand are modified nucleotides, or all nucleotides of the antisense strand are modified nucleotides, or all nucleotides of both the sense and antisense strands are modified nucleotides. As use herein, "substantially all nucleotides are modified" refers to that the majority, but not necessarily all, the nucleotides of the dsRNA, particularly siRNA, of the present invention are modified and may include no more than 5, 4, 3, 2, or 1 unmodified nucleotide(s).

A "modified nucleotide" herein include, but are not limited to, an SCP modified nucleotide, a 2'-O-alkyl modified nucleotide (e.g., a 2'-O-methyl modified nucleotide, or a 2'-methoxyethyl modified nucleotide), a 2'-fluoro modified nucleotide, a 2'-deoxy modified nucleotide, an inosine ribonucleotide, an abasic nucleotide, an inverted abasic deoxyribonucleotide, a phosphorothioate internucleotide linkage modification, a vinylphosphonate modified nucleotide, a locked nucleotide, an unlocked nucleotide, a 2'-amino modified nucleotide, a 2'-C-alkyl modified nucleotide, a 2'-O-allyl modified nucleotide, a morpholino nucleotide, a phosphoramidate, a non-natural base comprising nucleotide, a terminal nucleotide linked to a cholesteryl derivative or a dodecanoic acid bisdecylamide group, a deoxyribonucleotide, a 3'-terminal deoxythymine (dT) nucleotide, a conformationally restricted nucleotide, a constrained ethyl nucleotide, a 2'-hydroxy modified nucleotide, a nucleotide comprising methylphosphonate group, a nucleotide comprising 5'-phosphate, a nucleotide comprising 5'-phosphate mimic, a glycol modified nucleotide (GNA), and a 2-O-(N-methylacetamide) modified nucleotide, and the like.

A "2'-fluoro modified nucleotide" refers to a nucleotide in which the hydroxyl at the 2' position of the ribosyl in a nucleotide is replaced with a fluorine atom.

A "2'-O-methyl-modified nucleotide" refers to a nucleotide in which the 2'-hydroxyl of the ribosyl group is replaced with a methoxyl.

A "phosphorothioate internucleotide linkage modification" refers to a nucleotide in which one or more oxygen

atom(s) on the phosphate in a nucleotide are replaced with a sulfur atom. A "modification of phosphorothioate internucleotide bonding" refers to a modification where two adjacent nucleotides are linked by a phosphorothioate.

An "SCP modified nucleotide" refers to a modified nucleotide having the structure:

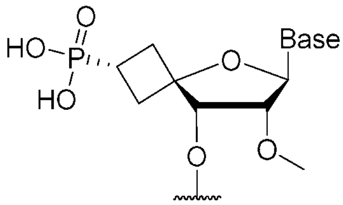

,

wherein Base is independently selected from H, a modified or an unmodified base, or a leaving group. Preferably, Base is an unmodified base, including an adenine base, a guanine base, a uracil base, and a cytosine base. In other embodiments, Base is a modified base.

In some embodiments, the sense strand of the dsRNA, particularly siRNA, of the present disclosure has a modification of two phosphorothioate internucleotide bondings at positions 1 to 5 (counting from the 5' end) and/or has a modification of two phosphorothioate internucleotide bondings at positions 1 to 5 (counting from the 3' end), and/or the antisense strand of the dsRNA, particularly siRNA, of the present disclosure has a modification of two phosphorothioate internucleotide bondings at positions 1 to 5 (counting from the 5' end) and/or has a modification of two phosphorothioate internucleotide bondings at positions 1 to 5 (counting from the 3' end).

As used herein, the interchangeably used term "ligand moiety" or "ligand" refers to a chemical component conjugated with a dsRNA, particularly siRNA, wherein the moiety is capable of altering the distribution, targeting, or lifespan of the dsRNA, particularly siRNA. In some embodiments, the ligand moiety offers enhanced affinity for selected targets, such as molecules, cells or cell types, and compartments (e.g., cellular or organ compartments, tissues, organs, or regions of the body), compared to for example a siRNA without such a ligand moiety. In some embodiments, the ligand moiety targets the asialoglycoprotein receptor (ASGPR) on hepatocytes. Binding of the ligand moiety to ASGPR mediates internalization via clathrin-coated vesicles. Maturation of endosomes leads to a decrease in the pH of lysosomes, which promotes the dissociation of the ligand-receptor complex, thereby releasing the dsRNA, particularly siRNA. Conjugation of the ligand moiety targeting the asialoglycoprotein receptor (ASGPR) on hepatocytes leads to efficacy and stability of the dsRNA, particularly siRNA, in vivo or intracellularly. This facilitates subcutaneous administration of the dsRNA, particularly siRNA.

As used herein, the term "inhibition" is used interchangeably with "reduction," "silencing," "downregulation," and other similar terms and includes any level of inhibition.

The phrase "inhibiting the expression of cell death-inducing DFFA-like effector B (CIDEB)" refers to inhibiting the expression of any *CIDEB* gene as well as variants or mutants of the *CIDEB* gene. Thus, the *CIDEB* gene may be a wild-type *CIDEB* gene, a mutant *CIDEB* gene, or a transgenic *CIDEB* gene in the context of a genetically manipulated cell, a cell population, or an organism.

"Inhibition of *CIDEB* gene expression" includes any level of inhibition of the *CIDEB* gene, such as at least partial inhibition of *CIDEB* gene expression. *CIDEB* gene expression can be assessed based on the level or change in the level of any variable associated with *CIDEB* gene expression, such as *CIDEB* mRNA level or CIDEB protein level. This level may be assessed in a single cell or population of cells (including, for example, samples derived from a subject).

Inhibition can be assessed by a reduction in absolute or relative levels of one or more variables associated with CIDEB expression compared to control levels. The control level can be any type of control level utilized in the art, such as a pre-dose baseline level or a level determined from a similar untreated or control (e.g., a buffer-only control or an inert control)-treated subject, cell, or sample.

A "hydroxy-protecting group" refers to a group that can prevent a hydroxyl from undergoing chemical reactions and can be removed under specific conditions to restore the hydroxyl. The hydroxy-protecting groups mainly include silane-type, acyl-type, or ether-type protecting groups, preferably the following: trimethylsilyl (TMS), triethylsilyl (TES), dimethylisopropylsilyl (DMIPS), diethylisopropylsilyl (DEIPS), tert-butyldimethylsilyl (TBDMS), tert-butyldiphenylsilyl (TBDPS), triisopropylsilyl (TIPS), acetyl (Ac), chloroacetyl, dichloroacetyl, trichloroacetyl, trifluoroacetyl (TFA), benzoyl, p-methoxybenzoyl, 9-fluorenylmethoxycarbonyl (Fmoc), allyloxycarbonyl (Alloc), 2,2,2-trichloroethoxycarbonyl (Troc), benzyloxycarbonyl (Cbz), tert-butoxycarbonyl (Boc), benzyl (Bn), p-methoxybenzyl (PMB), allyl, triphenylmethyl (Tr), di-p-methoxytrityl (DMTr), methoxymethyl (MOM), benzyloxymethyl (BOM), 2,2,2-trichloroethoxymethyl, 2-methoxyethoxymethyl (MEM), methylthiomethyl (MTM), p-methoxybenzyloxymethyl (PMBM).

"Halo-" or "halogen" refers to (substitution by) fluorine (F), chlorine (Cl), bromine (Br), and iodine (I).

"$C_{1-6}$ haloalkyl" refers to the aforementioned "$C_{1-6}$ alkyl", with one or more halogen groups. In some embodiments, a $C_{1-4}$ haloalkyl is particularly preferred, and a $C_{1-2}$ haloalkyl is even more preferred. Exemplary haloalkyls include,

but are not limited to: $-CF_3$, $-CH_2F$, $-CHF_2$, $-CHFCH_2F$, $-CH_2CHF_2$, $-CF_2CF_3$, $-CCl_3$, $-CH_2Cl$, $-CHCl_2$, and 2,2,2-trifluoro-1,1-dimethylethyl. The haloalkyls may be substituted at any substitutable connection site, for example, 1 to 5 substituent(s), 1 to 3 substituent(s), or 1 substituent.

"$C_{1-6}$ alkylene" refers to a divalent group formed by removing another hydrogen of $C_{1-6}$ alkyl and can be substituted or unsubstituted. In some embodiments, $C_{1-4}$ alkylene, $C_{2-4}$ alkylene, and $C_{1-2}$ alkylene are preferred. The unsubstituted alkylenes include, but are not limited to: methylene group ($-CH_2-$), ethylene group ($-CH_2CH_2-$), propylene group ($-CH_2CH_2CH_2-$), butylene group ($-CH_2CH_2CH_2CH_2-$), pentylene group ($-CH_2CH_2CH_2CH_2CH_2-$), and hexylene group ($-CH_2CH_2CH_2CH_2CH_2CH_2-$). Examples of said substituted alkylenes, such as a alkylene substituted by one or more alkyl (methyl) groups, include but are not limited to: substituted methylene ($-CH(CH_3)-$, and $-C(CH_3)_2-$), substituted ethylene ($-CH(CH_3)CH_2-$, $-CH_2CH(CH_3)-$, $-C(CH_3)_2CH_2-$, and $-CH_2C(CH_3)_2-$), substituted propylene ($-CH(CH_3)CH_2CH_2-$, $-CH_2CH(CH_3)CH_2-$, $-CH_2CH_2CH(CH_3)-$, $-C(CH_3)_2CH_2CH_2-$, $-CH_2C(CH_3)_2CH_2-$, $-CH_2CH_2C(CH_3)_2-$), etc.

As used herein, the terms "treat," "treatment," and the like refer to the administration of an agent or the performing of a procedure in order to achieve effects. These effects may be prophylactic in terms of complete or partial prevention of a disease or a symptom thereof, and/or may be therapeutic in terms of affecting partial or complete cure of a disease and/or a symptom thereof. As used herein, "treating" may include treating a disease or disorder (e.g., cancer) in a mammal, particularly a human, and comprises: (a) preventing the occurrence of a disease or the symptom thereof (e.g., including a disease that may be associated with or caused by a primary disease) in a subject who is susceptible to but has not been diagnosed with the disease; (b) inhibiting, i.e. preventing the progression of, a disease; (c) relieving, i.e. causing regression of, a disease. Treatment may refer to any reference to success in treating or ameliorating or preventing a cancer, including any objective or subjective parameter, such as elimination; remission; diminishing of a symptom, or making the disease condition more tolerable to the patient; slowing down the deterioration or decline; or debilitation or reduction of the worsening endpoint. Treatment or amelioration of a symptom is based on one or more objective or subjective parameters including the results of the examination by a doctor. Accordingly, the term "treatment" includes administration of the antibody or the composition or the conjugate disclosed herein to prevent or delay, alleviate or arrest, or inhibit the development of the symptom or condition associated with a disease (e.g., cancer). The term "therapeutic effect" refers to the reduction, elimination, or prevention of a disease, a symptom of the disease, or a side effect of the disease in a subject.

As used herein, the term "effective amount" refers to an amount sufficient to effect treatment of a disease when administered to a subject for treating the disease.

As used herein, the term "subject" refers to any mammalian subject for whom diagnosis, cure, or treatment is desired. A "mammal" for therapeutic purposes refers to any animal classified as a mammal, including humans, domestic and farm animals, as well as laboratory animals, zoo animals, sports animals, or pet animals, such as dogs, horses, cats, cows, sheep, goats, pigs, mice, rats, rabbits, guinea pigs, monkeys, and the like.

**I.** dsRNA

The present invention provides a double strand RNA (dsRNA) for inhibiting the expression of cell death-inducing DFFA-like effector B (CIDEB) in cells, the dsRNA comprising a sense strand and an antisense strand forming a double-stranded region, wherein the length of the sense strand and the antisense strand is each independently 15-30 nucleotides, and the antisense strand comprises a nucleotide sequence of at least 15 contiguous nucleotides from a nucleotide sequence as set forth in any one of SEQ ID NOs: 62-122.

In some embodiments, the double-stranded region formed by the sense strand and the antisense strand is completely complementary. In other embodiments, the double-stranded region formed by the sense strand and the antisense strand is substantially complementary and may contain 1, 2, 3, 4, or 5 non-complementary sites.

In some specific embodiments, the sense strand comprises a nucleotide sequence of at least 15 contiguous nucleotides from a nucleotide sequence as set forth in any one of SEQ ID NOs: 1-61.

In some embodiments, the length of the sense strand and the antisense strand is each independently 15-27 nucleotides, preferably 18-25 nucleotides, more preferably 19-21 nucleotides.

In some embodiments, the length of the double-stranded region is 15-25 base pairs, preferably 16-23 base pairs, more preferably 18-20 base pairs.

In some embodiments, the dsRNA of the present invention is an siRNA. In some other embodiments, a hairpin loop is formed between the sense strand and the antisense strand of the dsRNA of the present invention.

One or both of the sense strand and the antisense strand comprise a 3' overhang and/or a 5' overhang of at least 1 nucleotide; for example, one or both of the sense strand and the antisense strand comprise(s) a 3' overhang and/or a 5' overhang of at least 1 nucleotide. In some preferred embodiments, the antisense strand comprises a 3' overhang and/or a 5' overhang of at least 2 nucleotides, and preferably, the antisense strand comprises a 3' overhang and/or a 5' overhang of 2 nucleotides. In some embodiments, the sense strand and the antisense strand are of the same length. In some embodiments, the full length of the sense strand is complementary to the full length of the antisense strand to form a double strand, i.e., having blunt ends. In some other embodiments, the sense strand and the antisense strand are of the same

length, and a portion of the sense strand is complementary to a portion of the antisense strand, that is, both the sense strand and the antisense strand have 5' overhangs. In some embodiments, the sense strand and the antisense strand are of different lengths. In a preferred embodiment, the 5' end of the antisense strand has an overhang of at least 1 nucleotide, more preferably 2 or 3 nucleotides.

The dsRNA of the present disclosure includes a dsRNA having a nucleotide overhang at one end (i.e., a agent having one overhang and one blunt end) or having nucleotide overhangs at both ends. For example, the 5'-end of the sense strand of a dsRNA comprises an overhang having one or more nucleotides and the 3'-end of the sense strand comprises an overhang having one or more nucleotides. For example, the 5'-end of the antisense strand of a dsRNA comprises an overhang having one or more nucleotides and the 3'-end of the antisense strand comprises an overhang having one or more nucleotides. For example, the 5'-end of the sense strand of a dsRNA comprises an overhang having one or more nucleotides and the 5'-end of the antisense strand comprises an overhang having one or more nucleotides. For example, the 3'-end of the sense strand of a dsRNA comprises an overhang having one or more nucleotides and the 3'-end of the antisense strand comprises an overhang having one or more nucleotides. For example, the 5'-end of the sense strand of a dsRNA comprises an overhang having one or more nucleotides and the 3'-end of the sense strand comprises a blunt end. For example, the 3'-end of the sense strand of a dsRNA comprises an overhang having one or more nucleotides and the 3'-end of the sense strand comprises a blunt end. For example, the 5'-end of the antisense strand of a dsRNA comprises an overhang having one or more nucleotides and the 3'-end of the antisense strand comprises a blunt end. For example, the 3'-end of the antisense strand of a dsRNA comprises an overhang having one or more nucleotides and the 5'-end of the antisense strand comprises a blunt end.

In some embodiments, the antisense strand comprises a nucleotide sequence of at least 16 contiguous nucleotides, a nucleotide sequence of at least 17 contiguous nucleotides, a nucleotide sequence of at least 18 contiguous nucleotides, a nucleotide sequence of at least 19 contiguous nucleotides, or a nucleotide sequence of at least 20 contiguous nucleotides from the nucleotide sequence set forth in any one of SEQ ID NOs: 62-122, and preferably the antisense strand comprises the nucleotide sequence set forth in any one of SEQ ID NOs: 62-122.

In some embodiments, the sense strand comprises a nucleotide sequence of at least 16 contiguous nucleotides, a nucleotide sequence of at least 17 contiguous nucleotides, or a nucleotide sequence of at least 18 contiguous nucleotides from any one of the nucleotide sequences set forth in SEQ ID NOs: 1-61, and preferably the sense strand comprises the nucleotide sequence set forth in any one of SEQ ID NOs: 1-61.

In some embodiments, the dsRNA comprises any pair of the paired sense strand sequences and antisense strand sequences as shown in Table 3.

**II. Modifications to Nucleotide**

In some embodiments, substantially all nucleotides of the sense strand and substantially all nucleotides of the antisense strand are modified nucleotides. In some embodiments, at least 80%, at least 85%, at least 90%, at least 92%, or at least 95% of the nucleotides of the sense strand are modified nucleotides, and/or at least 80%, at least 85%, at least 90%, at least 92%, or at least 95% of the nucleotides of the antisense strand are modified nucleotides.

In some embodiments, all nucleotides of the sense strand are modified nucleotides and/or all nucleotides of the antisense strand are modified nucleotides.

The nucleotide modification described in the present invention may be a modification on the phosphate group, ribose group, and/or base group of the nucleotide.

In some specific embodiments, the sense strand and the antisense strand each independently comprise one or more nucleotide modifications selected from the group consisting of an SCP modified nucleotide, a 2'-O-alkyl modified nucleotide (e.g., a 2'-O-methyl modified nucleotide), a 2'-methoxyethyl modified nucleotide, a 2'-fluoro modified nucleotide, a 2'-deoxy modified nucleotide, an inosine ribonucleotide, an abasic nucleotide, an inverted abasic deoxyribonucleotide, a phosphorothioate internucleotide linkage modification, a vinylphosphonate modified nucleotide, a locked nucleotide, an unlocked nucleotide, a 2'-amino modified nucleotide, a 2'-C-alkyl modified nucleotide, a 2'-O-allyl modified nucleotide, a morpholino nucleotide, a phosphoramidate, a non-natural base comprising nucleotide, a terminal nucleotide linked to a cholesteryl derivative or a dodecanoic acid bisdecylamide group, a deoxyribonucleotide, a 3'-terminal deoxythymine (dT) nucleotide, a conformationally restricted nucleotide, a constrained ethyl nucleotide, a 2'-hydroxy modified nucleotide, a nucleotide comprising methylphosphonate group, a nucleotide comprising 5'-phosphate, a nucleotide comprising 5'-phosphate mimic, a glycol modified nucleotide (GNA), and a 2-O-(N-methylacetamide) modified nucleotide.

In some preferred embodiments, the sense strand and the antisense strand each independently comprise one or more nucleotide modifications selected from the group consisting of a 2'-O-methyl modified nucleotide, a 2'-fluoro modified nucleotide, and a phosphorothioate internucleotide linkage modification. In some preferred embodiments, the sense strand and/or the antisense strand comprises at least two 2'-fluoro modified nucleotides. In some preferred embodiments, the sense strand and/or the antisense strand comprises at least eight 2'-O-methyl modified nucleotides. In

some preferred embodiments, the 3' end and/or 5' end of the sense strand and/or the antisense strand comprises 1-5 phosphorothioate internucleotide bondings, preferably 2-3 phosphorothioate internucleotide bondings. In some embodiments, the sense strand and/or antisense strand comprises an adenine deoxyribonucleotide, a thymine deoxyribonucleotide, a guanine deoxyribonucleotide, and/or a cytosine deoxyribonucleotide. In a more preferred embodiment, the sense strand and/or antisense strand comprises a thymine deoxyribonucleotide. In the most preferred embodiment, the sense strand comprises a thymine deoxyribonucleotide.

In some preferred embodiments, the antisense strand comprises any one of the modified nucleotide sequences shown in Table 5, and/or the sense strand comprises any one of the modified nucleotide sequences shown in Table 4. In some preferred embodiments, the dsRNA comprises any pair of the paired modified sense strand sequence and the modified antisense strand sequence set forth in any one of Table 6.

III. Ligand Moiety

The dsRNA of the present invention is further conjugated to a ligand moiety comprising N-acetylgalactosamine. In a preferred embodiment, the sense strand of the dsRNA is conjugated to the ligand moiety. In some embodiments, the 3' end of the sense strand is conjugated to the ligand moiety. In some other embodiments, the 5' end of the sense strand is conjugated to the ligand moiety.

In some embodiments, the ligand moiety comprises the conjugation group of formula (X'):

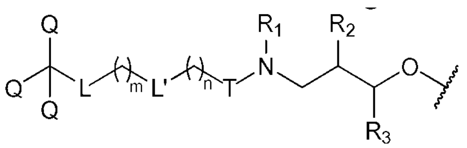
(X')

wherein,

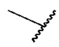

represents the point of attachment to dsRNA;

Q is independently H,

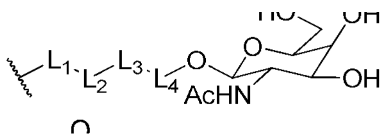
,

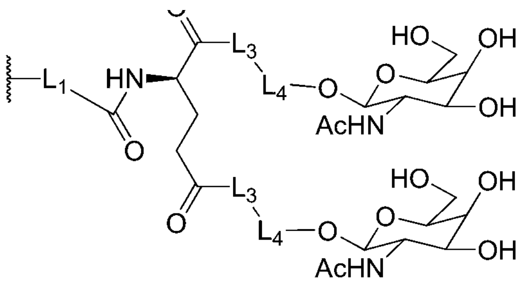
,

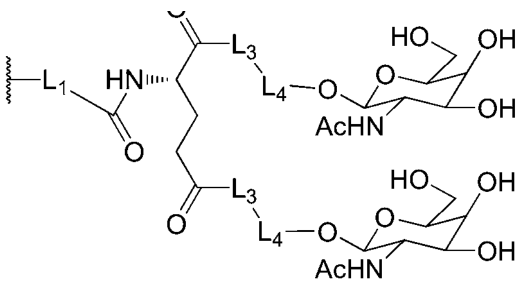
,

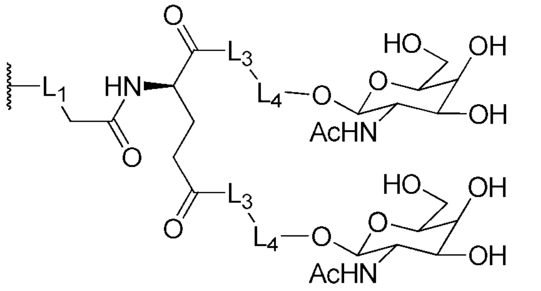
,
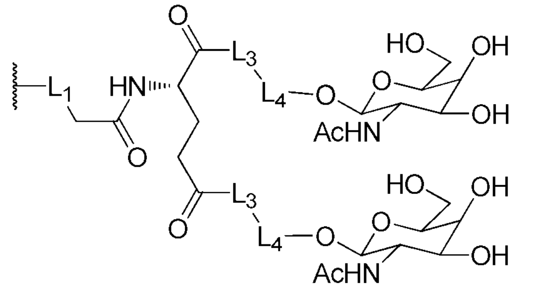
,

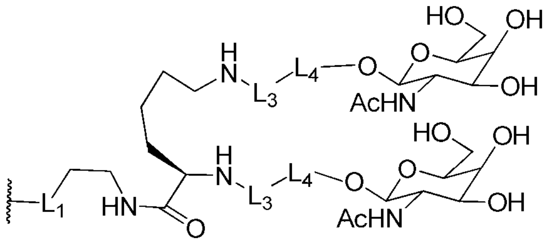
, or
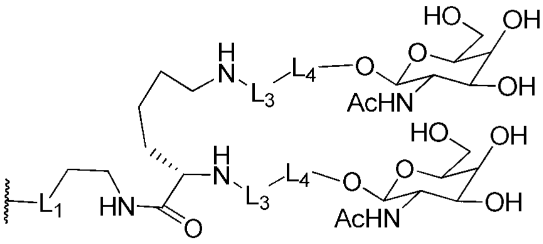
;

wherein $L_1$ is a bond, $-CH_2-$, $-CH_2CH_2-$, -C(O)-, $-CH_2O-$, $-CH_2O-CH_2CH_2O-$, or - $NHC(O)-(CH_2NHC(O))_a-$;
$L_2$ is a bond or $-CH_2CH_2C(O)-$;
$L_3$ is a bond, $-(NHCH_2CH_2)_b-$, $-(NHCH_2CH_2CH_2)_b-$, or $-C(O)CH_2-$;
$L_4$ is $-(OCH_2CH_2)_c-$, $-(OCH_2CH_2CH_2)_c-$, $-(OCH_2CH_2CH_2CH_2)_c-$, - $(OCH_2CH_2CH_2CH_2CH_2)_c-$, or $-NHC(O)-(CH_2)_d-$;

wherein a = 0, 1, 2, or 3;
b = 1, 2, 3, 4 or 5;
c 1, 2, 3, 4 or 5;
d = 1, 2, 3, 4, 5, 6, 7, or 8;

L is a bond, $-CH_2O-$, or -NHC(O)-;
L' is a bond, -C(O)NH-, -NHC(O)-, or $-O(CH_2CH_2O)_e-$;
wherein e is 1, 2, 3, 4, or 5;
T is a bond, $-CH_2-$, -C(O)-, -M-, $-CH_2-M-$, or -C(O)-M-;
wherein M is

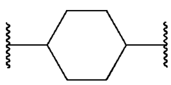
, or
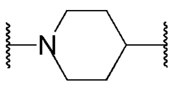
;

$R_1$ and $R_2$ together form $-CH_2CH_2O-$ or $-CH_2CH(R)-O-$, and $R_3$ is H;
or $R_1$ and $R_3$ together form $-C_{1-2}$ alkylene-, and $R_2$ is H;
wherein R is -OR', $-CH_2OR'$, or $-CH_2CH_2OR'$; wherein R' is H, a hydroxy-protecting group, or a solid support, and the hydroxy-protecting group is preferably - $C(O)CH_2CH_2C(O)OH$ or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

In some embodiments, the conjugation group is as shown in formula (I'):

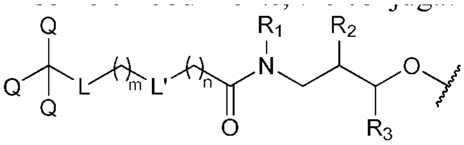
(I')

wherein,

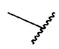

represents the point of attachment to dsRNA;

Q is independently H,

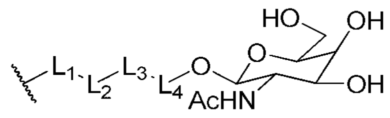

,

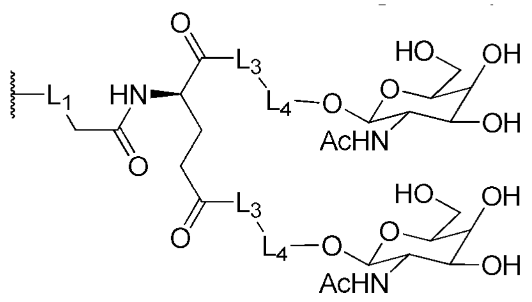

,

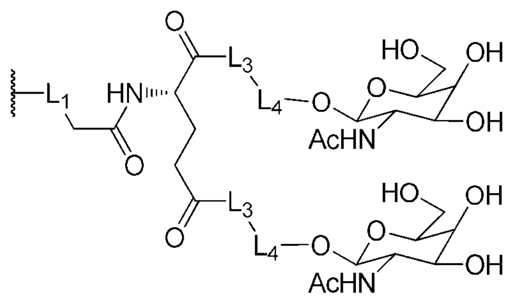

,

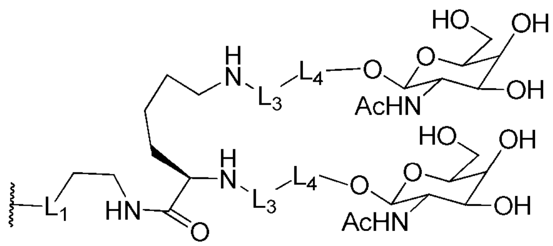

, or

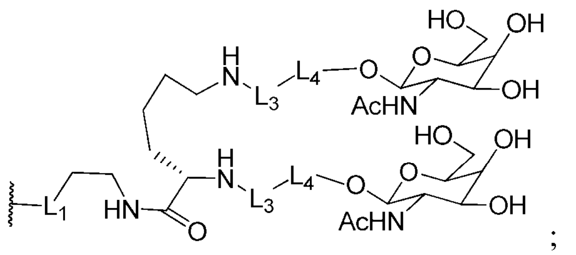

;

wherein $L_1$ is a bond, $-CH_2-$, $-CH_2CH_2-$, -C(O)-, $-CH_2O-$, $-CH_2O-CH_2CH_2O-$, or - $NHC(O)-(CH_2NHC(O))_a-$;

$L_2$ is a bond or $-CH_2CH_2C(O)-$;

$L_3$ is a bond, $-(NHCH_2CH_2)_b-$, $-(NHCH_2CH_2CH_2)_b-$, or $-C(O)CH_2-$;

$L_4$ is $-(OCH_2CH_2)_c-$, $-(OCH_2CH_2CH_2)_c-$, $-(OCH_2CH_2CH_2CH_2)_c-$, - $(OCH_2CH_2CH_2CH_2CH_2)_c-$, or $-NHC(O)-(CH_2)_d-$;

wherein a = 0, 1, 2, or 3;

b = 1, 2, 3, 4 or 5;

c = 1, 2, 3, 4 or 5;

d = 1, 2, 3, 4, 5, 6, 7, or 8;

L is $-CH_2O-$ or -NHC(O)-;

L' is a bond, -C(O)NH- or -NHC(O)-;

$R_1$ and $R_2$ together form $-CH_2CH_2O-$ or $-CH_2CH(R)-O-$, and $R_3$ is H;

or $R_1$ and $R_3$ together form $-C_{1-2}$ alkylene-, and $R_2$ is H;

wherein R is -OR', $-CH_2OR'$, or $-CH_2CH_2OR'$; wherein R' is H, a hydroxy-protecting group, or a solid support, and the hydroxy-protecting group is preferably - $C(O)CH_2CH_2C(O)OH$ or 4,4'-dimethoxytrityl;

m = 0, 1, 2, 3, 4, 5, 6, 7, 8, 9 or 10;

n = 0, 1, 2, 3, 4, 5, 6, 7, 8, 9 or 10.

In some specific embodiments, wherein,

Q is independently H or

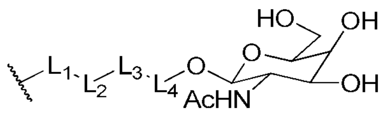;

wherein $L_1$ is $-CH_2O-$ or $-NHC(O)-(CH_2NHC(O))_a-$;
$L_2$ is $-CH_2CH_2C(O)-$;
$L_3$ is $-(NHCH_2CH_2)_b-$ or $-(NHCH_2CH_2CH_2)_b-$;
$L_4$ is $-(OCH_2CH_2)_c-$ or $-NHC(O)-(CH_2)_d-$;

wherein a = 0, 1, 2, or 3;
b = 1, 2, 3, 4 or 5;
c = 1, 2, 3, 4 or 5;
d = 1, 2, 3, 4, 5, 6, 7, or 8;

L is $-CH_2O-$;
L' is a bond;
$R_1$ and $R_2$ together form $-CH_2CH_2O-$ or $-CH_2CH(R)-O-$, and $R_3$ is H;
or $R_1$ and $R_3$ together form $-C_{1-2}$ alkylene-, and $R_2$ is H;
wherein R is -OR', $-CH_2OR'$, or $-CH_2CH_2OR'$; wherein R' is H, a hydroxy-protecting group, or a solid support, and the hydroxy-protecting group is preferably - $C(O)CH_2CH_2C(O)OH$ or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

In some embodiments, the conjugation group is as shown in formula (I'-1), formula (I'-2), or formula (I'-3):

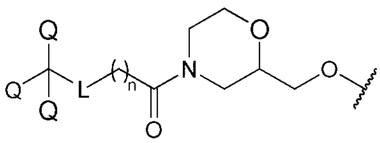

(I'-1),

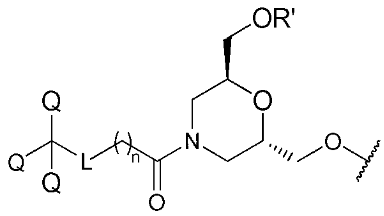

(I'-2),

or

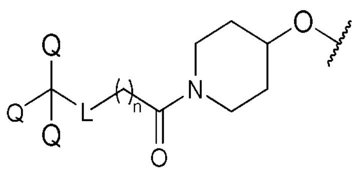

(I'-3)

wherein,

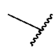

represents the point of attachment to dsRNA;
Q is

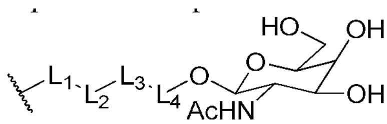;

wherein $L_1$ is $-CH_2O-$ or $-NHC(O)-$;
$L_2$ is $-CH_2CH_2C(O)-$;

$L_3$ is $-(NHCH_2CH_2)_b-$ or $-(NHCH_2CH_2CH_2)_b-$;
$L_4$ is $-(OCH_2CH_2)_c-$ or $-NHC(O)-(CH_2)_d-$;

wherein b=1, 2, 3, 4, or 5;
c = 1, 2, 3, 4 or 5;
d = 1, 2, 3, 4, 5, 6, 7, or 8;

L is $-CH_2O-$;
R' is H, a hydroxy-protecting group, or a solid support, wherein the hydroxy-protecting group is preferably $-C(O)CH_2CH_2C(O)OH$ or 4,4'-dimethoxytrityl;
n = 0, 1, 2, 3, 4, 5, 6, 7, 8, 9 or 10.

In some specific embodiments, wherein,

Q is independently H,

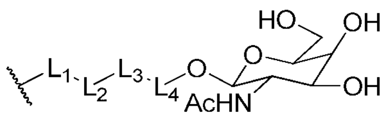

,

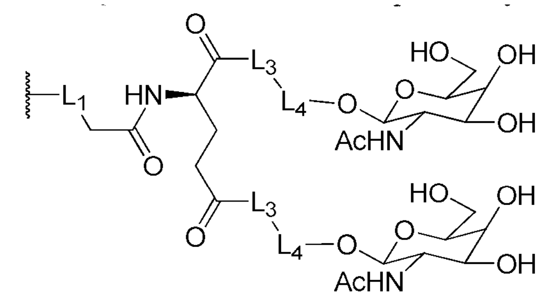

,

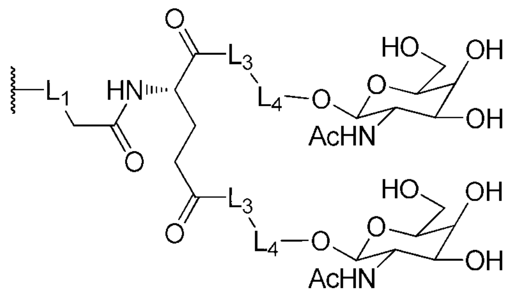

,

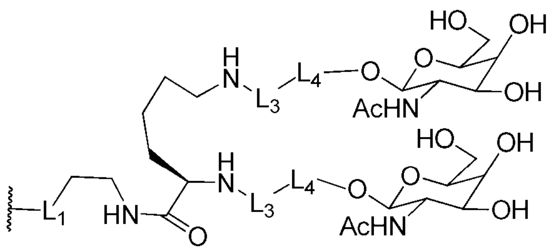

, or

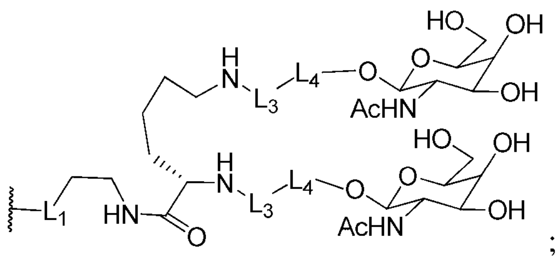

;

wherein $L_1$ is $-CH_2O-$, $-CH_2O-CH_2CH_2O-$, or $-NHC(O)-(CH_2NHC(O))_a-$;
$L_2$ is $-CH_2CH_2C(O)-$;
$L_3$ is $-(NHCH_2CH_2)_b-$, $-(NHCH_2CH_2CH_2)_b-$, or $-C(O)CH_2-$;
$L_4$ is $-(OCH_2CH_2)_c-$ or $-NHC(O)-(CH_2)_d-$;

wherein a = 0, 1, 2, or 3;
b = 1, 2, 3, 4 or 5;
c = 1, 2, 3, 4 or 5;
d = 1, 2, 3, 4, 5, 6, 7, or 8;

L is $-CH_2O-$ or $-NHC(O)-$;
L' is a bond or $-C(O)NH-$;
$R_1$ and $R_2$ together form $-CH_2CH_2O-$ or $-CH_2CH(R)-O-$, and $R_3$ is H;
or $R_1$ and $R_3$ together form $-C_{1-2}$ alkylene-, and $R_2$ is H;
wherein R is -OR', $-CH_2OR'$, or $-CH_2CH_2OR'$; wherein R' is H, a hydroxy-protecting group, or a solid support, and the hydroxy-protecting group is preferably - $C(O)CH_2CH_2C(O)OH$ or 4,4'-dimethoxytrityl;

m = 0, 1, 2, 3, 4, 5, 6, 7, 8, 9 or 10;
n = 0, 1, 2, 3, 4, 5, 6, 7, 8, 9 or 10.

In some embodiments, the conjugation group is as shown in formula (II' -1) or formula (II'-2):

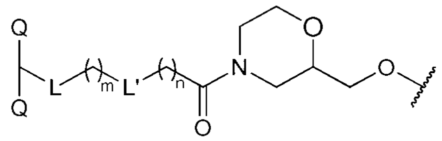
(II'-1) or
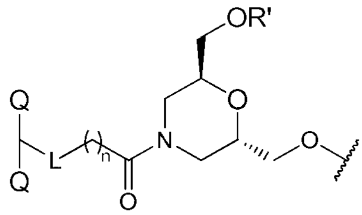
(II'-2)

wherein,

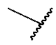

represents the point of attachment to siRNA;
Q is independently

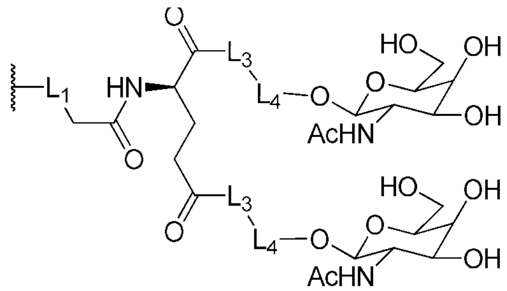
,

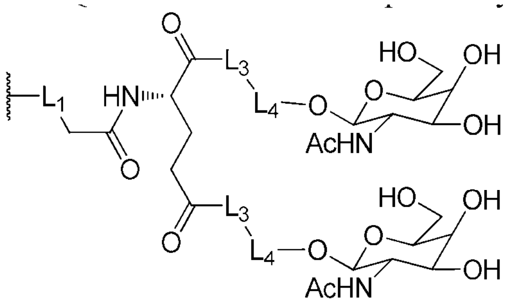
,
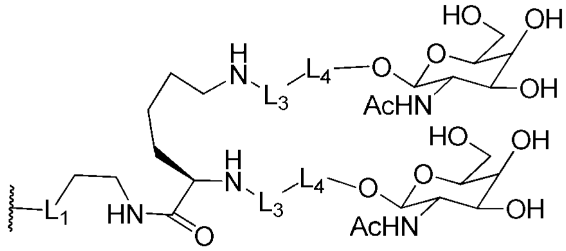
,

or

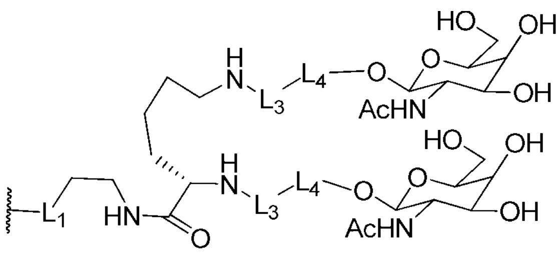
;

wherein Li is $-CH_2O-$ or $-CH_2O-CH_2CH_2O-$;
$L_3$ is $-(NHCH_2CH_2)_b-$, $-(NHCH_2CH_2CH_2)_b-$, or $-C(O)CH_2-$;
$L_4$ is $-(OCH_2CH_2)_c-$ or $-NHC(O)-(CH_2)_d-$;

wherein b=1, 2, 3, 4, or 5;
c = 1, 2, 3, 4 or 5;
d = 1, 2, 3, 4, 5, 6, 7, or 8;

L is -NHC(O)-;
L' is a bond or -C(O)NH-;
R' is H, a hydroxy-protecting group, or a solid support, wherein the hydroxy-protecting group is preferably -C(O)$CH_2CH_2C(O)OH$ or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

In some specific embodiments, wherein:

Q is independently H,

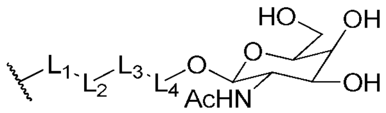

,

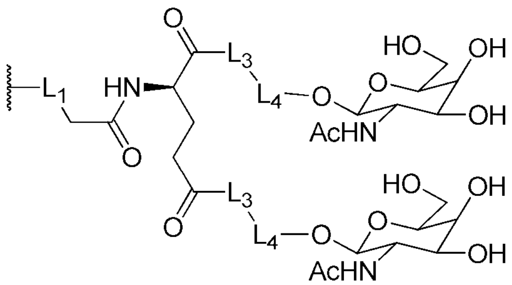

,

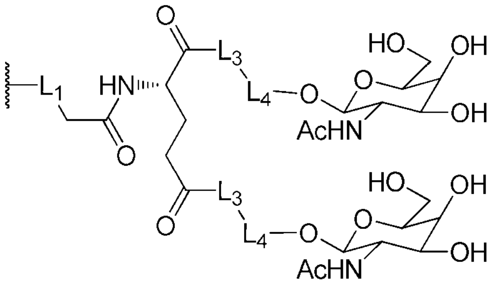

,

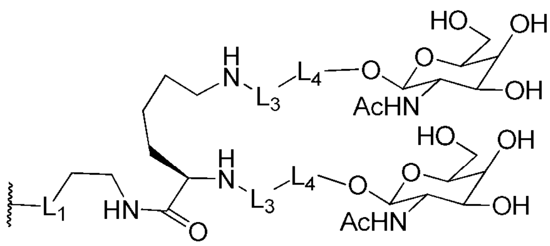

, or

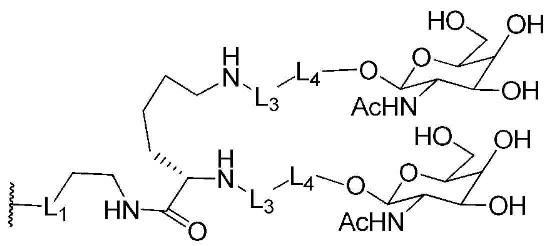

;

wherein $L_1$ is $-CH_2-$, -C(O)-, $-CH_2O-$, $-CH_2O-CH_2CH_2O-$, or $-NHC(O)-(CH_2NHC(O))_a-$;
$L_2$ is a bond;
$L_3$ is $-(NHCH_2CH_2)_b-$, $-(NHCH_2CH_2CH_2)_b-$, or $-C(O)CH_2-$;
$L_4$ is $-(OCH_2CH_2)_c-$ or $-NHC(O)-(CH_2)_d-$;

wherein a = 0, 1, 2, or 3;
b = 1, 2, 3, 4 or 5;
c = 1, 2, 3, 4 or 5;
d = 1, 2, 3, 4, 5, 6, 7, or 8;

L is $-CH_2O-$ or -NHC(O)-;
L' is a bond or -C(O)NH-;
$R_1$ and $R_2$ together form $-CH_2CH_2O-$ or $-CH_2CH(R)-O-$, and $R_3$ is H;
or $R_1$ and $R_3$ together form $-C_{1-2}$ alkylene-, and $R_2$ is H;
wherein R is -OR', $-CH_2OR'$, or $-CH_2CH_2OR'$; wherein R' is H, a hydroxy-protecting group, or a solid support, and the hydroxy-protecting group is preferably - $C(O)CH_2CH_2C(O)OH$ or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

In some embodiments, the conjugation group is as shown in formula (II'-2):

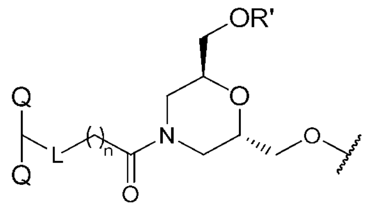
(II'-2)

wherein,

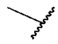

represents the point of attachment to dsRNA;
Q is independently

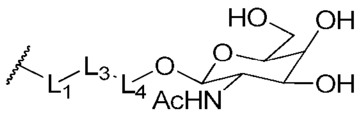
,
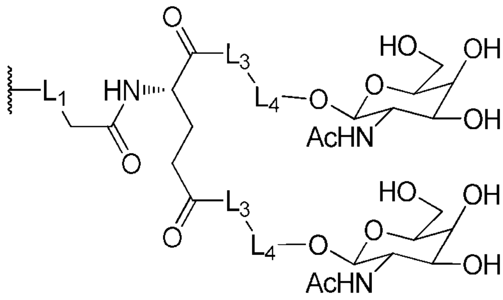
,

or

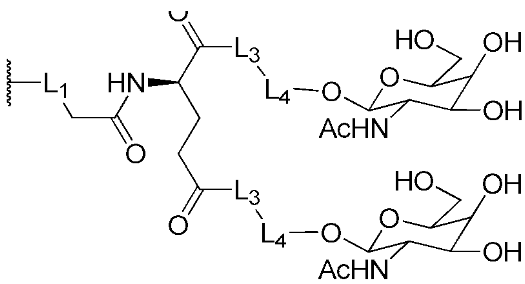
;

wherein $L_1$ is $-CH_2-$ or -C(O)-;
$L_3$ is $-(NHCH_2CH_2)_b-$;
$L_4$ is $-(OCH_2CH_2)_c-$;

wherein b=1, 2, 3, 4, or 5;
c = 1, 2, 3, 4 or 5;

L is $-CH_2O-$ or -NHC(O)-;
R' is H, a hydroxy-protecting group, or a solid support, wherein the hydroxy-protecting group is preferably -C(O) $CH_2CH_2C(O)OH$ or 4,4'-dimethoxytrityl;
n = 0, 1, 2, 3, 4, 5, 6, 7, 8, 9 or 10.

In some specific embodiments, wherein:

Q is independently H,

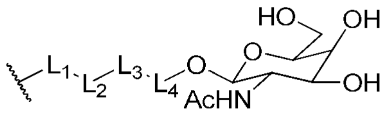,

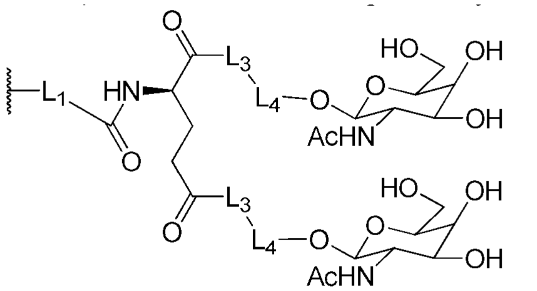, 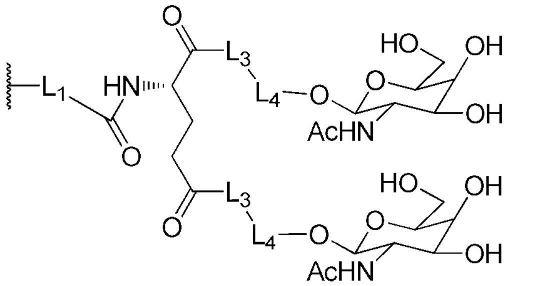,

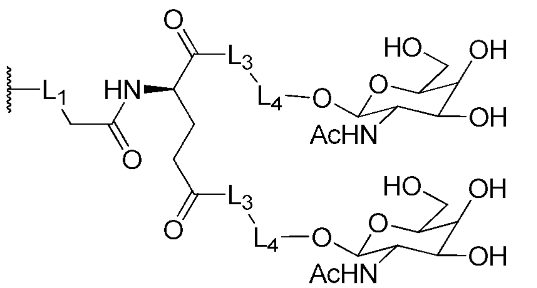, 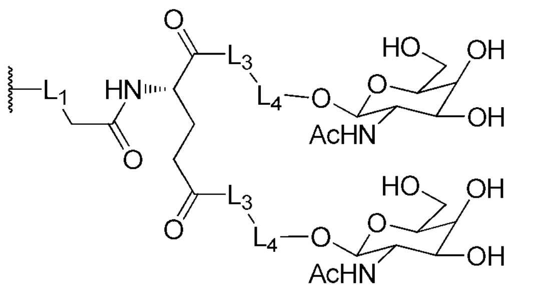,

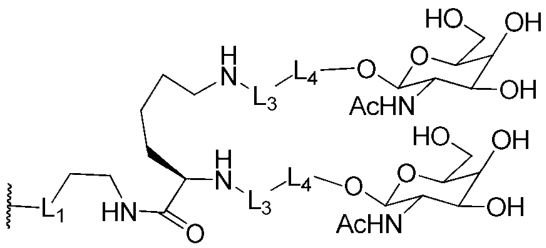, or 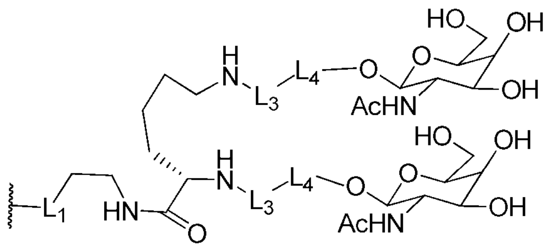;

wherein $L_1$ is a bond, $-CH_2-$, $-CH_2CH_2-$, -C(O)-, $-CH_2O-$, $-CH_2O-CH_2CH_2O-$, or - $NHC(O)-(CH_2NHC(O))_a-$;
$L_2$ is a bond or $-CH_2CH_2C(O)-$;
$L_3$ is a bond, $-(NHCH_2CH_2)_b-$, $-(NHCH_2CH_2CH_2)_b-$, or $-C(O)CH_2-$;
$L_4$ is $-(OCH_2CH_2)_c-$, $-(OCH_2CH_2CH_2)_c-$, $-(OCH_2CH_2CH_2CH_2)_c-$, - $(OCH_2CH_2CH_2CH_2CH_2)_c-$, or $-NHC(O)-(CH_2)_d-$;

wherein a = 0, 1, 2, or 3;
b = 1, 2, 3, 4 or 5;
c = 1, 2, 3, 4 or 5;
d = 1, 2, 3, 4, 5, 6, 7, or 8;

L is a bond, $-CH_2O-$, or -NHC(O)-;
L' is a bond, -C(O)NH-, -NHC(O)-, or $-O(CH_2CH_2O)_e-$;
wherein e is 1, 2, 3, 4, or 5;
T is a bond, $-CH_2-$, -M-, $-CH_2-M-$, or -C(O)-M-;
wherein M is

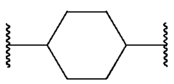, or 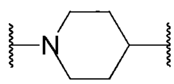;

$R_1$ and $R_2$ together form $-CH_2CH_2O-$ or $-CH_2CH(R)-O-$, and $R_3$ is H;

or $R_1$ and $R_3$ together form -$C_{1-2}$ alkylene-, and $R_2$ is H;
wherein R is -OR', -$CH_2$OR', or -$CH_2CH_2$OR'; wherein R' is H, a hydroxy-protecting group, or a solid support, and the hydroxy-protecting group is preferably - C(O)$CH_2CH_2$C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

In some specific embodiments, wherein,
T is -M-, -$CH_2$-M-, or -C(O)-M-, wherein M is

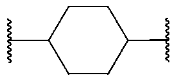
, or
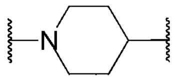
.

In some specific embodiments, wherein:

Q is independently H or

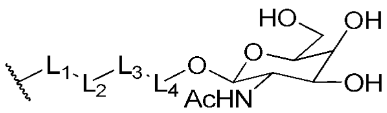
;

wherein Li is -$CH_2$O- or -NHC(O)-($CH_2$NHC(O))$_a$-;
$L_2$ is -$CH_2CH_2$C(O)-;
$L_3$ is -($NHCH_2CH_2$)$_b$- or -($NHCH_2CH_2CH_2$)$_b$-;
$L_4$ is -($OCH_2CH_2$)$_c$- or -NHC(O)-($CH_2$)$_d$-;

wherein a = 0, 1, 2, or 3;
b = 1, 2, 3, 4 or 5;
c = 1, 2, 3, 4 or 5;
d = 1, 2, 3, 4, 5, 6, 7, or 8;

L is a bond or -$CH_2$O-;
L' is a bond or -O($CH_2CH_2$O)$_e$-;
wherein e is 1, 2, 3, 4, or 5;
$R_1$ and $R_2$ together form -$CH_2CH_2$O- or -$CH_2$CH(R)-O-, and $R_3$ is H;
or $R_1$ and $R_3$ together form -$C_{1-2}$ alkylene-, and $R_2$ is H;
wherein R is -OR', -$CH_2$OR', or -$CH_2CH_2$OR'; wherein R' is H, a hydroxy-protecting group, or a solid support, and the hydroxy-protecting group is preferably - C(O)$CH_2CH_2$C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10;
wherein T is as defined in above embodiments.

In some embodiments, the conjugation group is as shown in formula (III'-1), formula (III'-2), or formula (III'-3):

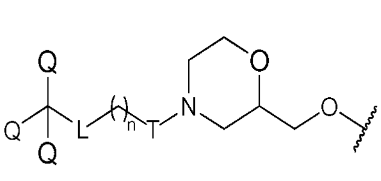
(III'-1),
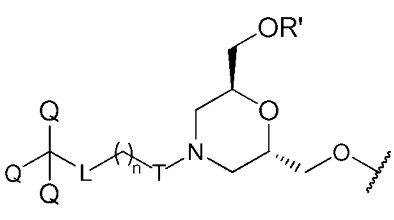
(III'-2),

or

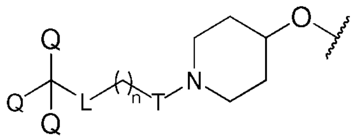

(III'-3)

wherein,

Q is

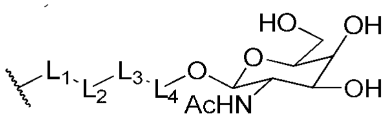

;

wherein $L_1$ is $-CH_2O-$ or -NHC(O)-;
$L_2$ is $-CH_2CH_2C(O)-$;
$L_3$ is $-(NHCH_2CH_2)_b-$ or $-(NHCH_2CH_2CH_2)_b-$;
$L_4$ is $-(OCH_2CH_2)_c-$ or $-NHC(O)-(CH_2)_d-$;

wherein b=1, 2, 3, 4, or 5;
c = 1, 2, 3, 4 or 5;
d = 1, 2, 3, 4, 5, 6, 7, or 8;

L is a bond or $-CH_2O-$;
wherein R' is H, a hydroxy-protecting group, or a solid support, wherein the hydroxy-protecting group is preferably $-C(O)CH_2CH_2C(O)OH$ or 4,4'-dimethoxytrityl;
n = 0, 1, 2, 3, 4, 5, 6, 7, 8, 9 or 10;
wherein T is as defined in above embodiments.

In some secific embodiments wherein:

Q is independently H,

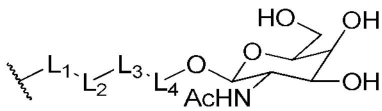

,

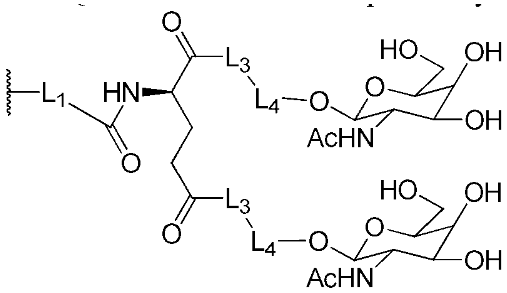

,

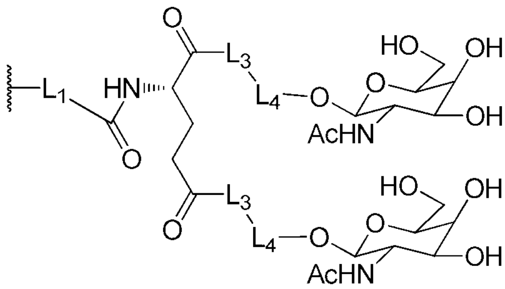

,

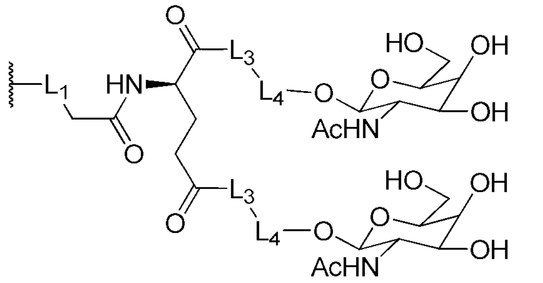
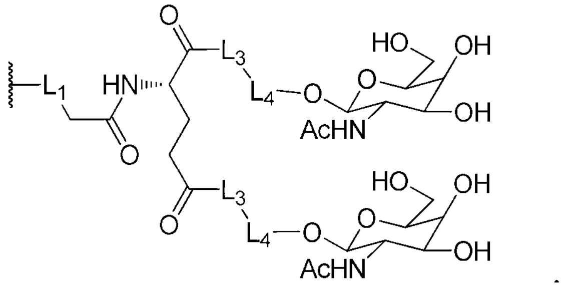
,
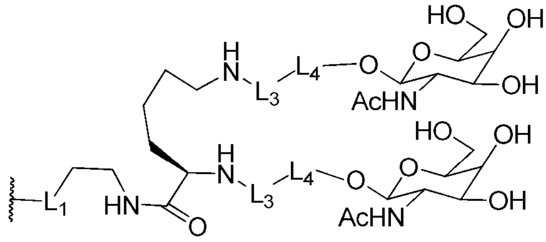
, or
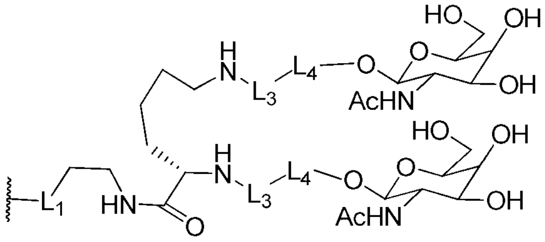
;

wherein $L_1$ is $-CH_2-$, $-CH_2O-$, or -C(O)-;
$L_2$ is a bond;
$L_3$ is $-(NHCH_2CH_2)_b-$, $-(NHCH_2CH_2CH_2)_b-$, or $-C(O)CH_2-$;
$L_4$ is $-(OCH_2CH_2)_c-$ or $-NHC(O)-(CH_2)_d-$;

wherein b=1, 2, 3, 4, or 5;
c = 1, 2, 3, 4 or 5;
d = 1, 2, 3, 4, 5, 6, 7, or 8;

L is a bond or -NHC(O)-;
L' is a bond;
$R_1$ and $R_2$ together form $-CH_2CH_2O-$ or $-CH_2CH(R)-O-$, and $R_3$ is H;
or $R_1$ and $R_3$ together form $-C_{1-2}$ alkylene-, and $R_2$ is H;
wherein R is -OR', $-CH_2OR'$, or $-CH_2CH_2OR'$; wherein R' is H, a hydroxy-protecting group, or a solid support, and the hydroxy-protecting group is preferably - $C(O)CH_2CH_2C(O)OH$ or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10;
wherein T is as defined in above embodiments.

In some embodiments, the conjugation group is as shown in formula (IV-1) or formula (IV-2):

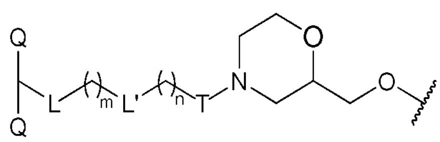
(IV-1) or
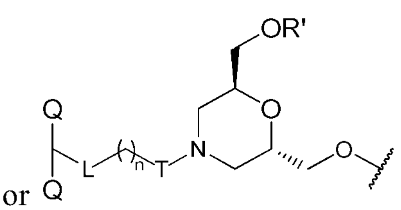
(IV-2)

wherein,

Q is independently

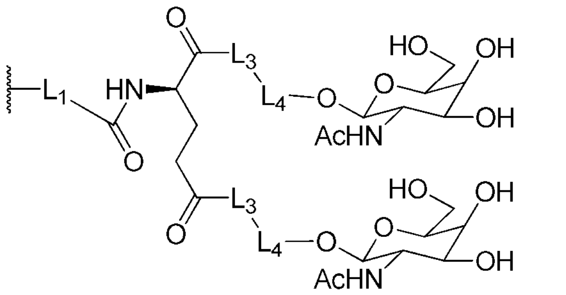

,

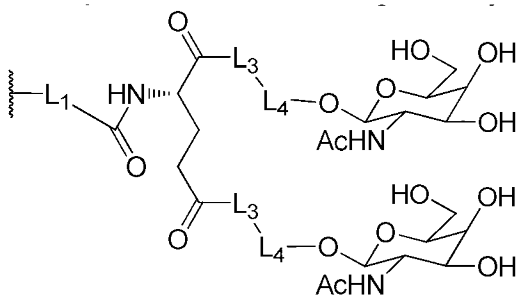

,

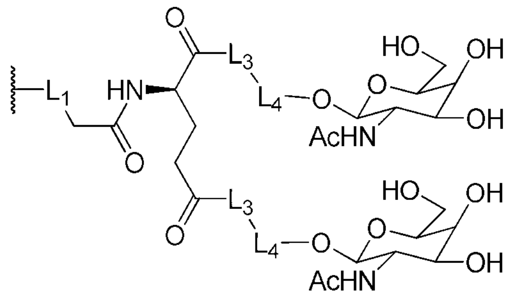

,

or

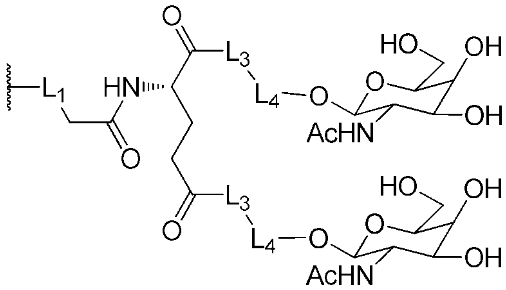

:

wherein $L_1$ is $-CH_2-$, $-CH_2O-$, or -C(O)-;
$L_3$ is $-(NHCH_2CH_2)_b-$, $-(NHCH_2CH_2CH_2)_b-$, or $-C(O)CH_2-$;
$L_4$ is $-(OCH_2CH_2)_c-$ or $-NHC(O)-(CH_2)_d-$;

wherein b=1, 2, 3, 4, or 5;
c = 1, 2, 3, 4 or 5;
d = 1, 2, 3, 4, 5, 6, 7, or 8;

L is a bond or -NHC(O)-;
L' is a bond;
wherein R' is H, a hydroxy-protecting group, or a solid support, wherein the hydroxy-protecting group is preferably $-C(O)CH_2CH_2C(O)OH$ or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10;
wherein T is as defined in the above embodiments.

In some specific embodiments, wherein:

Q is independently H,

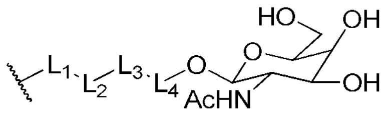

,

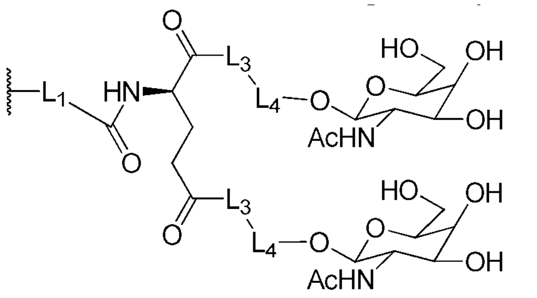

,

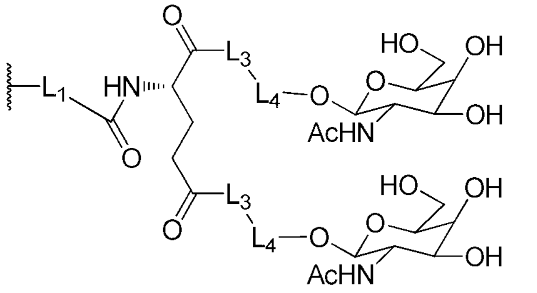

,

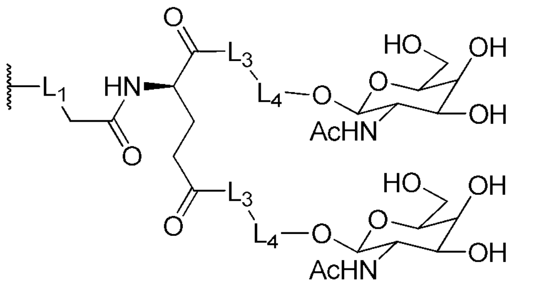

,

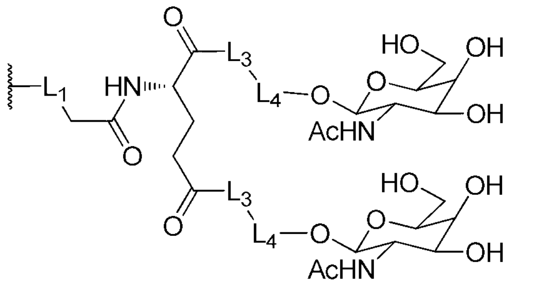

,

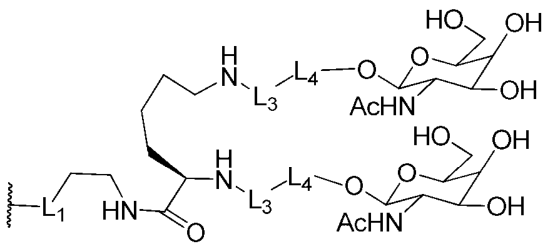

, or

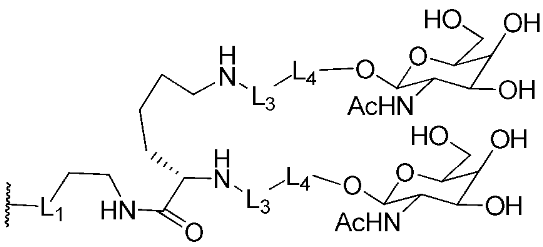

;

wherein $L_1$ is a bond, $-CH_2-$, $-CH_2CH_2-$, -C(O)-, $-CH_2O-$, $-CH_2O-CH_2CH_2O-$, or - $NHC(O)-(CH_2NHC(O))_a-$;

$L_2$ is a bond or $-CH_2CH_2C(O)-$;

$L_3$ is a bond, $-(NHCH_2CH_2)_b-$, $-(NHCH_2CH_2CH_2)_b-$, or $-C(O)CH_2-$;

$L_4$ is $-(OCH_2CH_2)_c-$, $-(OCH_2CH_2CH_2)_c-$, $-(OCH_2CH_2CH_2CH_2)_c-$, - $(OCH_2CH_2CH_2CH_2CH_2)_c-$, or $-NHC(O)-(CH_2)_d-$;

wherein a = 0, 1, 2, or 3;

b = 1, 2, 3, 4 or 5;

c = 1, 2, 3, 4 or 5;

d = 1, 2, 3, 4, 5, 6, 7, or 8;

L is a bond, $-CH_2O-$, or -NHC(O)-;

L' is $-O(CH_2CH_2O)_e-$;

wherein e is 1, 2, 3, 4, or 5;

T is a bond, $-CH_2-$, -C(O)-, -M-, $-CH_2-M-$, or -C(O)-M-;

wherein M is

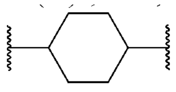

, or

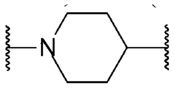

,;

$R_1$ and $R_2$ together form $-CH_2CH_2O-$ or $-CH_2CH(R)-O-$, and $R_3$ is H;

or $R_1$ and $R_3$ together form -$C_{1-2}$ alkylene-, and $R_2$ is H;
wherein R is -OR', -$CH_2OR'$, or -$CH_2CH_2OR'$; wherein R' is H, a hydroxy-protecting group, or a solid support, and the hydroxy-protecting group is preferably - $C(O)CH_2CH_2C(O)OH$ or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

In some preferred embodiments, the conjugation group is selected from Tables 1 and 2.

In some embodiments, the ligand targets an asialoglycoprotein receptor (ASGPR). In some embodiments, the ligand targets an asialoglycoprotein receptor (ASGPR) on a hepatocyte.

In some embodiments, the ligand has the following structure:

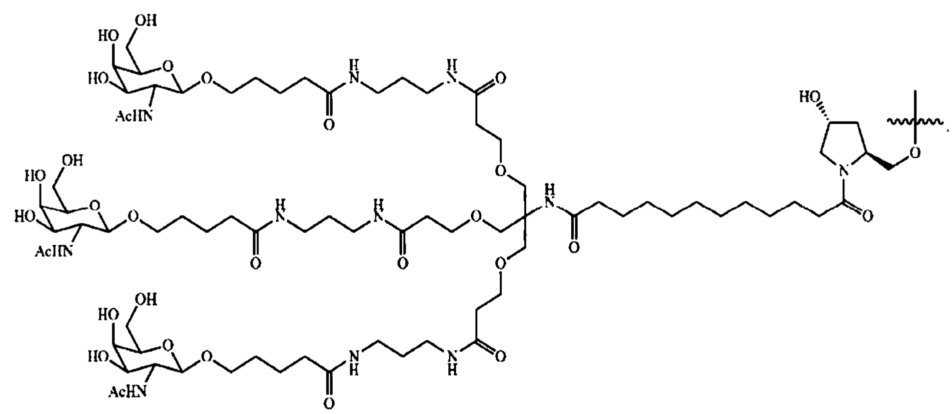

wherein

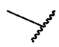

, represents the point of attachment to the sense strand (preferably, the 3' end of the sense strand) of the dsRNA via a phosphate group or a phosphorothioate group.

In some embodiments, the ligand has the following structure:

(NAG37) =

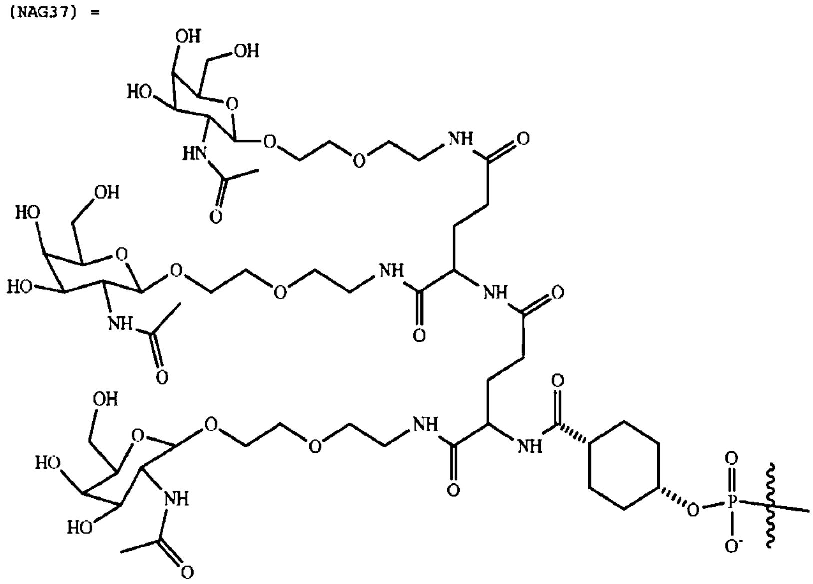

,

wherein

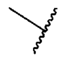

represents the point of attachment to the sense strand (preferably, the 3' end of the sense strand) of the dsRNA via a phosphate group or a phosphorothioate group.

In some embodiments, the ligand has the following structure:

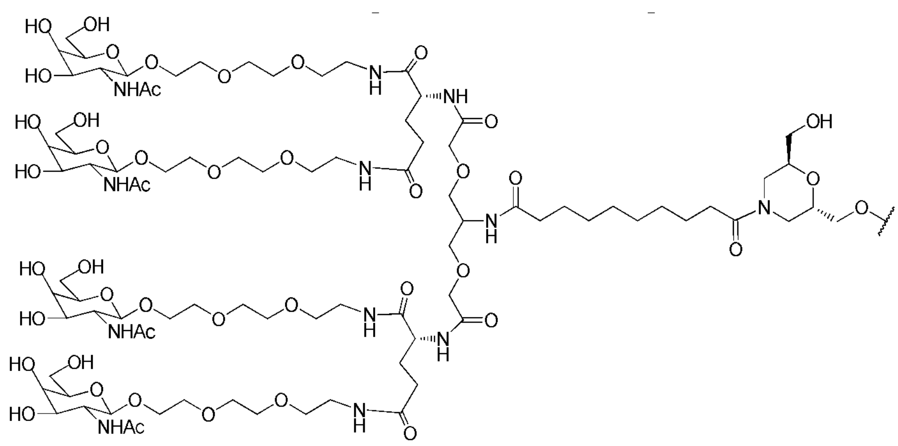

.

wherein

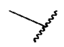

represents the point of attachment to the sense strand (preferably, the 3' end of the sense strand) of the dsRNA via a phosphate group or a phosphorothioate group.

In some embodiments, the ligand has the following structure:

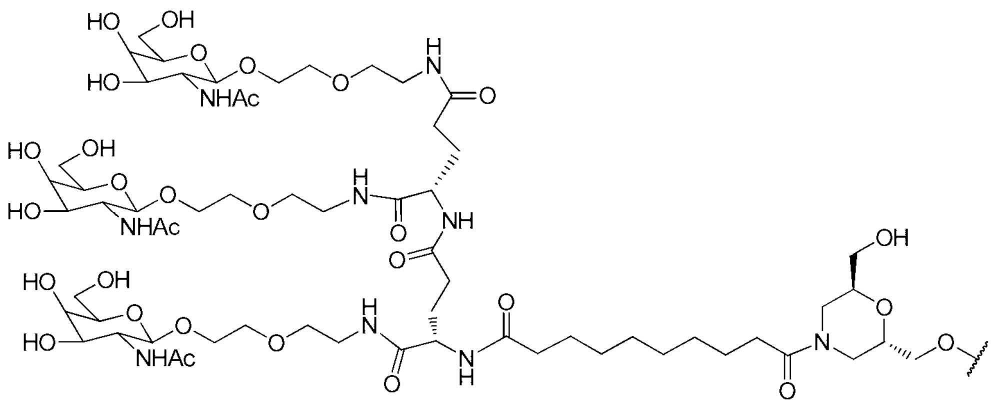

,

wherein

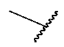

represents the point of attachment to the sense strand (preferably, the 3' end of the sense strand) of the dsRNA via a phosphate group or a phosphorothioate group.

IV. **Inhibition of APOC3 Gene Expression**

The dsRNA of the present invention is capable of inhibiting *CIDEB* gene expression by at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least

about 92%, at least about 93%, at least about 94%, at least about 95%, at least about 96%, at least about 97%, at least about 98%, or at least about 99%.

Inhibition of *CIDEB* gene expression may be manifested by a reduction in the levels of mRNA expressed by a first cell or population of cells (such cells may be present, for example, in a sample derived from a subject), wherein the *CIDEB* gene is transcribed and the cell or cells have been treated (e.g., by contacting the cell or cells with the dsRNA of the present invention, or by administering the dsRNA of the present invention to a subject in which the cells are present or previously present, such that the *CIDEB* gene expression is inhibited compared to a second cell or population of cells (one or more control cells) that is substantially identical to the first cell or population of cells but has not been so treated.

**In** a preferred embodiment, the inhibition is assessed by expressing the level of mRNA in treated cells as a percentage of the level of mRNA in control cells using the following formula. **In** some specific embodiments, the $2^{-\triangle\triangle Ct}$ value is calculated to compare the difference between the experimental group and the control group, where $\Delta\Delta Ct = [(Ct_{\text{target gene, experimental group}} - Ct_{\text{reference gene, experimental group}}) - (Ct_{\text{target gene, control group}} - Ct_{\text{reference gene, control group}})]$.

Alternatively, inhibition of *CIDEB* gene expression, e.g., CIDEB protein expression, may be assessed in terms of a decrease in parameters functionally related to *CIDEB* gene expression, e.g., lipid levels, cholesterol levels, such as LDLc levels. *CIDEB* gene silencing may be determined by any assay known in the art in any cell expressing CIDEB constitutively or by genomic engineering. The liver and kidney are the primary sites of CIDEB expression, and other significant sites for expression include the small intestine, white adipose tissue, and colon.

Inhibition of the expression of the CIDEB protein can be manifested by a reduction in the levels of CIDEB protein expressed by a cell or population of cells (e.g., levels of the protein expressed in a sample derived from a subject). As explained above with respect to the assessment of mRNA inhibition, the inhibition of protein expression levels in a treated cell or population of cells may similarly be expressed as a percentage of the level of the protein in a control cell or population of cells.

A control cell or population of cells that may be used to assess inhibition of *CIDEB* gene expression includes a cell or population of cells that has not been exposed to the dsRNA of the present invention. For example, the control cell or population of cells may be derived from an individual subject (e.g., a human or animal subject) prior to the treatment of the subject with the dsRNA.

## V. Cells

The present invention provides a cell comprising the dsRNA of the present invention.

## VI. **Pharmaceutical Compositions**

The present invention provides a pharmaceutical composition comprising the dsRNA or the cell disclosed herein, and optionally a pharmaceutically acceptable carrier or excipient.

As used herein, "pharmaceutically acceptable" refers to those compounds, materials, compositions, and/or dosage forms which, within the scope of sound medical judgment, are suitable for contact with tissues of human subjects and animal subjects without undue toxicity, irritation, allergic reactions, or other problems or complications, commensurate with a reasonable benefit/risk ratio.

As used herein, a pharmaceutically acceptable carrier refers to a pharmaceutical carrier that facilitates the administration of a dsRNA or a cell comprising the dsRNA to the human body and/or facilitates absorption or action thereof. For example: diluents, excipients such as water, and fillers such as starch and sucrose; binders such as cellulose derivatives, alginates, gelatin, and polyvinylpyrrolidone; wetting agents such as glycerin; disintegrants such as agar, calcium carbonate, and sodium bicarbonate; absorption accelerators such as quaternary ammonium compounds; surfactants such as cetyl alcohol; adsorption carriers such as kaolin and bentonite; lubricants such as talc, calcium/-magnesium stearate, and polyethylene glycol. Additional adjuvants such as flavoring agents and sweeteners can also be added to the composition.

A pharmaceutical composition of the present invention may comprise a pharmaceutically acceptable diluent or a sustained-release matrix into which the dsRNA or vector of the present invention is embedded.

The pharmaceutical composition of the present disclosure may comprise a drug delivery system for delivering dsRNA. The drug delivery systems of the present disclosure include, but are not limited to, nanoparticles (e.g., lipid nanoparticles, polymer-based nanoparticles), polymeric, PEG, or cationic delivery systems, polylactic acid (PLA) microspheres, poly(lactic-co-glycolic acid) (PLGA) microspheres, liposomes, micelles, inverse micelles, lipid cochleates, or lipid microtubules, Cholesterol, PEG lipids PEG-2000-C-DMG, PEG-2000-DMG (Moderna), ALC-0159, and DSPC.

In some embodiments, the siRNA in the pharmaceutical composition of the present invention may be comprised in a polymer or polymer-based nanoparticle.

In some specific embodiments, the polymer is a polymer based on a poly(lactic-co-glycolic acid), which is abbreviated as PLGA. In some specific embodiments, the PLGA-based polymer is engineered to contain a separate

cationic group.

In some specific embodiments, the polymer contains an amine group capable of becoming cationic, such as polyethyleneimine (PEI) and poly(L-lysine) (PLL), which is capable of forming a complex with the siRNA through electrostatic interaction and delivering the siRNA into a cell. In some embodiments, the PEG and PLL are chemically modified to improve in vivo potency and tolerability.

In some embodiments, the siRNA in the pharmaceutical composition of the present disclosure can be delivered via a cationic polymer poly(β-aminoester) (PBAE).

VII. Kit

The present invention provides a kit comprising the dsRNA or the cell of the present invention.

The present invention also provides a kit for using the dsRNA of the present invention and/or for performing the method of the present invention. Such a kit includes one or more of the dsRNAs or cells of the present invention, and may further include instructions for use. The instructions for inhibiting the expression of CIDEB in a cell by contacting the cell with the dsRNA of the present invention in an amount effective to inhibit CIDEB expression may be recorded in the instructions for use.

In the case where the dsRNA of the present invention is contacted with a cell *in vitro,* optionally, the kit of the present invention may further comprise means for contacting the cell with the dsRNA of the present invention (e.g., an injection device) or means for measuring inhibition of CIDEB (e.g., a device for measuring inhibition of CIDEB mRNA or protein). Such a device for measuring inhibition of CIDEB may comprise a device for obtaining a sample (e.g., a plasma sample) from a subject.

In the case where the dsRNA of the present invention, or a cell into which the dsRNA has been introduced *in vitro* is administered into the body, the kit of the present invention may optionally further comprise a device for administering the dsRNA or the cell of the present invention to a subject, or a device for determining a therapeutically effective amount or a prophylactically effective amount.

**VIII. Treatment Methods and Pharmaceutical** Uses

The present disclosure provides a method of reducing cell death-inducing DFFA-like effector B (CIDEB) in a subject, comprising a step of administering to the subject a dsRNA, a cell, or a pharmaceutical composition of the present disclosure.

The present disclosure provides a method of treating, preventing, inhibiting or alleviating a disease or disorder benefiting from reduced expression of cell death-inducing DFFA-like effector B (CIDEB) in a subject, comprising a step of administering to the subject a dsRNA, a cell, or a pharmaceutical composition of the present disclosure. The present disclosure also provides a method of treating, preventing, inhibiting or alleviating at least one symptom in a patient having a disease or disorder benefiting from reduced expression of cell death-inducing DFFA-like effector B (CIDEB).

In some embodiments, the disease or disorder benefiting from reduced expression of cell death-inducing DFFA-like effector B (CIDEB) is a CIDEB-related disease.

In some embodiments, the inventive method of reducing cell death-inducing DFFA-like effector B (CIDEB) in a subject, the inventive method of treating, preventing, inhibiting or alleviating a disease or disorder benefiting from reduced expression of cell death-inducing DFFA-like effector B (CIDEB) in a subject, or the inventive method of treating, preventing, inhibiting or alleviating at least one symptom in a patient having a disease or disorder benefiting from reduced expression of cell death-inducing DFFA-like effector B (CIDEB), comprises administering the dsRNA, cell, or pharmaceutical composition to the subject subcutaneously, locally, or intravenously. In some embodiments, the subject is a human patient.

The present invention also relates to the dsRNA, cell, or pharmaceutical composition of the present invention for use in treating a disease or symptom related to CIDEB expression in a subject.

The present invention also relates to use of the dsRNA, cell, or pharmaceutical composition of the present invention in the manufacture of a medicament for treating, preventing, or alleviating a disease or disorder benefiting from reduced expression of cell death-inducing DFFA-like effector B (CIDEB) in a subject. The present disclosure also relates to use of the dsRNA, cell, or pharmaceutical composition of the present disclosure in the manufacture of a medicament for reducing cell death-inducing DFFA-like effector B (CIDEB) in a subject. The present disclosure also relates to use of the dsRNA, cell, or pharmaceutical composition of the present disclosure for the manufacture of a medicament for treating, preventing, inhibiting or alleviating at least one symptom in a patient having a disease or disorder benefiting from reduced expression of cell death-inducing DFFA-like effector B (CIDEB).

Sequence

The RNA sequence provided by the present invention targets a human *CIDEB* gene (or a target gene, a target

mRNA sequence, or a target sequence). The target *CIDEB* mRNA sequence is the gene as shown in Genbank Accession No. NM_001393338.1.

Table 3. Nucleotide sequences targeting the sense and antisense strands of *CIDEB* mRNA

| SEQ ID NO: | Sequences for sense strands (5'->3') | SEQ ID NO: | Sequences for antisense strand (5'->3') |
|---|---|---|---|
| 1 | CACCAUGGAGUACCUCUCA | 62 | UGAGAGGUACUCCAUGGUGGA |
| 2 | GCUCUGAACCCCAGUGACU | 63 | AGUCACUGGGGUUCAGAGCUG |
| 3 | UACUCAGGUCAGUAUCUAA | 64 | UUAGAUACUGACCUGAGUAAG |
| 4 | AGGUCAGUAUCUAAUAUAA | 65 | UUAUAUUAGAUACUGACCUGA |
| 5 | GCCAGGAGCUGCUAGCCAA | 66 | UUGGCUAGCAGCUCCUGGCUG |
| 6 | CCUUUGACGUGUACAAGCA | 67 | UGCUUGUACACGUCAAAGGUG |
| 7 | CUCUAUGAGUUGUGACUUU | 68 | AAAGUCACAACUCAUAGAGUA |
| 8 | GCCAUAUGUUGCUGGGAAU | 69 | AUUCCCAGCAACAUAUGGCUC |
| 9 | CCGCCUCCAUUCCUACUAA | 70 | UUAGUAGGAAUGGAGGCGGUC |
| 10 | GCAAAGACUAUGACAGCAU | 71 | AUGCUGUCAUAGUCUUUGCAG |
| 11 | AGACUAUGACAGCAUCAAA | 72 | UUUGAUGCUGUCAUAGUCUUU |
| 12 | GACUAUGACAGCAUCAAAU | 73 | AUUUGAUGCUGUCAUAGUCUU |
| 13 | ACUAUGACAGCAUCAAAUU | 74 | AAUUUGAUGCUGUCAUAGUCU |
| 14 | CUAUGACAGCAUCAAAUUU | 75 | AAAUUUGAUGCUGUCAUAGUC |
| 15 | AUGACAGCAUCAAAUUUCA | 76 | UGAAAUUUGAUGCUGUCAUAG |
| 16 | UGACAGCAUCAAAUUUCAA | 77 | UUGAAAUUUGAUGCUGUCAUA |
| 17 | CAGACAGUACAGGCUAGAU | 78 | AUCUAGCCUGUACUGUCUGCA |
| 18 | AGACAGUACAGGCUAGAUA | 79 | UAUCUAGCCUGUACUGUCUGC |
| 19 | GACAGUACAGGCUAGAUAA | 80 | UUAUCUAGCCUGUACUGUCUG |
| 20 | AAACAUUUCCAAUAAAAAU | 81 | AUUUUUAUUGGAAAUGUUUUU |
| 21 | ACAUUUCCAAUAAAAAUAU | 82 | AUAUUUUUAUUGGAAAUGUUU |
| 22 | CUCUGAACCCCAGUGACUU | 83 | AAGUCACUGGGGUUCAGAGCU |
| 23 | GACCUUUCCGUGUCUGUGA | 84 | UCACAGACACGGAAAGGUCGC |
| 24 | CCUCACAUCCCAAGUCUAU | 85 | AUAGACUUGGGAUGUGAGGCG |
| 25 | CCCUAAACUCCCCAGCAUA | 86 | UAUGCUGGGGAGUUUAGGGAC |
| 26 | AUUUCCAAUAAAAAUAUCA | 87 | UGAUAUUUUUAUUGGAAAUGU |
| 27 | CCCUUACCUGAAGAAUGCU | 88 | AGCAUUCUUCAGGUAAGGGUA |
| 28 | UCACAUCCCAAGUCUAUAA | 89 | UUAUAGACUUGGGAUGUGAGG |
| 29 | CUUACCUGAAGAAUGCUGU | 90 | ACAGCAUUCUUCAGGUAAGGG |
| 30 | UAUGACAGCAUCAAAUUUA | 91 | UAAAUUUGAUGCUGUCAUAGU |
| 31 | GCCUGACUGCUGCUACAUA | 92 | UAUGUAGCAGCAGUCAGGCUG |
| 32 | UACCCUUACCUGAAGAAUA | 93 | UAUUCUUCAGGUAAGGGUAUA |
| 33 | CCUUACCUGAAGAAUGCUA | 94 | UAGCAUUCUUCAGGUAAGGGU |
| 34 | UAGAUAACCCACCCAAUUU | 95 | AAAUUGGGUGGGUUAUCUAGC |
| 35 | CUAAACUCCCCAGCAUACU | 96 | AGUAUGCUGGGGAGUUUAGGG |
| 36 | CAGCCUGACUGCUGCUACA | 97 | UGUAGCAGCAGUCAGGCUGGG |

(continued)

| SEQ ID NO: | Sequences for sense strands (5'->3') | SEQ ID NO: | Sequences for antisense strand (5'->3') |
|---|---|---|---|
| 37 | ACAAAAACAUUUCCAAUAA | 98 | UUAUUGGAAAUGUUUUUGUUA |
| 38 | ACUGCUGAAUGGAGUGCUA | 99 | UAGCACUCCAUUCAGCAGUAG |
| 39 | ACCUUUCCGUGUCUGUGAU | 100 | AUCACAGACACGGAAAGGUCG |
| 40 | GACUUACUCAGGUCAGUAU | 101 | AUACUGACCUGAGUAAGUCAC |
| 41 | CUAGAUAACCCACCCAAUU | 102 | AAUUGGGUGGGUUAUCUAGCC |
| 42 | ACCCCAGUGACUUACUCAA | 103 | UUGAGUAAGUCACUGGGGUUC |
| 43 | GUACCUCUCAGCUCUGAAA | 104 | UUUCAGAGCUGAGAGGUACUC |
| 44 | ACAUCCCAAGUCUAUACCA | 105 | UGGUAUAGACUUGGGAUGUGA |
| 45 | AGUACAGGCUAGAUAACCA | 106 | UGGUUAUCUAGCCUGUACUGU |
| 46 | CUUACUCAGGUCAGUAUCU | 107 | AGAUACUGACCUGAGUAAGUC |
| 47 | CUCAGGUCAGUAUCUAAUA | 108 | UAUUAGAUACUGACCUGAGUA |
| 48 | CCCCAGAAUCAUUCCAACA | 109 | UGUUGGAAUGAUUCUGGGGGC |
| 49 | CAGCAUAACGCCUCACAUA | 110 | UAUGUGAGGCGUUAUGCUGAA |
| 50 | AACAAAAACAUUUCCAAUA | 111 | UAUUGGAAAUGUUUUUGUUAG |
| 51 | CCUAAACUCCCCAGCAUAA | 112 | UUAUGCUGGGGAGUUUAGGGA |
| 52 | AAUAUAAGCUCGGAGUUUA | 113 | UAAACUCCGAGCUUAUAUUAG |
| 53 | UCCCUAAACUCCCCAGCAU | 114 | AUGCUGGGGAGUUUAGGGACA |
| 54 | CCCAGUGACUUACUCAGGU | 115 | ACCUGAGUAAGUCACUGGGGU |
| 55 | GUCAGUAUCUAAUAUAAGA | 116 | UCUUAUAUUAGAUACUGACCU |
| 56 | UACCUCUCAGCUCUGAACA | 117 | UGUUCAGAGCUGAGAGGUACU |
| 57 | UAAACUCCCCAGCAUACUA | 118 | UAGUAUGCUGGGGAGUUUAGG |
| 58 | GGUGUUGCAGUCUGGUCAA | 119 | UUGACCAGACUGCAACACCAU |
| 59 | GUGACUUACUCAGGUCAGU | 120 | ACUGACCUGAGUAAGUCACUG |
| 60 | UGUCAAAGCCACAUUCUAA | 121 | UUAGAAUGUGGCUUUGACAUU |
| 61 | GGACUUGGCCCAAAGAAAA | 122 | UUUUCUUUGGGCCAAGUCCUU |

**Tables 4 and 5 below show the modified RNA sequences used in the present invention, respectively.**

The meanings of the abbreviations used herein are as follows:

A. U, G, and C represent a natural adenine ribonucleotide, a uracil ribonucleotide, a guanine ribonucleotide, and a cytosine ribonucleotide, respectively.

d represents that the nucleotide immediately to the right is a deoxyribonucleotide (i.e., 2'-deoxy modification). For example, dA, dT, dG and dC represent adenine deoxyribonucleotide, a thymine deoxyribonucleotide, a guanine deoxyribonucleotide, and a cytosine deoxyribonucleotide, respectively.

m represents that the nucleotide immediately to the left is a 2'-$OCH_3$ modified nucleotide. For example, Am, Um, Gm, and Cm represent 2'-$OCH_3$ modified A, U, G, and C, respectively.

f represents that the left nucleotide is a 2'-fluoro modified nucleotide. For example, Af, Uf, Gf, and Cf represent 2'-fluoro modified A, U, G, and C, respectively.

"s" represents that two adjacent nucleotides to the left and right are linked by phosphorothioate.

"s-" represents that the nucleotide immediately to the left and the delivery moiety immediately to the right are linked via a phosphorothioate linkage.

VP represents that the nucleotide to the right is a vinylphosphonate modified nucleotide, which is well-known in the art. See, for example, PCT Publication Nos. WO2011139702, WO2013033230, and WO2019105419.

An "SCP modified nucleotide" refers to a modified nucleotide having the structure:

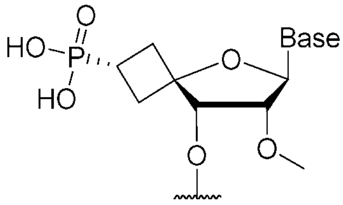

,

wherein Base is independently selected from H, a modified or an unmodified base, or a leaving group. Preferably, Base is an unmodified base, including an adenine base, a guanine base, a uracil base, and a cytosine base. In other embodiments, Base is a modified base.

L96 represents a GalNAc delivery moiety of the following structure, which is well known in the art, wherein

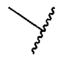

represents the point of attachment to dsRNA via a phosphate group or a phosphorothioate group. See, for example, PCT Publication Nos. WO2009073809 and WO2009082607.

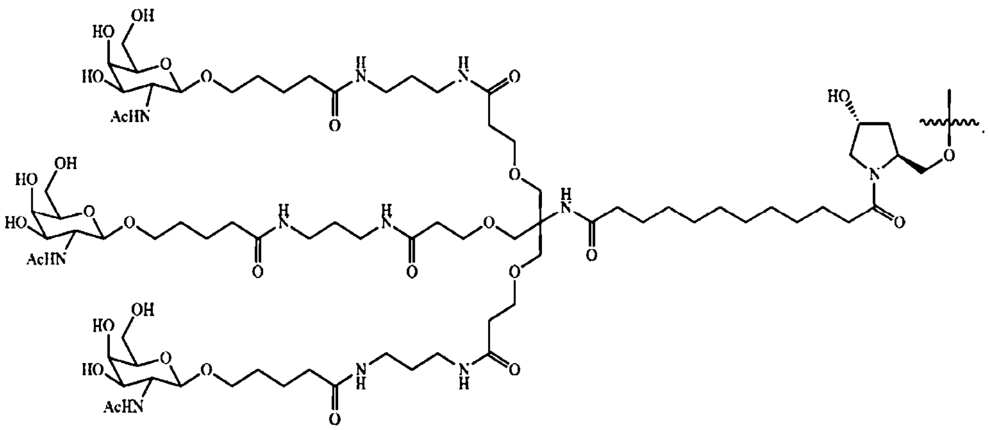

.

GL6 represents a GalNAc delivery moiety of the following structure, wherein

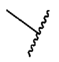

represents the point of attachment to dsRNA via a phosphate group or a phosphorothioate group:

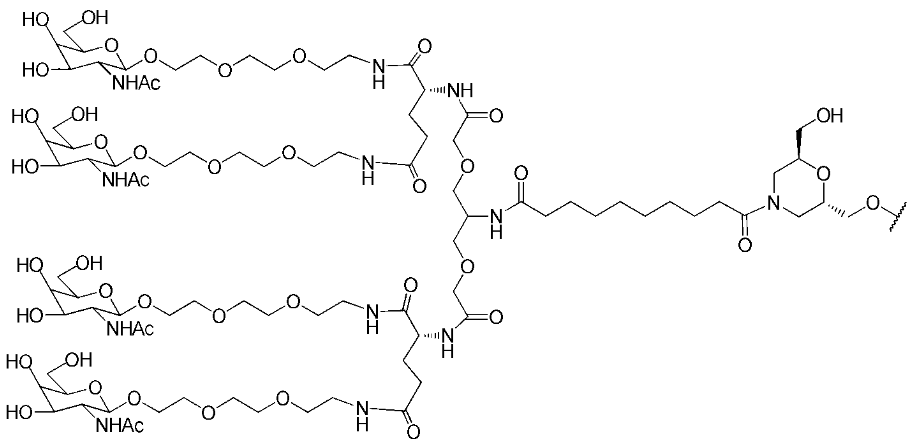

.

Table 4. Modified siRNA sense strand sequences targeting *CIDEB* mRNA

| Single Strand No. | SEQ ID NO: | Sequence (5'->3') |
|---|---|---|
| SR013956S | 123 | CmsAmsCmCmAmUmGfGfAfGmUmAmCmCmUmCmUmCmAm |
| SR013968S | 124 | GmsCmsUmCmUmGmAfAfCfCmCmCmAmGmUmGmAmCmUm |
| SR013969S | 125 | UmsAmsCmUmCmAmGfGfUfCmAmGmUmAmUmCmUmAmAm |
| SR013974S | 126 | AmsGmsGmUmCmAmGfUfAfUmCmUmAmAmUmAmUmAmAm |
| SR014011S | 127 | GmsCmsCmAmGmGmAfGfCfUmGmCmUmAmGmCmCmAmAm |
| SR014143S | 128 | CmsCmsUmUmUmGmAfCfGfUmGmUmAmCmAmAmGmCmAm |
| SR014205S | 129 | CmsUmsCmUmAmUmGfAfGfUmUmGmUmGmAmCmUmUmUm |
| SR014251S | 130 | GmsCmsCmAmUmAmUfGfUfUmGmCmUmGmGmGmAmAmUm |
| SR014267S | 131 | CmsCmsGmCmCmUmCfCfAfUmUmCmCmUmAmCmUmAmAm |
| SR014275S | 132 | GmsCmsAmAmAmGmAfCfUfAmUmGmAmCmAmGmCmAmUm |
| SR014279S | 133 | AmsGmsAmCmUmAmUfGfAfCmAmGmCmAmUmCmAmAmAm |
| SR014280S | 134 | GmsAmsCmUmAmUmGfAfCfAmGmCmAmUmCmAmAmAmUm |
| SR014281S | 135 | AmsCmsUmAmUmGmAfCfAfGmCmAmUmCmAmAmAmUmUm |
| SR014282S | 136 | CmsUmsAmUmGmAmCfAfGfCmAmUmCmAmAmAmUmUmUm |
| SR014283S | 137 | AmsUmsGmAmCmAmGfCfAfUmCmAmAmAmUmUmUmCmAm |
| SR014284S | 138 | UmsGmsAmCmAmGmCfAfUfCmAmAmAmUmUmUmCmAmAm |
| SR014307S | 139 | CmsAmsGmAmCmAmGfUfAfCmAmGmGmCmUmAmGmAmUm |
| SR014308S | 140 | AmsGmsAmCmAmGmUfAfCfAmGmGmCmUmAmGmAmUmAm |
| SR014309S | 141 | GmsAmsCmAmGmUmAfCfAfGmGmCmUmAmGmAmUmAmAm |
| SR014344S | 142 | AmsAmsAmCmAmUmUfUfCfCmAmAmUmAmAmAmAmAmUm |
| SR014346S | 143 | AmsCmsAmUmUmUmCfCfAfAmUmAmAmAmAmAmUmAmUm |
| SR014348S | 144 | CmsUmsCmUmGmAmAfCfCfCmCmAmGmUmGmAmCmUmUm |
| SR014350S | 145 | GmsAmsCmCmUmUmUfCfCfGmUmGmUmCmUmGmUmGmAm |
| SR014358S | 146 | CmsCmsUmCmAmCmAfUfCfCmCmAmAmGmUmCmUmAmUm |
| SR014360S | 147 | CmsCmsCmUmAmAmAfCfUfCmCmCmCmAmGmCmAmUmAm |
| SR014361S | 148 | AmsUmsUmUmCmCmAfAfUfAmAmAmAmAmUmAmUmCmAm |
| SR0152022 S | 149 | UmsAmsCmUmCmAmGfGfUfCmAmGmUmAmUmCmUmAmsAms-GL6 |
| SR0152023 S | 150 | CmsAmsCmCmAmUmGfGfAfGmUmAmCmCmUmCmUmCmsAms-GL6 |
| SR0152024 S | 151 | AmsGmsGmUmCmAmGfUfAfUmCmUmAmAmUmAmUmAmsAms-GL6 |
| SR0152025 S | 152 | CmsUmsCmUmAmUmGfAfGfUmUmGmUmGmAmCmUmUmsUms-GL6 |
| SR0152026 S | 153 | AmsGmsAmCmUmAmUfGfAfCmAmGmCmAmUmCmAmAmsAms-GL6 |
| SR0152027 S | 154 | GmsAmsCmUmAmUmGfAfCfAmGmCmAmUmCmAmAmAmsUms-GL6 |
| SR0152028 S | 155 | AmsCmsUmAmUmGmAfCfAfGmCmAmUmCmAmAmAmUmsUms-GL6 |
| SR0152029 S | 156 | CmsUmsAmUmGmAmCfAfGfCmAmUmCmAmAmAmUmUmsUms-GL6 |
| SR0152030 S | 157 | AmsUmsGmAmCmAmGfCfAfUmCmAmAmAmUmUmUmCmsAms-GL6 |
| SR0152031 S | 158 | UmsGmsAmCmAmGmCfAfUfCmAmAmAmUmUmUmCmAmsAms-GL6 |
| SR0152032 S | 159 | CmsAmsGmAmCmAmGfUfAfCmAmGmGmCmUmAmGmAmsUms-GL6 |
| SR0152033 S | 160 | AmsGmsAmCmAmGmUfAfCfAmGmGmCmUmAmGmAmUmsAms-GL6 |
| SR0152034 S | 161 | GmsAmsCmAmGmUmAfCfAfGmGmCmUmAmGmAmUmAmsAms-GL6 |

(continued)

| Single Strand No. | SEQ ID NO: | Sequence (5'->3') |
|---|---|---|
| SR0152035 S | 162 | AmsAmsAmCmAmUmUfUfCfCmAmAmUmAmAmAmAmAmsUms-GL6 |
| SR0152036 S | 163 | AmsCmsAmUmUmUmCfCfAfAmUmAmAmAmAmAmUmAmsUms-GL6 |
| SR0152037 S | 164 | CmsCmsCmUmAmAmAfCfUfCmCmCmCmAmGmCmAmUmsAms-GL6 |
| SR017698S | 165 | GmsAmsCmUmAmUmGfAfCfAmGmCmAmUmCmAmAmAmsAms-GL6 |
| SR017699S | 166 | AmsAmsAmCmAmUmUfUfCfCmAmAmUmAmAmAmAmAmsAms-GL6 |
| SR017700S | 167 | AmsCmsAmUmUmUmCfCfAfAmUmAmAmAmAmAmUmAmsAms-GL6 |
| SR017939S | 168 | GmsCmsUmCmUmGmAfAfCfCmCmCmAmGmUmGmAmCmsAms-GL6 |
| SR017940S | 169 | GmsCmsCmAmGmGmAfGfCfUmGmCmUmAmGmCmCmAmsAms-GL6 |
| SR017941S | 170 | CmsCmsUmUmUmGmAfCfGfUmGmUmAmCmAmAmGmCmsAms-GL6 |
| SR017942S | 171 | GmsCmsCmAmUmAmUfGfUfUmGmCmUmGmGmGmAmAmsAms-GL6 |
| SR017943S | 172 | CmsCmsGmCmCmUmCfCfAfUmUmCmCmUmAmCmUmAmsAms-GL6 |
| SR017944S | 173 | GmsCmsAmAmAmGmAfCfUfAmUmGmAmCmAmsAms-GL6 |
| SR017945S | 174 | CmsUmsCmUmGmAmAfCfCfCmCmAmGmUmGmAmCmUmsAms-GL6 |
| SR017946S | 175 | GmsAmsCmCmUmUmUfCfCfGmUmGmUmCmUmGmUmGmsAms-GL6 |
| SR017947S | 176 | CmsCmsUmCmAmCmAfUfCfCmCmAmAmGmUmCmUmAmsAms-GL6 |
| SR017948S | 177 | AmsUmsUmUmCmCmAfAfUfAmAmAmAmAmUmAmUmCmsAms-GL6 |
| SR017961S | 178 | AmsAmsAmAmAmCmAmUmUfUfCfCmAmAmUmAmAmAmAmAmsAm s-GL6 |
| SR017962S | 179 | AmsAmsAmCmAmUmUmUmCfCfAfAmUmAmAmAmAmAmUmAmsAm s-GL6 |
| SS1 | 180 | AmsCmsAmGmAmAmCfCfUfUmUmCmAmGmCmAmUmsAmsAm |
| SS2 | 181 | UmsGmsAmCmUmGmCfUfGfCmUmAmCmAmUmCmUmsAmsAm |
| SS3 | 182 | AmsGmsCmCmUmGmAfCfUfGmCmUmGmCmUmAmCmsAmsUm |
| SS4 | 183 | CmsCmsCmUmUmAmCfCfUfGmAmAmGmAmAmUmGmsCmsUm |
| SS5 | 184 | UmsCmsAmCmAmUmCfCfCfAmAmGmUmCmUmAmUmsAmsAm |
| SS6 | 185 | CmsUmsUmAmCmCmUfGfAfAmGmAmAmUmGmCmUmsGmsUm |
| SS7 | 186 | UmsAmsUmGmAmCmAfGfCfAmUmCmAmAmAmUmUmsUmsAm |
| SS8 | 187 | GmsCmsCmUmGmAmCfUfGfCmUmGmCmUmAmCmAmsUmsAm |
| SS9 | 188 | UmsAmsCmCmCmUmUfAfCfCmUmGmAmAmGmAmAmsUmsAm |
| SS10 | 189 | CmsCmsUmUmAmCmCfUfGfAmAmGmAmAmUmGmCmsUmsAm |
| SS11 | 190 | UmsAmsGmAmUmAmAfCfCfCmAmCmCmCmAmAmUmsUmsUm |
| SS12 | 191 | CmsUmsAmAmAmCmUfCfCfCmCmAmGmCmAmUmAmsCmsUm |
| SS13 | 192 | CmsAmsGmCmCmUmGfAfCfUmGmCmUmGmCmUmAmsCmsAm |
| SS14 | 193 | AmsCmsAmAmAmAmAfCfAfUmUmUmCmCmAmAmUmAmAm |
| SS15 | 194 | AmsCmsUmGmCmUmGfAfAfUmGmGmAmGmUmGmCmUmAm |
| SS16 | 195 | AmsCmsCmUmUmUmCfCfGfUmGmUmCmUmGmUmGmsAmsUm |
| SS17 | 196 | GmsAmsCmUmUmAmCfUfCfAmGmGmUmCmAmGmUmsAmsUm |
| SS18 | 197 | CmsUmsAmGmAmUmAfAfCfCmCmAmCmCmCmAmAmsUmsUm |
| SS19 | 198 | AmsCmsCmCmCmAmGfUfGfAmCmUmUmAmCmUmCmsAmsAm |
| SS20 | 199 | GmsUmsAmCmCmUmCfUfCfAmGmCmUmCmUmGmAmAmAm |
| SS21 | 200 | AmsCmsAmUmCmCmCfAfAfGmUmCmUmAmUmAmCmsCmsAm |

(continued)

| Single Strand No. | SEQ ID NO: | Sequence (5'->3') |
|---|---|---|
| SS22 | 201 | AmsGmsUmAmCmAmGfGfCfUmAmGmAmUmAmAmCmCmAm |
| SS23 | 202 | CmsUmsUmAmCmUmCfAfGfGmUmCmAmGmUmAmUmsCmsUm |
| SS24 | 203 | CmsUmsCmAmGmGmUfCfAfGmUmAmUmCmUmAmAmUmAm |
| SS25 | 204 | CmsCmsCmCmAmGmAfAfUfCmAmUmUmCmCmAmAmsCmsAm |
| SS26 | 205 | CmsAmsGmCmAmUmAfAfCfGmCmCmUmCmAmCmAmsUmsAm |
| SS27 | 206 | AmsAmsCmAmAmAmAfAfCfAmUmUmUmCmCmAmAmUmAm |
| SS28 | 207 | CmsCmsUmAmAmAmCfUfCfCmCmCmAmGmCmAmUmsAmsAm |
| SS29 | 208 | AmsAmsUmAmUmAmAfGfCfUmCmGmGmAmGmUmUmUmAm |
| SS30 | 209 | UmsCmsCmCmUmAmAfAfCfUmCmCmCmCmAmGmCmsAmsUm |
| SS31 | 210 | CmsCmsCmAmGmUmGfAfCfUmUmAmCmUmCmAmGmsGmsUm |
| SS32 | 211 | GmsUmsCmAmGmUmAfUfCfUmAmAmUmAmUmAmAmGmAm |
| SS33 | 212 | UmsAmsCmCmUmCmUfCfAfGmCmUmCmUmGmAmAmCmAm |
| SS34 | 213 | UmsAmsAmAmCmUmCfCfCfCmAmGmCmAmUmAmCmsUmsAm |
| SS35 | 214 | GmsGmsUmGmUmUmGfCfAfGmUmCmUmGmGmUmCmsAmsAm |
| SS36 | 215 | GmsUmsGmAmCmUmUfAfCfUmCmAmGmGmUmCmAmsGmsUm |
| SS37 | 216 | UmsGmsUmCmAmAmAfGfCfCmAmCmAmUmUmCmUmAmAm |
| SS38 | 217 | GmsGmsAmCmUmUmGfGfCfCmCmAmAmAmGmAmAmsAmsAm |
| SR018716S | 218 | AmsCmsAmGmAmAmCfCfUfUmUmCmAmGmCmAmUmAmsAms-GL6 |
| SR018717S | 219 | UmsGmsAmCmUmGmCfUfGfCmUmAmCmAmUmCmUmAmsAms-GL6 |
| SR018718S | 220 | AmsGmsCmCmUmGmAfCfUfGmCmUmGmCmUmAmCmAmsUms-GL6 |
| SR018719S | 221 | CmsCmsCmUmUmAmCfCfUfGmAmAmGmAmAmUmGmCmsUms-GL6 |
| SR018720S | 222 | UmsCmsAmCmAmUmCfCfCfAmAmGmUmCmUmAmUmAmsAms-GL6 |
| SR018721S | 223 | CmsUmsUmAmCmCmUfGfAfAmGmAmAmUmGmCmUmGmsUms-GL6 |
| SR018722S | 224 | UmsAmsUmGmAmCmAfGfCfAmUmCmAmAmAmUmUmUmsAms-GL6 |
| SR018723S | 225 | GmsCmsCmUmGmAmCfUfGfCmUmGmCmUmAmCmAmUmsAms-GL6 |
| SR018724S | 226 | UmsAmsCmCmCmUmUfAfCfCmUmGmAmAmGmAmAmUmsAms-GL6 |
| SR018725S | 227 | CmsCmsUmUmAmCmCfUfGfAmAmGmAmAmUmGmCmUmsAms-GL6 |
| SR018726S | 228 | UmsAmsGmAmUmAmAfCfCfCmAmCmCmCmAmAmUmUmsUms-GL6 |
| SR018727S | 229 | CmsUmsAmAmAmCmUfCfCfCmCmAmGmCmAmUmAmCmsUms-GL6 |
| SR018728S | 230 | CmsAmsGmCmCmUmGfAfCfUmGmCmUmGmCmUmAmCmsAms-GL6 |
| SR018729S | 231 | AmsCmsAmAmAmAmAfCfAfUmUmUmCmCmAmAmUmAmsAms-GL6 |
| SR018730S | 232 | AmsCmsUmGmCmUmGfAfAfUmGmGmAmGmUmGmCmUmsAms-GL6 |
| SR018731S | 233 | AmsCmsCmUmUmUmCfCfGfUmGmUmCmUmGmUmGmAmsUms-GL6 |
| SR018732S | 234 | GmsAmsCmUmUmAmCfUfCfAmGmGmUmCmAmGmUmAmsUms-GL6 |
| SR018733S | 235 | CmsUmsAmGmAmUmAfAfCfCmCmAmCmCmCmAmAmUmsUms-GL6 |
| SR018734S | 236 | AmsCmsCmCmCmAmGfUfGfAmCmUmUmAmCmUmCmAmsAms-GL6 |
| SR018735S | 237 | GmsUmsAmCmCmUmCfUfCfAmGmCmUmCmUmGmAmAmsAms-GL6 |
| SR018736S | 238 | AmsCmsAmUmCmCmCfAfAfGmUmCmUmAmUmAmCmCmsAms-GL6 |
| SR018737S | 239 | AmsGmsUmAmCmAmGfGfCfUmAmGmAmUmAmAmCmCmsAms-GL6 |

(continued)

| Single Strand No. | SEQ ID NO: | Sequence (5'->3') |
|---|---|---|
| SR018738S | 240 | CmsUmsUmAmCmUmCfAfGfGmUmCmAmGmUmAmUmCmsUms-GL6 |
| SR018739S | 241 | CmsUmsCmAmGmGmUfCfAfGmUmAmUmCmUmAmAmUmsAms-GL6 |
| SR018740S | 242 | CmsCmsCmCmAmGmAfAfUfCmAmUmUmCmCmAmAmCmsAms-GL6 |
| SR018741S | 243 | CmsAmsGmCmAmUmAfAfCfGmCmCmUmCmAmCmAmUmsAms-GL6 |
| SR018742S | 244 | AmsAmsCmAmAmAmAfAfCfAmUmUmUmCmCmAmAmUmsAms-GL6 |
| SR018743S | 245 | CmsCmsUmAmAmAmCfUfCfCmCmCmAmGmCmAmUmAmsAms-GL6 |
| SR018744S | 246 | AmsAmsUmAmUmAmAfGfCfUmCmGmGmAmGmUmUmUmsAms-GL6 |
| SR018745S | 247 | UmsCmsCmCmUmAmAfAfCfUmCmCmCmCmAmGmCmAmsUms-GL6 |
| SR018746S | 248 | CmsCmsCmAmGmUmGfAfCfUmUmAmCmUmCmAmGmGmsUms-GL6 |
| SR018747S | 249 | GmsUmsCmAmGmUmAfUfCfUmAmAmUmAmUmAmAmGmsAms-GL6 |
| SR018748S | 250 | UmsAmsCmCmUmCmUfCfAfGmCmUmCmUmGmAmAmCmsAms-GL6 |
| SR018749S | 251 | UmsAmsAmAmCmUmCfCfCfCmAmGmCmAmUmAmCmUmsAms-GL6 |
| SR018750S | 252 | GmsGmsUmGmUmUmGfCfAfGmUmCmUmGmGmUmCmAmsAms-GL6 |
| SR018789S | 253 | GmsUmsGmAmCmUmUfAfCfUmCmAmGmGmUmCmAmGmsUms-GL6 |
| SR018790S | 254 | UmsGmsUmCmAmAmAfGfCfCmAmCmAmUmUmCmUmAmsAms-GL6 |
| SR018791S | 255 | GmsGmsAmCmUmUmGfGfCfCmCmAmAmAmGmAmAmAmsAms-GL6 |
| SR015209S | 256 | CmsUmsAmUmGmAmCmAmGfCfAfUmCmAmAmAmUmUmUmCmUm-L96 |

Table 5. Modified siRNA antisense strand sequences targeting *CIDEB* mRNA

| Single Strand No. | SE Q ID NO: | Sequence (5'->3') |
|---|---|---|
| SR014433A | 257 | UmsGfsAmGfAmGfGmUfAmCfUmCfCmAfUmGfGmUfGmsGfsAm |
| SR014445A | 258 | AmsGfsUmCfAmCfUmGfGmGfGmUfUmCfAmGfAmGfCmsUfsGm |
| SR014446A | 259 | UmsUfsAmGfAmUfAmCfUmGfAmCfCmUfGmAfGmUfAmsAfsGm |
| SR014451A | 260 | UmsUfsAmUfAmUfUmAfGmAfUmAfCmUfGmAfCmCfUmsGfsAm |
| SR014488A | 261 | UmsUfsGmGfCmUfAmGfCmAfGmCfUmCfCmUfGmGfCmsUfsGm |
| SR014620A | 262 | UmsGfsCmUfUmGfUmAfCmAfCmGfUmCfAmAfAmGfGmsUfsGm |
| SR014682A | 263 | AmsAfsAmGfUmCfAmCfAmAfCmUfCmAfUmAfGmAfGmsUfsAm |
| SR014728A | 264 | AmsUfsUmCfCmCfAmGfCmAfAmCfAmUfAmUfGmGfCmsUfsCm |
| SR014744A | 265 | UmsUfsAmGfUmAfGmGfAmAfUmGfGmAfGmGfCmGfGmsUfsCm |
| SR014752A | 266 | AmsUfsGmCfUmGfUmCfAmUfAmGfUmCfUmUfUmGfCmsAfsGm |
| SR014756A | 267 | UmsUfsUmGfAmUfGmCfUmGfUmCfAmUfAmGfUmCfUmsUfsUm |
| SR014757A | 268 | AmsUfsUmUfGmAfUmGfCmUfGmUfCmAfUmAfGmUfCmsUfsUm |
| SR014758A | 269 | AmsAfsUmUfUmGfAmUfGmCfUmGfUmCfAmUfAmGfUmsCfsUm |
| SR014759A | 270 | AmsAfsAmUfUmUfGmAfUmGfCmUfGmUfCmAfUmAfGmsUfsCm |
| SR014760A | 271 | UmsGfsAmAfAmUfUmUfGmAfUmGfCmUfGmUfCmAfUmsAfsGm |
| SR014761A | 272 | UmsUfsGmAfAmAfUmUfUmGfAmUfGmCfUmGfUmCfAmsUfsAm |
| SR014784A | 273 | AmsUfsCmUfAmGfCmCfUmGfUmAfCmUfGmUfCmUfGmsCfsAm |
| SR014785A | 274 | UmsAfsUmCfUmAfGmCfCmUfGmUfAmCfUmGfUmCfUmsGfsCm |
| SR014786A | 275 | UmsUfsAmUfCmUfAmGfCmCfUmGfUmAfCmUfGmUfCmsUfsGm |

(continued)

| Single Strand No. | SE Q ID NO: | Sequence (5'->3') |
|---|---|---|
| SR014821A | 276 | AmsUfsUmUfUmUfAmUfUmGfGmAfAmAfUmGfUmUfUmsUfsUm |
| SR014823A | 277 | AmsUfsAmUfUmUfUmUfAmUfUmGfGmAfAmAfUmGfUmsUfsUm |
| SR014825A | 278 | AmsAfsGmUfCmAfCmUfGmGfGmGfUmUfCmAfGmAfGmsCfsUm |
| SR014827A | 279 | UmsCfsAmCfAmGfAmCfAmCfGmGfAmAfAmGfGmUfCmsGfsCm |
| SR014835A | 280 | AmsUfsAmGfAmCfUmUfGmGfGmAfUmGfUmGfAmGfGmsCfsGm |
| SR014837A | 281 | UmsAfsUmGfCmUfGmGfGmGfAmGfUmUfUmAfGmGfGmsAfsCm |
| SR014838A | 282 | UmsGfsAmUfAmUfUmUfUmUfAmUfUmGfGmAfAmAfUmsGfsUm |
| SR017701A | 283 | (SCP-U)sdTsAmGmdAUmdACmUmGmAmdCCmUfGmAmGmUmAmsAmsGm |
| SR017702A | 284 | (SCP-U)sdTsAmUmdAUmdTAmGmAmUmdACmUfGmAmCmCmUmsGmsAm |
| SR017703A | 285 | (SCP-U)sdTsUmGmdAUmdGCmUmGmUmdCAmUfAmGmUmCmUmsUmsUm |
| SR017704A | 286 | (SCP-U)sdTsUmUmdGAmdTGmCmUmGmdTCmAfUmAmGmUmCmsUmsUm |
| SR017705A | 287 | (SCP-U)sdGsAmAmdAUmdTUmGmAmUmdGCmUfGmUmCmAmUmsAmsGm |
| SR017706A | 288 | (SCP-U)sdTsGmAmdAAmdTUmUmGmAmdTGmCfUmGmUmCmAmsUmsAm |
| SR017707A | 289 | (SCP-U)sdAsUmCmdTAmdGCmCmUmGmdTAmCfUmGmUmCmUmsGmsCm |
| SR017708A | 290 | (SCP-U)sdTsAmUmdCUmdAGmCmCmUmdGUmAfCmUmGmUmCmsUmsGm |
| SR017709A | 291 | (SCP-U)sdTsUmUmdTUmdAUmUmGmGmdAAmAfUmGmUmUmUmsUmsUm |
| SR017710A | 292 | (SCP-U)sdTsAmUmdTUmdTUmAmUmUmdGGmAfAmAmUmGmUmsUmsUm |
| SR017949A | 293 | (SCP-U)sdGsUmCmdACmdTGmGmGmGmdTUmCfAmGmAmGmCmsUmsGm |
| SR017950A | 294 | (SCP-U)sdTsGmGmdCUmdAGmCmAmGmdCUmCfCmUmGmGmCmsUmsGm |
| SR017951A | 295 | (SCP-U)sdGsCmUmdTGmdTAmCmAmCmdGUmCfAmAmAmGmGmsUmsGm |
| SR017952A | 296 | (SCP-U)sdTsUmCmdCCmdAGmCmAmAmdCAmUfAmUmGmGmCmsUmsCm |
| SR017953A | 297 | (SCP-U)sdTsAmGmdTAmdGGmAmAmUmdGGmAfGmGmCmGmGmsUmsCm |
| SR017954A | 298 | (SCP-U)sdTsGmCmdTGmdTCmAmUmAmdGUmCfUmUmUmGmCmsAmsGm |
| SR017955A | 299 | (SCP-U)sdAsGmUmdCAmdCUmGmGmGmdGUmUfCmAmGmAmGmsCmsUm |
| SR017956A | 300 | (SCP-U)sdCsAmCmdAGmdACmAmCmGmdGAmAfAmGmGmUmCmsGmsCm |
| SR017957A | 301 | (SCP-U)sdTsAmGmdACmdTUmGmGmGmdAUmGfUmGmAmGmGmsCmsGm |
| SR017958A | 302 | (SCP-U)sdGsAmUmdAUmdTUmUmUmAmdTUmGfGmAmAmAmUmsGmsUm |
| SR017959A | 303 | (SCP-U)sUfsUmUfUmUfAmUfUmGfGmAfAmAfUmGfUmUfUmUmUmsGmsUm |
| SR017960A | 304 | (SCP-U)sUfsAmUfUmUfUmUfAmUfUmGfGmAfAmAfUmGfUmUmUmsUmsUm |
| AS1 | 305 | UmsUfsAmUfGmCfUmGfAmAfAmGfGmUfUmCfUmGfUmsCmsAm |
| AS2 | 306 | UmsUfsAmGfAmUfGmUfAmGfCmAfGmCfAmGfUmCfAmsGmsGm |
| AS3 | 307 | AmsUfsGmUfAmGfCmAfGmCfAmGfUmCfAmGfGmCfUmsGmsGm |
| AS4 | 308 | AmsGfsCmAfUmUfCmUfUmCfAmGfGmUfAmAfGmGfGmsUmsAm |
| AS5 | 309 | UmsUfsAmUfAmGfAmCfUmUfGmGfGmAfUmGfUmGfAmsGmsGm |
| AS6 | 310 | AmsCfsAmGfCmAfUmUfCmUfUmCfAmGfGmUfAmAfGmsGmsGm |
| AS7 | 311 | UmsAfsAmAfUmUfUmGfAmUfGmCfUmGfUmCfAmUfAmsGmsUm |
| AS8 | 312 | UmsAfsUmGfUmAfGmCfAmGfCmAfGmUfCmAfGmGfCmsUmsGm |
| AS9 | 313 | UmsAfsUmUfCmUfUmCfAmGfGmUfAmAfGmGfGmUfAmsUmsAm |
| AS10 | 314 | UmsAfsGmCfAmUfUmCfUmUfCmAfGmGfUmAfAmGfGmsGmsUm |

(continued)

| Single Strand No. | SE Q ID NO: | Sequence (5'->3') |
|---|---|---|
| AS11 | 315 | AmsAfsAmUfUmGfGmGfUmGfGmGfUmUfAmUfCmUfAmsGmsCm |
| AS12 | 316 | AmsGfsUmAfUmGfCmUfGmGfGmGfAmGfUmUfUmAfGmsGmsGm |
| AS13 | 317 | UmsGfsUmAfGmCfAmGfCmAfGmUfCmAfGmGfCmUfGmsGmsGm |
| AS14 | 318 | UmsUfsAmUfUmGfGmAfAmAfUmGfUmUfUmUfUmGfUmsUfsAm |
| AS15 | 319 | UmsAfsGmCfAmCfUmCfCmAfUmUfCmAfGmCfAmGfUmsAfsGm |
| AS16 | 320 | AmsUfsCmAfCmAfGmAfCmAfCmGfGmAfAmAfGmGfUmsCmsGm |
| AS17 | 321 | AmsUfsAmCfUmGfAmCfCmUfGmAfGmUfAmAfGmUfCmsAmsCm |
| AS18 | 322 | AmsAfsUmUfGmGfGmUfGmGfGmUfUmAfUmCfUmAfGmsCmsCm |
| AS19 | 323 | UmsUfsGmAfGmUfAmAfGmUfCmAfCmUfGmGfGmGfUmsUmsCm |
| AS20 | 324 | UmsUfsUmCfAmGfAmGfCmUfGmAfGmAfGmGfUmAfCmsUfsCm |
| AS21 | 325 | UmsGfsGmUfAmUfAmGfAmCfUmUfGmGfGmAfUmGfUmsGmsAm |
| AS22 | 326 | UmsGfsGmUfUmAfUmCfUmAfGmCfCmUfGmUfAmCfUmsGfsUm |
| AS23 | 327 | AmsGfsAmUfAmCfUmGfAmCfCmUfGmAfGmUfAmAfGmsUmsCm |
| AS24 | 328 | UmsAfsUmUfAmGfAmUfAmCfUmGfAmCfCmUfGmAfGmsUfsAm |
| AS25 | 329 | UmsGfsUmUfGmGfAmAfUmGfAmUfUmCfUmGfGmGfGmsGmsCm |
| AS26 | 330 | UmsAfsUmGfUmGfAmGfGmCfGmUfUmAfUmGfCmUfGmsAmsAm |
| AS27 | 331 | UmsAfsUmUfGmGfAmAfAmUfGmUfUmUfUmUfGmUfUmsAfsGm |
| AS28 | 332 | UmsUfsAmUfGmCfUmGfGmGfGmAfGmUfUmUfAmGfGmsGmsAm |
| AS29 | 333 | UmsAfsAmAfCmUfCmCfGmAfGmCfUmUfAmUfAmUfUmsAfsGm |
| AS30 | 334 | AmsUfsGmCfUmGfGmGfGmAfGmUfUmUfAmGfGmGfAmsCmsAm |
| AS31 | 335 | AmsCfsCmUfGmAfGmUfAmAfGmUfCmAfCmUfGmGfGmsGmsUm |
| AS32 | 336 | UmsCfsUmUfAmUfAmUfUmAfGmAfUmAfCmUfGmAfCmsCfsUm |
| AS33 | 337 | UmsGfsUmUfCmAfGmAfGmCfUmGfAmGfAmGfGmUfAmsCfsUm |
| AS34 | 338 | UmsAfsGmUfAmUfGmCfUmGfGmGfGmAfGmUfUmUfAmsGmsGm |
| AS35 | 339 | UmsUfsGmAfCmCfAmGfAmCfUmGfCmAfAmCfAmCfCmsAmsUm |
| AS36 | 340 | AmsCfsUmGfAmCfCmUfGmAfGmUfAmAfGmUfCmAfCmsUmsGm |
| AS37 | 341 | UmsUfsAmGfAmAfUmGfUmGfGmCfUmUfUmGfAmCfAmsUfsUm |
| AS38 | 342 | UmsUfsUmUfCmUfUmUfGmGfGmCfCmAfAmGfUmCfCmsUmsUm |
| SR018751A | 343 | UmsUfsAmUfGmCfUmGfAmAfAmGfGmUfUmCfUmGfUmsCmsAm |
| SR018752A | 344 | UmsUfsAmGfAmUfGmUfAmGfCmAfGmCfAmGfUmCfAmsGmsGm |
| SR018753A | 345 | AmsUfsGmUfAmGfCmAfGmCfAmGfUmCfAmGfGmCfUmsGmsGm |
| SR018754A | 346 | AmsGfsCmAfUmUfCmUfUmCfAmGfGmUfAmAfGmGfGmsUmsAm |
| SR018755A | 347 | UmsUfsAmUfAmGfAmCfUmUfGmGfGmAfUmGfUmGfAmsGmsGm |
| SR018756A | 348 | AmsCfsAmGfCmAfUmUfCmUfUmCfAmGfGmUfAmAfGmsGmsGm |
| SR018757A | 349 | UmsAfsAmAfUmUfUmGfAmUfGmCfUmGfUmCfAmUfAmsGmsUm |
| SR018758A | 350 | UmsAfsUmGfUmAfGmCfAmGfCmAfGmUfCmAfGmGfCmsUmsGm |
| SR018759A | 351 | UmsAfsUmUfCmUfUmCfAmGfGmUfAmAfGmGfGmUfAmsUmsAm |
| SR018760A | 352 | UmsAfsGmCfAmUfUmCfUmUfCmAfGmGfUmAfAmGfGmsGmsUm |
| SR018761A | 353 | AmsAfsAmUfUmGfGmGfUmGfGmGfUmUfAmUfCmUfAmsGmsCm |

(continued)

| Single Strand No. | SE Q ID NO: | Sequence (5'->3') |
|---|---|---|
| SR018762A | 354 | AmsGfsUmAfUmGfCmUfGmGfGmGfAmGfUmUfUmAfGmsGmsGm |
| SR018763A | 355 | UmsGfsUmAfGmCfAmGfCmAfGmUfCmAfGmGfCmUfGmsGmsGm |
| SR018764A | 356 | UmsUfsAmUfUmGfGmAfAmAfUmGfUmUfUmUfUmGfUmsUmsAm |
| SR018765A | 357 | UmsAfsGmCfAmCfUmCfCmAfUmUfCmAfGmCfAmGfUmsAmsGm |
| SR018766A | 358 | AmsUfsCmAfCmAfGmAfCmAfCmGfGmAfAmAfGmGfUmsCmsGm |
| SR018767A | 359 | AmsUfsAmCfUmGfAmCfCmUfGmAfGmUfAmAfGmUfCmsAmsCm |
| SR018768A | 360 | AmsAfsUmUfGmGfGmUfGmGfGmUfUmAfUmCfUmAfGmsCmsCm |
| SR018769A | 361 | UmsUfsGmAfGmUfAmAfGmUfCmAfCmUfGmGfGmGfUmsUmsCm |
| SR018770A | 362 | UmsUfsUmCfAmGfAmGfCmUfGmAfGmAfGmGfUmAfCmsUmsCm |
| SR018771A | 363 | UmsGfsGmUfAmUfAmGfAmCfUmUfGmGfGmAfUmGfUmsGmsAm |
| SR018772A | 364 | UmsGfsGmUfUmAfUmCfUmAfGmCfCmUfGmUfAmCfUmsGmsUm |
| SR018773A | 365 | AmsGfsAmUfAmCfUmGfAmCfCmUfGmAfGmUfAmAfGmsUmsCm |
| SR018774A | 366 | UmsAfsUmUfAmGfAmUfAmCfUmGfAmCfCmUfGmAfGmsUmsAm |
| SR018775A | 367 | UmsGfsUmUfGmGfAmAfUmGfAmUfUmCfUmGfGmGfGmsGmsCm |
| SR018776A | 368 | UmsAfsUmGfUmGfAmGfGmCfGmUfUmAfUmGfCmUfGmsAmsAm |
| SR018777A | 369 | UmsAfsUmUfGmGfAmAfAmUfGmUfUmUfUmUfGmUfUmsAmsGm |
| SR018778A | 370 | UmsUfsAmUfGmCfUmGfGmGfGmAfGmUfUmUfAmGfGmsGmsAm |
| SR018779A | 371 | UmsAfsAmAfCmUfCmCfGmAfGmCfUmUfAmUfAmUfUmsAmsGm |
| SR018780A | 372 | AmsUfsGmCfUmGfGmGfGmAfGmUfUmUfAmGfGmGfAmsCmsAm |
| SR018781A | 373 | AmsCfsCmUfGmAfGmUfAmAfGmUfCmAfCmUfGmGfGmsGmsUm |
| SR018782A | 374 | UmsCfsUmUfAmUfAmUfUmAfGmAfUmAfCmUfGmAfCmsCmsUm |
| SR018783A | 375 | UmsGfsUmUfCmAfGmAfGmCfUmGfAmGfAmGfGmUfAmsCmsUm |
| SR018784A | 376 | UmsAfsGmUfAmUfGmCfUmGfGmGfGmAfGmUfUmUfAmsGmsGm |
| SR018785A | 377 | UmsUfsGmAfCmCfAmGfAmCfUmGfCmAfAmCfAmCfCmsAmsUm |
| SR018792A | 378 | AmsCfsUmGfAmCfCmUfGmAfGmUfAmAfGmUfCmAfCmsUmsGm |
| SR018793A | 379 | UmsUfsAmGfAmAfUmGfUmGfGmCfUmUfUmGfAmCfAmsUfsUm |
| SR018794A | 380 | UmsUfsUmUfCmUfUmUfGmGfGmCfCmAfAmGfUmCfCmsUmsUm |
| SR0152010 A | 381 | AmsdGsAmAmdAUmdTUmGmAmUmdGCmUfGmUmCmAmUmAmGmsU msCm |

Table 6. Paired siRNA sense and antisense strands targeting *CIDEB* mRNA

| Double Strand No. | Sense Strand No. | Antisense Strand No. | Double Strand No. | Sense Strand No. | Antisense Strand No. |
|---|---|---|---|---|---|
| DR007248 | SR013956S | SR014433A | DR009688 | SS1 | AS1 |
| DR007260 | SR013968S | SR014445A | DR009703 | SS2 | AS2 |
| DR007261 | SR013969S | SR014446A | DR009700 | SS3 | AS3 |
| DR007266 | SR013974S | SR014451A | DR009695 | SS4 | AS4 |
| DR007303 | SR014011S | SR014488A | DR009691 | SS5 | AS5 |
| DR007435 | SR014143S | SR014620A | DR009697 | SS6 | AS6 |
| DR007497 | SR014205S | SR014682A | DR009674 | SS7 | AS7 |

(continued)

| Double Strand No. | Sense Strand No. | Antisense Strand No. | Double Strand No. | Sense Strand No. | Antisense Strand No. |
|---|---|---|---|---|---|
| DR007543 | SR014251S | SR014728A | DR009701 | SS8 | AS8 |
| DR007559 | SR014267S | SR014744A | DR009693 | SS9 | AS9 |
| DR007567 | SR014275S | SR014752A | DR009696 | SS10 | AS10 |
| DR007571 | SR014279S | SR014756A | DR009676 | SS11 | AS11 |
| DR007572 | SR014280S | SR014757A | DR009708 | SS12 | AS12 |
| DR007573 | SR014281S | SR014758A | DR009699 | SS13 | AS13 |
| DR007574 | SR014282S | SR014759A | DR007632 | SS14 | AS14 |
| DR007575 | SR014283S | SR014760A | DR007334 | SS15 | AS15 |
| DR007576 | SR014284S | SR014761A | DR009662 | SS16 | AS16 |
| DR007599 | SR014307S | SR014784A | DR009659 | SS17 | AS17 |
| DR007600 | SR014308S | SR014785A | DR009675 | SS18 | AS18 |
| DR007601 | SR014309S | SR014786A | DR009651 | SS19 | AS19 |
| DR007636 | SR014344S | SR014821A | DR007257 | SS20 | AS20 |
| DR007638 | SR014346S | SR014823A | DR009692 | SS21 | AS21 |
| DR007640 | SR014348S | SR014825A | DR007648 | SS22 | AS22 |
| DR007642 | SR014350S | SR014827A | DR009660 | SS23 | AS23 |
| DR007650 | SR014358S | SR014835A | DR007263 | SS24 | AS24 |
| DR007652 | SR014360S | SR014837A | DR009673 | SS25 | AS25 |
| DR007653 | SR014361S | SR014838A | DR009690 | SS26 | AS26 |
| DR007898 | SR0152022S | SR014446A | DR007631 | SS27 | AS27 |
| DR007899 | SR0152023S | SR014433A | DR009707 | SS28 | AS28 |
| DR007900 | SR0152024S | SR014451A | DR007276 | SS29 | AS29 |
| DR007901 | SR0152025S | SR014682A | DR009706 | SS30 | AS30 |
| DR007902 | SR0152026S | SR014756A | DR009653 | SS31 | AS31 |
| DR007903 | SR0152027S | SR014757A | DR007268 | SS32 | AS32 |
| DR007904 | SR0152028S | SR014758A | DR007258 | SS33 | AS33 |
| DR007905 | SR0152029S | SR014759A | DR009709 | SS34 | AS34 |
| DR007906 | SR0152030S | SR014760A | DR009668 | SS35 | AS35 |
| DR007907 | SR0152031S | SR014761A | DR009657 | SS36 | AS36 |
| DR007908 | SR0152032S | SR014784A | DR007477 | SS37 | AS37 |
| DR007909 | SR0152033S | SR014785A | DR009672 | SS38 | AS38 |
| DR007910 | SR0152034S | SR014786A | DR009798 | SR018716S | SR018751A |
| DR007911 | SR0152035S | SR014821A | DR009799 | SR018717S | SR018752A |
| DR007912 | SR0152036S | SR014823A | DR009800 | SR018718S | SR018753A |
| DR007913 | SR0152037S | SR014837A | DR009801 | SR018719S | SR018754A |
| DR009202 | SR0152022S | SR017701A | DR009802 | SR018720S | SR018755A |
| DR009203 | SR0152024S | SR017702A | DR009803 | SR018721S | SR018756A |
| DR009204 | SR0152026S | SR017703A | DR009804 | SR018722S | SR018757A |
| DR009205 | SR017698S | SR017704A | DR009805 | SR018723S | SR018758A |

(continued)

| Double Strand No. | Sense Strand No. | Antisense Strand No. | Double Strand No. | Sense Strand No. | Antisense Strand No. |
|---|---|---|---|---|---|
| DR009206 | SR0152030S | SR017705A | DR009806 | SR018724S | SR018759A |
| DR009207 | SR0152031S | SR017706A | DR009807 | SR018725S | SR018760A |
| DR009208 | SR0152033S | SR017707A | DR009808 | SR018726S | SR018761A |
| DR009209 | SR0152034S | SR017708A | DR009809 | SR018727S | SR018762A |
| DR009210 | SR017699S | SR017709A | DR009810 | SR018728S | SR018763A |
| DR009211 | SR017700S | SR017710A | DR009811 | SR018729S | SR018764A |
| DR009347 | SR017939S | SR017949A | DR009812 | SR018730S | SR018765A |
| DR009348 | SR017940S | SR017950A | DR009813 | SR018731S | SR018766A |
| DR009349 | SR017941S | SR017951A | DR009814 | SR018732S | SR018767A |
| DR009350 | SR017942S | SR017952A | DR009815 | SR018733S | SR018768A |
| DR009351 | SR017943S | SR017953A | DR009816 | SR018734S | SR018769A |
| DR009352 | SR017944S | SR017954A | DR009817 | SR018735S | SR018770A |
| DR009353 | SR017945S | SR017955A | DR009818 | SR018736S | SR018771A |
| DR009354 | SR017946S | SR017956A | DR009819 | SR018737S | SR018772A |
| DR009355 | SR017947S | SR017957A | DR009820 | SR018738S | SR018773A |
| DR009356 | SR017948S | SR017958A | DR009821 | SR018739S | SR018774A |
| DR009357 | SR017961S | SR017959A | DR009822 | SR018740S | SR018775A |
| DR009358 | SR017962S | SR017960A | DR009823 | SR018741S | SR018776A |
| DR007886 | SR015209S | SR0152010 A | DR009824 | SR018742S | SR018777A |
| | | | DR009825 | SR018743S | SR018778A |
| | | | DR009826 | SR018744S | SR018779A |
| | | | DR009827 | SR018745S | SR018780A |
| | | | DR009828 | SR018746S | SR018781A |
| | | | DR009829 | SR018747S | SR018782A |
| | | | DR009830 | SR018748S | SR018783A |
| | | | DR009831 | SR018749S | SR018784A |
| | | | DR009832 | SR018750S | SR018785A |
| | | | DR009837 | SR018789S | SR018792A |
| | | | DR009838 | SR018790S | SR018793A |
| | | | DR009839 | SR018791S | SR018794A |

The content of the present disclosure will be further illustrated below with reference to Examples. It is understood that the following Examples are merely illustrative and should not be construed as limiting the scope of the invention.

## EXAMPLES

Unless otherwise specified, the sources of materials used in the examples are as follows:

Huh7 cell line, Nanjing Cobioer Biotechnology Co., Ltd., Cat# CBP60202;
PHH cell line, Shanghai Xuanyi Biotechnology Co., Ltd., Cat# QYLF-HPMC;
PCH cell line, Milestone Biotechnologies, Cat# cmTCSC;
Hep3B cell line, Nanjing Cobioer Biotechnology Co., Ltd., Cat# CBP60197.

**Example 1 Preparation of Compound E7**

**1. Preparation of Intermediate 3-4**

*1.1 Preparation of Compound 2*

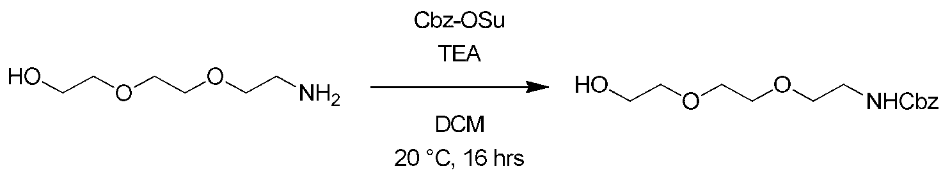


**1** **2**

To Compound 1 (300 g, 2.01 mol) in DCM (1.80 L), benzyl(2,5-dioxopyrrolidin-1-yl) carbonate (600 g, 2.40 mol) was added slowly, and TEA (203 g, 2.01 mol, 280 mL) was added dropwise at 15°C. The mixture was then stirred at 25°C for 16 h until TLC (dichloromethane: methanol = 10:1) showed retention of reactant 1 ($R_f$= 0.32) and detection of a significant new spot ($R_f$ = 0.52). The reaction mixture was washed with saturated sodium bicarbonate solution (1.00 L × 2). The organic phase was washed with brine (1.00 L), dried over anhydrous $Na_2SO_4$, and subjected to vacuum concentration. Compound 2 (about 385 g) was obtained as a yellow oil without further purification.

*1.2 Preparation of Compound 2A*

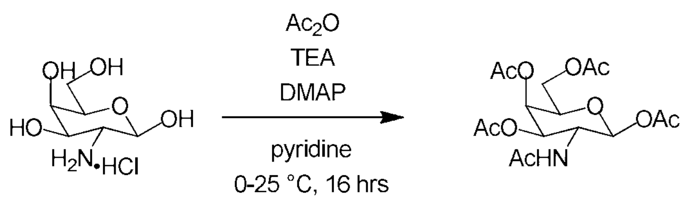


**4** **2A**

To a solution of Compound 4 (350 g, 1.62 mol, HCl) and $Ac_2O$ (994 g, 9.74 mol, 912 mL) in pyridine (1.75 L), DMAP (19.8 g, 162 mmol) was added in one portion and TEA (164 g, 1.62 mol, 226 mL) was added dropwise at 0-15°C. The mixture was stirred at 25°C for 16 h until LCMS (product: RT=0.687 min) showed complete consumption of the starting reactant. EtOAc (1.40 L) was added to the mixture at 25°C and stirred for 30 min. The resulting mixture was then filtered, and the filter cake was washed with EtOAc (300 mL). The filter cake was ground with water (1.45 L) at 25°C for 30 min. The mixture was filtered and the filter cake was washed with water (175 mL × 3). The filter cake was collected to obtain Compound 2A (about 580 g) as a white solid.

*1.3 Preparation of Compound 2B*

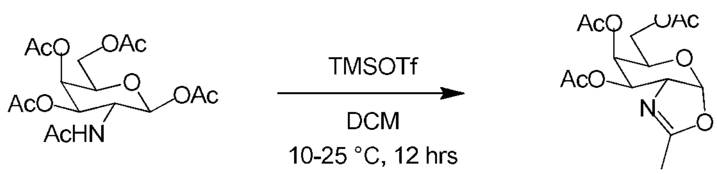


**2A** **2B**

Three reactions were performed in parallel.

To a solution of Compound 2A (200 g, 514 mmol) in DCM (800 mL), TMSOTf (137 g, 616 mmol, 111 mL) was added dropwise over 0.5 h at 10-15°C. The mixture was then stirred at 25°C for 3 h until TLC (dichloromethane: methanol = 20:1) showed complete consumption of Compound 2A ($R_f$ = 0.54) and formation of a new spot ($R_f$= 0.24). The three reactions were combined. The mixture was cooled to 0-15°C and slowly poured into $NaHCO_3$ (300 g dissolved in 3.00 L of water) at 0-5°C. The organic phase was separated, and the aqueous phase was extracted with DCM (1.00 L × 3). The combined organic phases were dried over $Na_2SO_4$, filtered, and subjected to vacuum concentration. Compound 2B (about 507 g) was obtained as a yellow oil for the next step without further purification.

*1.4 Preparation of Compound 3*

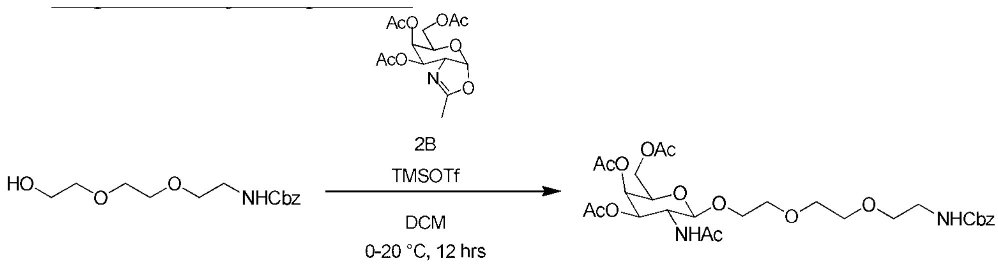


2 3

To a mixture of Compound 2B (250 g, 759 mmol) and Compound 2 (151 g, 531 mmol) in DCM (1.00 L), TMSOTf (84.4 g, 380 mmol, 69.0 mL) was added dropwise at 0-10°C. The mixture was stirred at 20°C for 12 h until TLC (dichloromethane:methanol = 20:1) showed complete consumption of Compound 2 ($R_f$= 0.33) and formation of a new spot ($R_f$= 0.03). The combined reaction mixture was cooled to 0-5°C and then poured into $NaHCO_3$ (100 g in 1 L of water) and stirred at 5-10°C for 10 min to separate the phases. The aqueous phase was extracted with DCM (500 mL × 2). The combined organic phases were dried over $Na_2SO_4$, filtered, and subjected to vacuum concentration. Compound 3 (about 360 g) was obtained as a yellow oil without further purification.

$^1$H NMR : (400 MHz, DMSO).

δ=7.79-7.37 (m, 1H), 7.35-7.26 (m, 5H), 5.21-5.20 (m, 1H), 5.00-4.95 (m, 3H), 4.55-4.53 (m, 1H), 4.03-3.86 (m, 3H), 3.61-3.59 (m, 1H), 3.59-3.57 (m, 1H), 3.48-3.40 (m, 6H), 3.39-3.31 (m, 2H), 3.14-3.13 (m, 2H), 2.09 (s, 3H), 1.99 (s, 3H), 1.88 (s, 3H), 1.76-1.74 (m, 3H).

*1.5 Preparation of Intermediate 3-4 (TFA salt)*

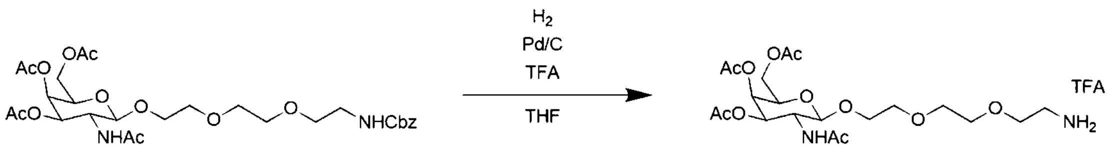


3 Intermediate 3-4 (TFA salt)

Three reactions were performed in parallel.

To a mixture of Pd/C (18.0 g, 16.3 mmol, 10% content) in THF (1.80 L), Compound 3 (180 g, 293 mmol) and TFA (33.5 g, 293 mmol, 21.8 mL) were added under an argon atmosphere. The suspension was degassed and purged three times with hydrogen. The mixture was stirred under $H_2$ (50 Psi) at 30°C for 2 h until LCMS (product: RT = 0.697 min) showed consumption of Compound 3 and detection of the product peak. The three reactions were combined. The mixture was filtered through celite and the filtrate was concentrated under reduced pressure to remove the solvent. Intermediate 3-4 (TFA salt) (393 g, 660 mmol, yield 74.8%, purity 99.6%, TFA) was obtained as a yellow solid without further purification.

$^1$H NMR : (400 MHz, DMSO-d6)

δ = 7.92 (d, *J* = 9.1 Hz, 4H), 5.27-5.17 (m, 1H), 5.03-4.91 (m, 1H), 4.60-4.50 (m, 1H), 4.09-3.97 (m, 4H), 3.85 (s, 2H), 3.65-3.46 (m, 10H), 3.04-2.92 (m, 2H), 2.10 (s, 3H), 2.00 (s, 3H), 1.94-1.86 (m, 3H), 1.82-1.71 (m, 4H).

**2. Preparation of Intermediate 3-3**

2.1 Preparation of Compound 5

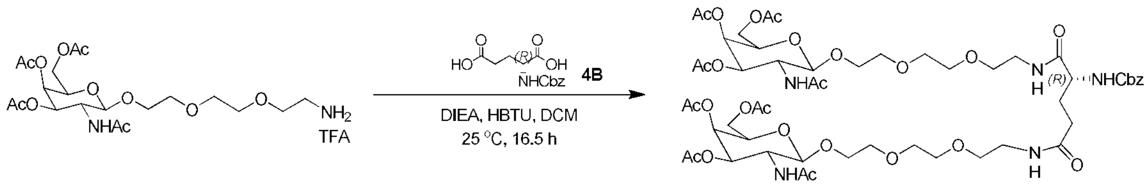


3-4 5

To a solution of Compound 4B (10.0 g, 35.5 mmol, 1.00 eq) and Compound 3-4 (46.3 g, 78.2 mmol, 2.20 eq, TFA) prepared above in DCM (1.00 L), DIEA (30.3 g, 234 mmol, 40.8 mL, 6.60 eq) was added in one portion at 25°C. The mixture was stirred at 25°C for half an hour. HBTU (30.3 g, 234 mmol, 40.8 mL, 6.60 eq) was added to the mixture. The mixture was stirred at 25°C for 16 h until LCMS (product: RT = 0.681 min) showed completion of the reaction. The mixture was subjected to vacuum concentration. The mixture was added with 0.50 N HCl (200 mL × 2) at 20°C, and then extracted with DCM (3 × 500 mL). The combined organic phases were washed with saturated $NaHCO_3$ (3 × 800 mL) until the pH = 8, then washed with brine (3 × 500 mL), dried over $Na_2SO_4$, and subjected to vacuum concentration. The residue was purified by column chromatography ($SiO_2$, DCM:MeOH = 50:1-15:1). The residue was subjected to vacuum concentration at 40°C and purified by preparative-MPLC (column: 800 g Agela C18; mobile phase: [water-ACN]; 15-45%, 25 min; 45%, 10 min). Compound 5 (about 180 g + 75.0 g + 87.0 g + 40.0 g + 38.0 g) was obtained by vacuum drying as a yellow solid.

417.0 g of Compound 3-4 was converted to Compound 5 over 9 batches.

2.2 Preparation of Intermediate 3-3

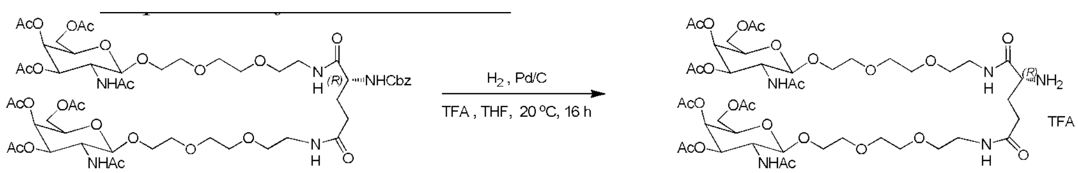


5 3-3

To Pd/C (3.00 g, 10% content) in THF (300 mL), Compound 5 (73.0 g, 61.7 mmol, 1.00 eq) and TFA (7.04 g, 61.7 mmol, 4.57 mL, 1.00 eq) were added under an argon atmosphere. The suspension was degassed and purged three times with hydrogen. The mixture was stirred under $H_2$ (20 Psi) at 20°C for 16 h until TLC (dichloromethane:methanol = 8:1, $R_f$ = 0.0) showed completion of the reaction. The mixture was filtered through celite, and the filtrate was concentrated under pressure to remove the solvent to obtain Compound 3-3 (about 33.4 g + 129 g + 75.0 g) as a white solid.

$^1$H NMR: (400 MHz, DMSO)

$\delta$=8.53 (t, $J$ = 5.2 Hz, 1H), 8.18 (d, $J$ = 2.4 Hz, 3H), 8.03 (t, $J$ = 5.2 Hz, 1H), 7.84 (dd, $J$ = 3.6 Hz, 2H), 5.22 (d, $J$ = 3.2 Hz, 2H), 4.96 (dd, $J$ = 3.2 Hz, 2H), 4.55 (d, $J$ =8.4 Hz, 2H), 4.02 (t, $J$ =8.8 Hz, 6H), 3.77-3.59 (m, 5H), 3.58-3.45 (m, 21H), 3.40-3.20 (m, 4H), 2.18 (t, $J$ = 7.6 Hz, 2H), 2.17 (d, $J$ =8.0 Hz, 6H), 2.10 (s, 6H), 1.99 (s, 6H), 1.90-1.80 (m, 8H), 1.77 (s, 6H).

**3. Preparation of Compound E7**

3.1 Preparation of Compound 3

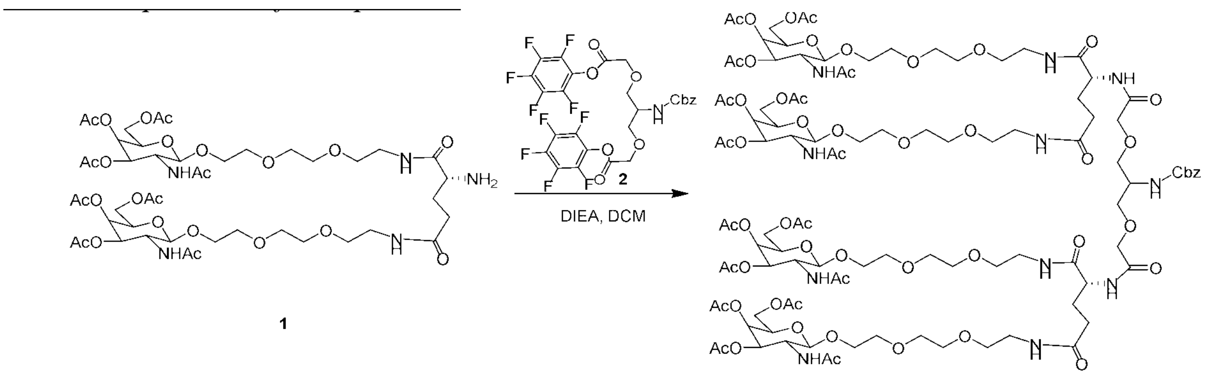


1

3

Compound 1 (2.00 g, 1.87 mmol, prepared as described above for intermediate 3-3) was dissolved in DCM (20.0 mL) at room temperature. DIEA (0.135 mL, 0.814 mmol) and Compound 2 (0.550 g, 0.814 mmol) were added sequentially to the solution, which was then replaced with nitrogen three times. The reaction mixture was stirred at 25°C for 16 h until Liquid Chromatography-Tandem Mass Spectrometry (LC-MS/MS) detected the MS response of the product, and Thin Layer Chromatography (dichloromethane/methanol = 5/1) showed the disannearance of the starting material and formation of a new snot. The reaction mixture was concentrated under reduced pressure. The resulting crude product was purified by column chromatography (dichloromethane/methanol = 5/1) to obtain Compound 3 (about 780 mg) as a white solid.

$^1$H NMR (400 MHz, $CD_3OD$)

$\delta$=7.28-7.42 (m, 5H), 5.30-5.34 (m, 4H), 5.04-5.14 (m, 6H), 4.63-4.67 (m, 4H), 4.36-4.44 (m, 2H), 4.00-4.20 (m, 23H), 3.91-3.95 (m, 4H), 3.69-3.77 (m, 9H), 3.52-3.67 (m, 32H), 3.34-3.43 (m, 9H), 2.29-2.31 (m, 4H), 2.14 (s, 12H), 2.03 (s, 12H), 1.92-1.96 (m, 24H). LCMS: m/z = 1221.6 $(M/2+H)^+$.

*3.2 Preparation of Compound 4*

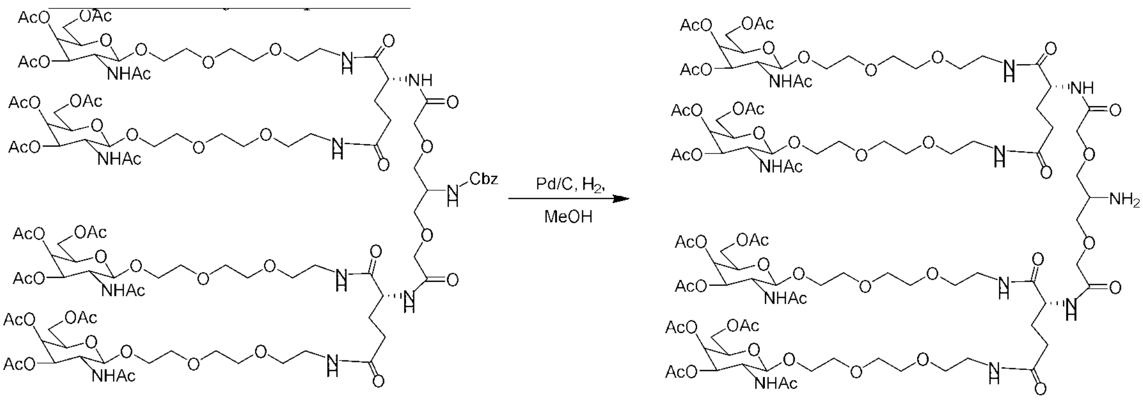


3

4

Compound 3 (1.10 g, 0.451 mmol) was dissolved in MeOH (10.0 mL) at room temperature, and wet Pd/C (0.050 g, 0.451 mmol) with a mass fraction of 10% was added to the solution. The reaction mixture was replaced with hydrogen three times and then stirred at 25°C for 18 h under a hydrogen atmosphere (14.696 psi) until LC-MS/MS detected the MS response of the product, and TLC (dichloromethane/methanol = 10/1, color development: phosphomolybdic acid) showed complete consumption of the starting material and formation of a new spot. The reaction mixture was filtered and the filtrate was concentrated under reduced pressure to obtain Compound 4 (about 840 mg) as a white solid.

$^1$H NMR (400 MHz, $CD_3OD$)

$\delta$=5.32-5.34 (m 4H), 5.06-5.10 (m, 4H), 4.63-4.65 (m, 4H), 4.38-4.40 (m, 2H), 3.99-4.20 (m, 20H), 3.90-3.97 (m, 4H), 3.69-3.76 (m, 6H), 3.50-3.68 (m, 36H), 3.35-3.44 (m, 11H), 2.28-2.38 (m, 4H), 2.15 (s, 12H), 2.03 (s, 12H), 1.90-1.94 (m, 24H). LCMS: m/z = 1154.7 $(M/2+H)^+$.

*3.3 Preparation of Compound 6*

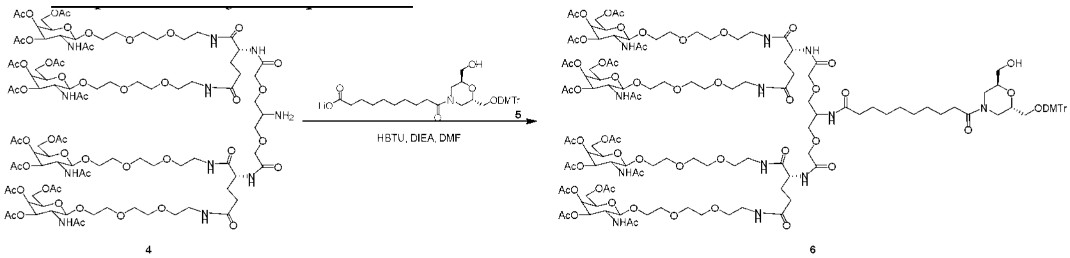


Compound 5 (232 mg, 0.364 mmol) was dissolved in DCM (10.0 mL) at room temperature, and HBTU (207 mg, 0.546 mmol), DIEA (0.181 mL, 1.09 mmol), and Compound 4 (840 mg,0.364 mmol) were added sequentially to the solution, which was then replaced with nitrogen three times The reaction mixture was stirred at 25°C for 1 h until LC-MS/MS detected the disappearance of the starting material, and TLC (dichloromethane/methanol = 5/1) showed the disappearance of the starting material and formation of a new spot. The reaction mixture was concentrated under reduced pressure. The resulting crude product was purified by column chromatography (dichloromethane/methanol = 8/1-5/1) to obtain Compound 6 (about 620 mg) as a white solid.

$^1$H NMR (400 MHz, $CD_3OD$)

$\delta$=7.41-7.43 (m, 2H), 7.23-7.34 (m, 7H), 6.83-6.90 (m, 4H), 5.31-5.35 (m, 4H), 5.01-5.12 (m, 4H), 4.63-4.65 (m, 4H), 4.41-4.45 (m, 2H), 4.31-4.33 (m, 1H), 3.99-4.22 (m, 22H), 3.87-3.97 (m, 6H), 3.58-3.81 (m, 45H), 3.34-3.43 (m, 10H), 2.19-2.40 (m, 10H), 2.14 (s, 12H), 2.02 (s, 12H), 1.92-1.96 (mz, 24H), 1.48-1.63 (m, 4H), 1.28-1.38 (m, 8H). LCMS: m/z = 1460.0 $(M/2+H)^+$.

*4. Preparation of Compound E7*

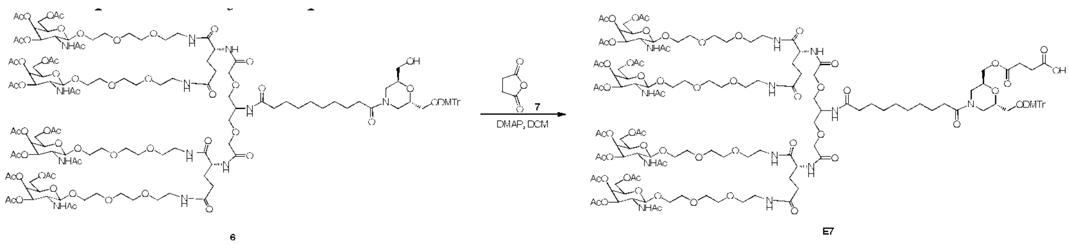


Compound 6 (300 mg, 0.103 mmol) was dissolved in DCM (10.0 mL) at room temperature, and DIEA (0.102 mL, 0.618 mmol), Compound 7 (10.3 mg, 0.103 mmol), and DMAP (12.6 mg, 0.103 mmol) were added sequentially to the solution, which was then replaced with nitrogen three times. The reaction mixture was stirred at 25°C for 2 h until LC-MS/MS detected the disappearance of the starting material. The reaction mixture was concentrated under reduced pressure. The resulting crude product was separated by preparative-MPLC (prep-HPLC, column: Waters Xbridge BEH C18 100*30 mm*10 μm; mobile phase: water-ACN; B%: 17%-57%, 5 min) to obtain Compound E7 (53.0 mg, yield 17.08%, purity 78.94%) as a white solid.

$^1$H NMR (400 MHz, $CD_3OD$)

$\delta$=7.41-7.45 (m, 2H), 7.17-7.34 (m, 7H), 6.85-6.89 (m, 4H), 5.32-5.36 (m, 4H), 5.03-5.13 (m, 4H), 4.63-4.67 (m, 4H), 4.38-4.47 (m, 2H), 4.32-4.34 (m, 1H), 4.01-4.26 (m, 22H), 3.88-4.00 (m, 6H), 3.77-3.81 (m, 7H), 3.49-3.76 (m, 45H), 3.33-3.47 (m, 10H), 2.56-2.62 (m, 2H), 2.45-2.55 (m, 3H), 2.21-2.38 (m, 7H), 2.14 (s, 12H), 2.05-2.11 (m, 2H), 2.02 (s, 12H), 1.92-1.96 (m, 24H), 1.47-1.68 (m, 4H), 1.28-1.34 (m, 8H)

MS: m/z = 3022.36 $(M+H)^+$.

**Example 2 Preparation of Compounds E1-1 and E1-2**

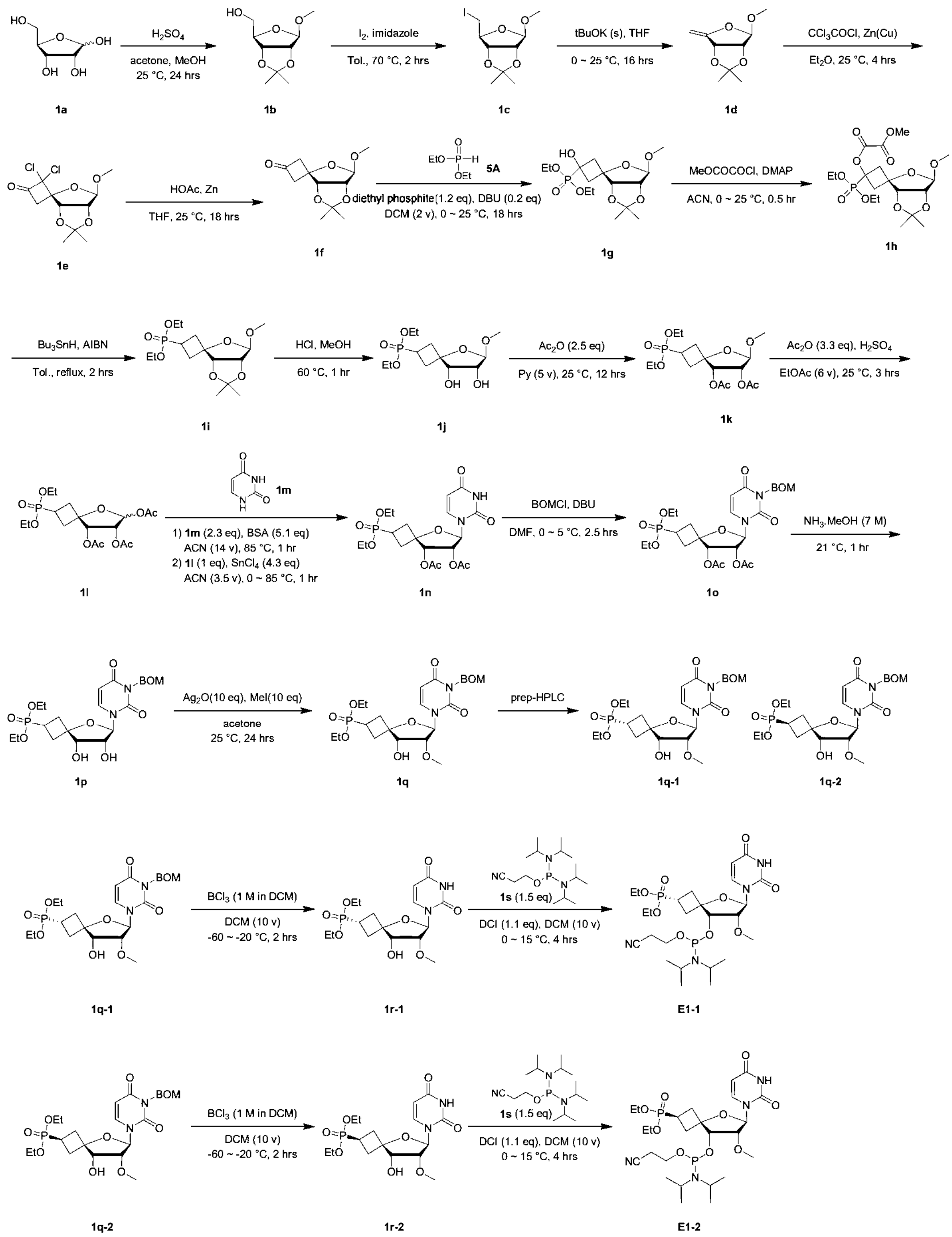


1. Preparation of Compound **1b**

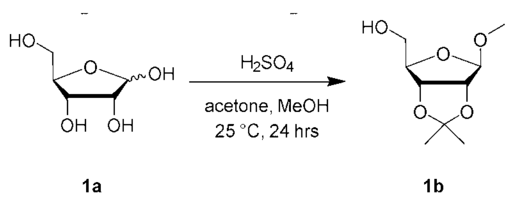


1a 1b

Compound **1a** (200 g, 1.33 mol, 1.00 eq) was suspended in a mixed solution of anhydrous acetone (1.00 L) and anhydrous methanol (1.00 L). Concentrated sulfuric acid (20.0 mL, 0.27 eq) was added dropwise, and the mixture was

allowed to react at 25°C for 24 h. The reaction mixture was neutralized with saturated sodium bicarbonate and concentrated. The resulting residue was dissolved in ethyl acetate and washed three times with saturated brine (500 mL). The organic phase was dried over anhydrous sodium sulfate and concentrated to obtain Compound **1b** (242 g, 88.9%).

**$^1$H NMR:** 400 MHz $CDCl_3$ $\delta$ 4.97 (s, 1H), 4.84 (d, *J* = 6.0 Hz, 1H), 4.59 (d, *J* = 6.0 Hz, 1H), 4.44-4.43 (m, 1H), 3.72-3.59 (m, 2H), 3.44 (s, 3H), 1.49 (s, 3H), 1.32 (s, 3H).

2. Preparation of Compound **1c**

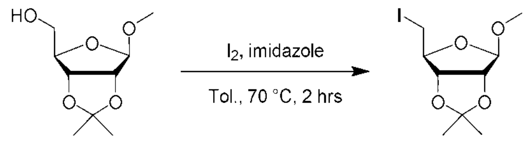


**1b** **1c**

Compound **1b** (310 g, 1.52 mol, 1.00 eq), imidazole (206 g, 3.02 mol, 2.00 eq), and triphenylphosphine (476 g, 1.82 mol, 1.20 eq) were dissolved in toluene (2.1 L), and elemental iodine (446 g, 1.76 mmol, 1.16 eq) was added in portions at room temperature. The mixture was heated up to 70°C and stirred for 1.5 h until TLC showed the completion of the reaction. The reaction mixture was quenched with methanol (60 mL), then cooled, and saturated aqueous sodium thiosulfate solution (2.1 L) was added. The organic phase was separated and collected, washed twice with saturated brine (1.5 L), dried over anhydrous sodium sulfate, filtered, and concentrated. The resulting residue was pulped with methyl tert-butyl ether and filtered. The filtrate was concentrated, and the resulting crude product was purified by silica gel column chromatography (eluent: petroleum ether/ethyl acetate) to obtain Compound **1c** (401 g, 1.27 mol, 91.1%).

**$^1$H NMR:** 400 MHz $CDCl_3$ $\delta$ 4.98 (s, 1H), 4.69 (d, J = 6.0 Hz, 1H), 4.56 (d, J = 6.0 Hz, 1H), 4.39-4.35 (m, 1H), 3.30 (s, 3H), 3.24-3.20 (m, 1H), 3.09 (t, J = 10.0 Hz, 1H) , 1.41 (s, 3H), 1.26 (s, 3H).

3. Preparation of Compound **1d**

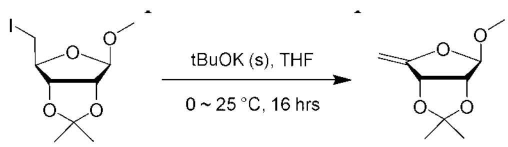


**1c** **1d**

Compound **1c** (203 g, 646 mmol, 1.00 eq) was dissolved in tetrahydrofuran (2.03 L), and potassium tert-butoxide (145 g, 1.29 mol, 1.29 eq) was added in portions at 0°C. The mixture was stirred at 25°C for 16 h and then cooled to 5°C and quenched with ice water (1.1 L). The mixture was extracted with methyl tert-butyl ether (2.0 L), washed with saturated brine (2.0 L), dried over anhydrous sodium sulfate, filtered, and concentrated. The resulting crude product was purified by silica gel column chromatography (eluent: petroleum ether/ethyl acetate) to obtain Compound **1d** (160 g, 91.3%).

**$^1$H NMR:** 400 MHz $CDCl_3$ & 5.11 (s, 1H), 5.02 (d, J = 5.6 Hz, 1H), 4.06 (d, J = 1.6 Hz, 1H), 4.50 (d, J = 5.6 Hz, 1H), 4.39 (d, J = 1.6 Hz, 1H), 3.41 (s, 3H), 3.24-3.20 (m, 1H), 3.09 (t, J = 10.0 Hz, 1H) , 1.47 (s, 3H), 1.35 (s, 3H).

4. Prearation of Comoound **1e**

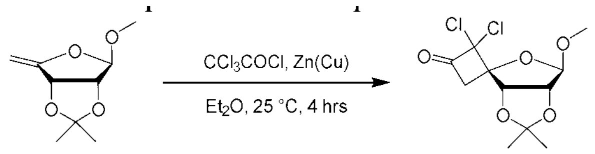


1d 1e

A zinc-copper couple (90.0 g, 1.37 mol, 5.50 eq) was dispersed in diethyl ether (200 mL). Compound **1d** (60 g, 241 mmol, 1.00 eq) was added at 25°C, and a solution of trichloroacetyl chloride (61.5 g, 338 mmol, 1.40 eq) in diethyl ether (200 mL) was added dropwise. The mixture was stirred for an additional 1 h. After filtration, the filter cake was rinsed with methyl tert-butyl ether (500 mL), and the filtrate was poured into a saturated aqueous sodium bicarbonate solution (2.50 L) and filtered. The filtrate was washed three times with saturated brine (500 mL), dried over anhydrous sodium sulfate, filtered, and concentrated to obtain crude Compound **1e** (71.8 g).

**$^1$H NMR:** 400 MHz $CDCl_3$ $\delta$ 5.12 (d, J = 5.6 Hz, 1H), 5.09 (s, 1H), 4.70 (d, J = 6.0 Hz, 1H), 3.70-3.55 (m, 2H), 3.54 (s, 3H), 1.45 (s, 3H), 1.36 (s, 3H).

5. Preparation of Compound **1f**

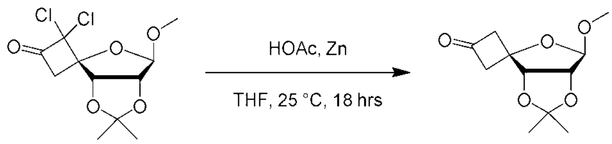


1e 1f

Compound **1e** (71.8 g, 242 mmol, 1.00 eq) was dissolved in tetrahydrofuran (1.60 L). Glacial acetic acid (69.1 mL, 1.20 mol, 5.00 eq) was added, and zinc powder (142 g, 2.17 mol, 9.00 eq) was added in portions. The mixture was stirred at 25°C for 18 h. After completion, the reaction was filtered, and the filtrate was concentrated to obtain a crude product. The crude product was dissolved in methyl tert-butyl ether, poured into a saturated aqueous sodium bicarbonate solution (2.0 L), and filtered. The filtrate was washed twice with saturated brine (500 mL), dried over anhydrous sodium sulfate, filtered, and concentrated. The crude product was purified by silica gel column chromatography (eluent: petroleum ether/ethyl acetate/dichloromethane) to obtain Compound **1f** (31 g, 56.3% yield in two steps).

**$^1$H NMR:** 400 MHz $CDCl_3$ $\delta$ 4.97 (s, 1H), 4.70-4.67 (m, 2H), 3.54-3.48 (m, 2H), 3.37-3.31 (m, 4H), 3.16-3.10 (m, 1H), 3.09-3.03 (m, 1H), 1.43 (s, 3H), 1.34 (s, 3H).

6. Preparation of Compound **1g**

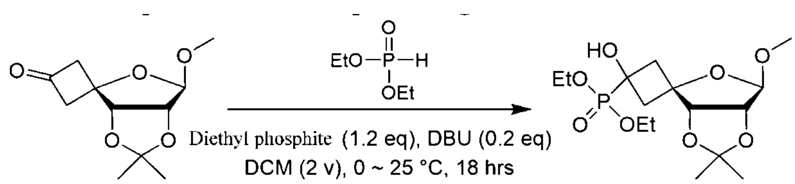


1f 1g

Compound **1f** (50 g, 219 mmol, 1.00 eq) and diethyl phosphite (33.2 g, 240 mmol, 1.20 eq) were dissolved in dichloromethane (100 mL), and 1,8-diazabicycloundec-7-ene (DBU, 6.67 g, 43.8 mmol, 0.20 eq) was added at 0°C, and the mixture was stirred at 25°C for 18 h. The reaction mixture was washed three times with saturated $NH_4Cl$ (100 mL) and once with saturated brine (100 mL), dried over anhydrous sodium sulfate, and concentrated. The resulting crude product was purified by silica gel column chromatography (eluent: petroleum ether/dichloromethane) to obtain Compound **1g** (70 g, 87.2%).

**$^1$H NMR:** 400 MHz $CDCl_3$ $\delta$ 5.27 (s, 1H), 5.01 (brs, 1H), 4.78 (s, 1H), 4.65 (d, J = 5.6 Hz, 1H), 4.49 (d, J = 6.0 Hz, 1H), 4.20-4.05 (m, 5H), 3.31 (s, 1H), 3.29-3.05 (m, 1H), 2.75-2.68 (m, 1H), 2.50-2.40 (m, 1H), 2.35-2.25 (m, 1H), 1.36-1.25

(m, 12 H).

7. Preparation of Compound **1h**

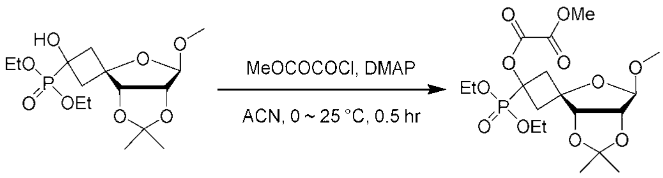


**1g** **1h**

Compound 1g (80.2 g, 219 mmol, 1.00 eq) and DMAP (40.1 g, 328 mmol, 1.50 eq) were dissolved in acetonitrile (562 mL). Methyl oxalyl chloride (40.2 g, 328 mmol, 1.50 eq) was added at 5°C. The mixture was stirred at 25°C for half an hour. The reaction mixture was concentrated. The resulting crude product was dissolved in ethyl acetate, washed five times with saturated ammonium chloride (300 mL), once with water (200 mL), and once with brine (200 mL), dried over anhydrous sodium sulfate, filtered, and concentrated to obtain crude Compound **1h** (99 g).

**$^1$H NMR:** 400 MHz $CDCl_3$ $\delta$ 4.88-4.80 (m, 2H), 4.67-4.53 (m, 2H), 4.24-4.12 (m, 4H), 3.89 (s, 3H), 3.51-3.43 (m, 1H), 3.35-3.33 (m, 3H), 3.15-3.07 (m, 1H), 2.92-2.80 (m, 1H), 2.69-2.61 (m, 1H),1.39-1.29 (m, 12H).

8. Preparation of Compound **1i**

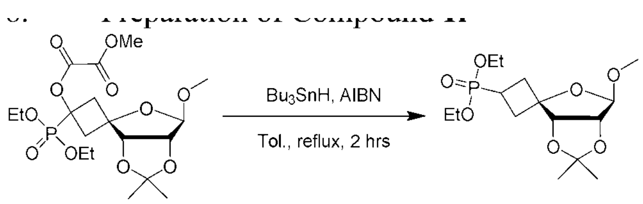


**1h** **1i**

Compound **1h** (99 g, 218 mmol, 1.00 eq) was dissolved in anhydrous toluene (1.00 L). Tri-n-butyltin hydride (76.4 g, 262 mmol, 1.20 eq) and azobisisobutyronitrile (AIBN, 1.08 g, 6.56 mmol, 0.03 eq) were added. The mixture was refluxed for 2 h. The reaction mixture was concentrated. The resulting crude product was purified by silica gel column chromatography (eluent: petroleum ether/ethyl acetate) to obtain crude Compound **1i** (146 g).

**$^1$H NMR:** 400 MHz $CDCl_3$ $\delta$ 4.75-4.49 (m, 3H), 4.05-3.95 (m, 4H), 3.26-3.24 (m, 3H), 2.76-2.07 (m, 5H), 1.39-1.29 (m, 12H).

9. Preparation of Compound **1j**

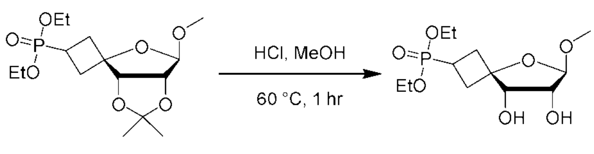


**1i** **1j**

Compound **1i** (141 g, 201 mmol, 1.00 eq) was dissolved in methanol (1.41 L). An aqueous solution of HCl (704 mL, 1.40 mol, 2 M, 7.00 eq) was added, and the reaction mixture was stirred at 60°C for 1 h. The reaction mixture was extracted with a mixture of methyl tert-butyl ether/petroleum ether. The aqueous phase was collected, adjusted to pH 8 with saturated sodium bicarbonate, and concentrated. Tetrahydrofuran was added to the resulting residue and filtered. The filtrate was concentrated to obtain crude Compound **1j** (62.4 g, 201 mmol).

10. Preparation of Compound **1k**

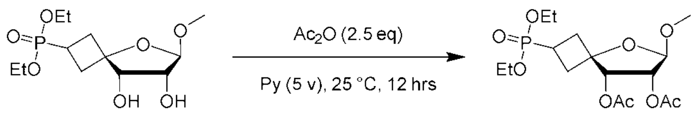


1j 1k

Compound **1j** (62.4 g, 201 mmol, 1.00 eq) was dissolved in pyridine (300 mL). Acetic anhydride (47.4 mL, 502 mmol, 2.50 eq) was added, and the mixture was stirred at 25°C for 12 h. The reaction mixture was diluted with saturated sodium bicarbonate (1.00 L), extracted twice with ethyl acetate (600 mL), dried over anhydrous sodium sulfate, filtered, and concentrated. The crude product was purified by silica gel column chromatography (eluent: petroleum ether/ethyl acetate) to obtain compound **1k** (74 g, 93.3%)

**$^1$H NMR:** 400 MHz $CDCl_3$ $\delta$ 5.38-5.10 (m, 3H), 4.07-4.03 (m, 4H), 3.36-3.32 (m, 3H), 2.41-2.05 (m, 5H), 2.04-1.98 (m, 9H), 1.27-1.23 (m, 6H).

11. Preparation of Compound 11

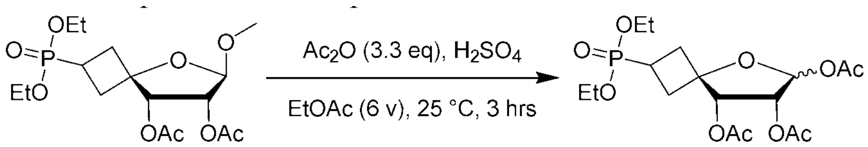


**1k** **1l**

Compound **1k** (79.3 g, 201 mmol, 1.00 eq) was dissolved in ethyl acetate (476 mL). Acetic anhydride (62.6 mL, 663 mmol, 3.30 eq) and concentrated sulfuric acid (5.38 mL, 100 mmol, 0.50 eq) were added, and the mixture was stirred at 25°C for 3 h. The reaction mixture was neutralized with saturated aqueous sodium bicarbonate solution, and extracted twice with ethyl acetate (1.00 L). The combined organic phases were washed twice with saturated brine (500 mL), dried over anhydrous sodium sulfate, filtered, and concentrated. The crude product was purified by silica gel column chromatography to obtain Compound 11 (43 g, 50.6%).

**$^1$H NMR:** 400 MHz $CDCl_3$ $\delta$ 6.11 (d, J = 2.8 Hz, 1H), 5.44-5.39 (m, 1H), 5.33-5.32 (m, 1H), 4.10-4.05 (m, 5H), 2.80-2.35 (m, 4H), 2.12-2.02 (m, 9H), 1.32-1.23 (m, 6H).

12. Preparation of Compound **1n**

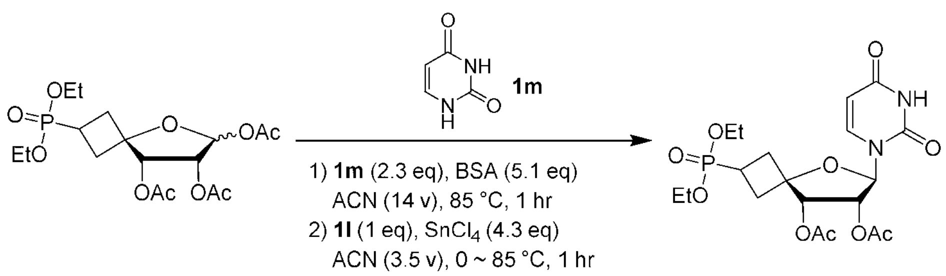


**1l** **1n**

Compound **1m** (8.55 g, 76.2 mmol, 2.30 eq) and bis(trimethylsilyl)acetamide (BSA, 34.3 g, 169 mmol, 5.10 eq) were suspended in acetonitrile (200 mL) and stirred at 85°C for 1 h. After cooling, a solution of Compound **11** (14.0 g, 33.1 mmol, 1.00 eq) in acetonitrile (50.0 mL) was added, followed by tin tetrachloride (37.1 g, 142 mmol, 4.30 eq). The mixture was stirred at 25°C for 15 min and then stirred in an oil bath at 85°C for 45 min. After cooling, the reaction mixture was

poured into a saturated aqueous sodium bicarbonate solution (1.50 L), and extracted three times with dichloromethane (700 mL). The combined organic phases were washed with saturated brine (500 mL), dried over anhydrous sodium sulfate, filtered, and concentrated. The crude product was purified by silica gel column chromatography (eluent: dichloromethane/methanol) to obtain Compound **1n** (14.4 g, 84.3%).

**$^1$H NMR:** 400 MHz $CDCl_3$ $\delta$ 9.70 (brs, 0.46H), 9.60 (brs, 0.60H), 7.17 (d, J = 8.0 Hz, 0.60H), 7.12 (d, J = 8.0 Hz, 0.49H), 5.98 (d, J = 6.4 Hz, 0.43H), 5.93 (d, J = 5.2 Hz, 0.55H), 5.78 (dt, J1 = 8.0 Hz, J2 = 2.0 Hz, 1H), 5.50-5.45 (m, 2H), 4.45-4.02 (m, 5H), 2.77-2.42 (m, 6H), 2.18 (s, 1.22H), 2.16 (s, 1.74 H), 2.04 (s, 1.31H), 2.02 (s, 1.67H), 1.32-1.28 (m, 6H).

13. Preparation of Compound **1o**

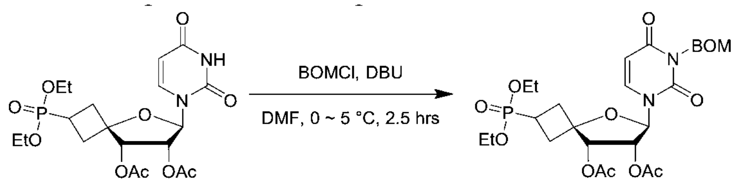


**1n** **1o**

Compound **1n** (8.30 g, 17.4 mmol, 1.00 eq) was dissolved in DMF (40.0 mL). DBU (2.93 g, 19.2 mmol, 1.10 eq) was added, followed by benzyl chloromethyl ether (BOMCl, 3.01 g, 19.2 mmol, 1.10 eq) after cooling. The mixture was stirred at 0-5°C for 2.5 h. The reaction mixture was diluted with saturated ammonium chloride (160 mL), and extracted twice with dichloromethane (80.0 mL). The combined organic phases were washed with saturated brine (80 mL), dried over anhydrous sodium sulfate, filtered, and concentrated. The crude product was purified by silica gel column chromatography (eluent: dichloromethane/methanol) to obtain Compound **1o** (12.0 g, 92.3%).

**$^1$H NMR:** 400 MHz $CDCl_3$ $\delta$ 7.36-7.24 (m, 5H), 7.15-7.09 (m, 1H), 5.96-5.92 (m, 1H), 5.81-5.79 (m, 1H), 5.52-5.42 (m, 4H), 4.67 (s, 2H), 4.15-4.05 (m, 4H), 2.80-2.45 (m, 5H), 2.19-2.17 (m, 3H), 2.05-2.03 (m, 3H), 1.34-1.30 (m, 6H).

14. Preparation of Compound **1p**

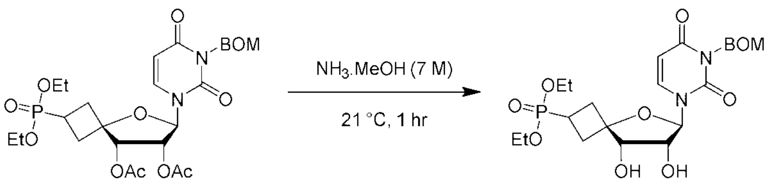


**1o** **1p**

Compound **1o** (10.4 g, 17.4 mmol, 1.00 eq) was dissolved in ammonia methanol solution (29.9 mL, 7 M) and stirred at 21°C for 1 h. The reaction mixture was concentrated to obtain a crude product, which was in turn purified by silica gel column chromatography (eluent dichloromethane/methanol) to obtain Compound **1p** (8.00 g, 62.7%).

**$^1$H NMR:** 400 MHz $CDCl_3$ $\delta$ 7.34-7.06 (m, 5H), 5.83 (s, 0.52H), 5.71 (d, J = 8.4 Hz, 0.41H), 5.661 (d, J = 8.4 Hz, 0.53H), 5.60 (d, J = 2.0 Hz, 0.59H), 5.53 (d, J = 5.2 Hz, 0.41H), 5.42-5.36 (m, 2H), 4.62 (s, 2H), 4.51 (d, J = 3.6 Hz, 0.45H), 3.72 (d, J = 2.8 Hz, 0.36H), 3.54 (d, J = 2.0 Hz, 0.48H), 2.93-2.40 (m, 5H), 2.24-2.13 (m, 1H), 1.27-1.23 (m, 6H).

15. Preparation of Compound **1q**

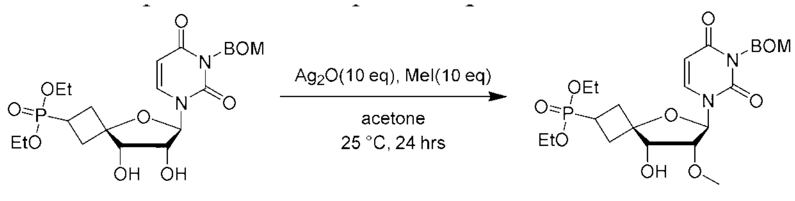


1p 1q

Compound **1p** (1 g, 1.95 mmol, 1.00 eq) was dissolved in acetone (19.6 mL). Silver oxide (3.63 g, 15.6 mmol, 10.0 eq) and methyl iodide (1.22 mL, 19.5 mmol, 10.0 eq) were added, and the mixture was stirred at 25°C for 24 h. The reaction mixture was filtered and concentrated to obtain crude Compound **1q** (1.36 g).

**$^1$H NMR:** 400 MHz $CDCl_3$ $\delta$ 5.11 (s, 1H), 5.02 (d, J = 5.6 Hz, 1H), 4.06 (d, J = 1.6 Hz, 1H), 4.50 (d, J = 5.6 Hz, 1H), 4.39 (d, J = 1.6 Hz, 1H), 3.41 (s, 3H), 3.24-3.20 (m, 1H), 3.09 (t, J = 10.0 Hz, 1H) , 1.47 (s, 3H), 1.35 (s, 3H).

16. Preparation of Compounds **1q-1** and **1q-2**

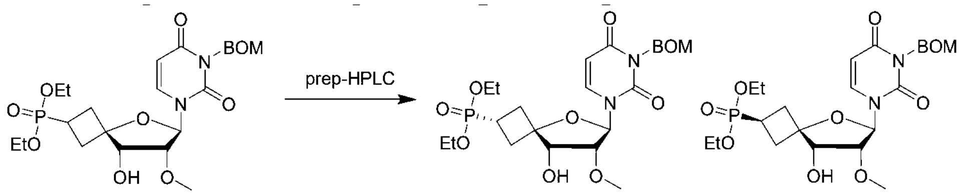


1q 1q-1 1q-2

Crude Compound **1q** (10.9 g, 20.7 mmol, 1.00 eq) was purified by C18 reversed-phase column chromatography (aqueous ammonium bicarbonate solution/acetonitrile) to obtain Compound **1q-1** (1.50 g, 11.5%) and Compound **1q-2** (1.50 g, 11.5%).

Compound **1q-1**:

**m/z:** ES+ [M+H]+ 525.2

**HPLC:** Retention time 0.820 min (Column: XBridge C18 2.1*50mm,5$\mu$m; Mobile phase: A: 10mM $NH_4HCO_3$ aqueous solution B: Acetonitrile; Gradient: 0-0.01 min 5% B, 0.01-0.7 min 5-95% B, 0.7-1.16 min 95% B, 1.16-1.5min, 95%-5% B; Flow rate: 1.5 mL/min; Column temp.: 40°C)

**$^1$H NMR:** 400 MHz $CDCl_3$ $\delta$ 7.37-7.08 (m, 5H), 7.09 (d, J = 8.0 Hz, 1H), 5.76-5.74 (m, 2H), 5.49-5.44 (m, 2H), 4.73-4.67 (m, 2H), 4.15-4.06 (m, 4H), 4.02 (d, J = 4.8 Hz, 1H), 3.87-3.85 (m, 1H), 3.56 (s, 3H), 2.81-2.64 (m, 2H), 2.50-2.39 (m, 2H), 2.36-2.27 (m, 1H), 1.34-1.30 (m, 6H).

Compound **1q-2:**

**m/z:** ES+ [M+H]+ 525.2

**HPLC:** Retention time 0.836 min (Column: XBridge C18 2.1*50mm, 5$\mu$m; Mobile phase: A: 10mM $NH_4HCO_3$ aqueous solution B: Acetonitrile; Gradient: 0-0.01 min 5% B, 0.01-0.7 min 5-95% B, 0.7-1.16 min 95% B, 1.16-1.5min, 95%-5% B; Flow rate: 1.5 mL/min; Column temp.: 40°C)

**$^1$H NMR:** 400 MHz $CDCl_3$ $\delta$ 7.37-7.27 (m, 5H), 7.08 (d, J = 8.0 Hz, 1H), 5.75-5.73 (m, 2H), 5.50-5.45 (m, 2H), 4.73-4.67 (m, 2H), 4.18-4.08 (m, 4H), 4.06 (d, J = 4.8 Hz, 1H), 3.88-3.86 (m, 1H), 3.56 (s, 3H), 3.06-2.95 (m, 1H), 2.83-2.41 (m, 5H), 1.34-1.31 (m, 6H).

17. Preparation of Compound **1r-1**

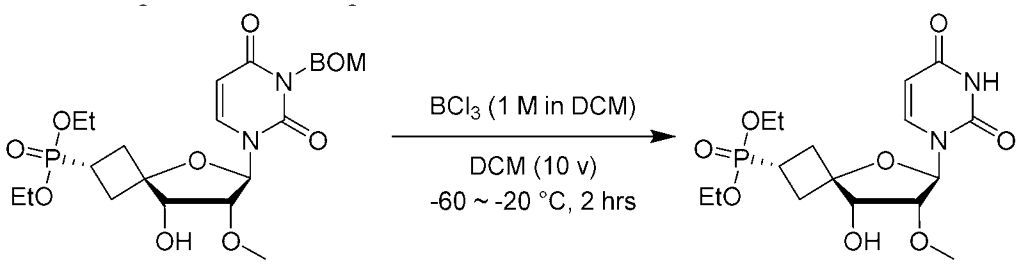


1q-1 1r-1

Compound **1q-1** (1.00 g, 1.90 mmol, 1.00 eq) was dissolved in anhydrous dichloromethane (DCM, 20.0 mL) and cooled to -60°C. Boron trichloride (7.62 mL, 7.62 mmol, 1 M in dichloromethane, 4.00 eq) was added, and the mixture was stirred at -20°C for 1 h. The reaction mixture was quenched with absolute ethanol (10.0 mL), neutralized to about pH 7 with concentrated ammonia, and concentrated. The crude product was added to a mixed solution of dichloromethane and ethanol, and then filtered. The filtrate was concentrated, and the resulting residue was purified by C18 reversed-phase column chromatography (aqueous ammonium bicarbonate solution/acetonitrile) to obtain Compound **1r-1** (400 mg, 51.9%).

**m/z:** ES+ [M+H]+ 405.3

**$^1$H NMR:** 400 MHz $CDCl_3$ $\delta$ 8.51-8.43 (m, 1H), 7.15 (d, J = 8.4 Hz, 1H), 5.78 (d, J = 3.2 Hz, 1H), 5.76 (dd, J1 = 8.0 Hz, J2 = 2.4 Hz, 1H), 4.16-4.07 (m, 5H), 3.94 (dd, J1 = 4.8 Hz, J2 = 3.2 Hz, 1H), 3.55 (s, 3H), 2.96-2.94 (m, 1H), 2.79-2.66 (m, 2H), 2.52-2.41 (m, 2H), 2.35-2.26 (m, 1H), 1.34-1.31 (m, 6H).

18. Preparation of Compound **1r-2**

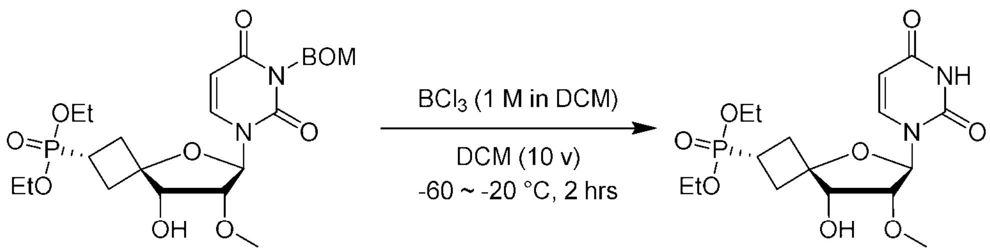


1q-2 1r-2

Compound **1r-2** was obtained using the same synthetic method as for Compound **1r-1.**

**$^1$H NMR:** 400 MHz $CDCl_3$ $\delta$ 9.16-8.81 (m, 1H), 7.15 (d, J = 8.0 Hz, 1H), 5.78 (d, J = 2.8 Hz, 1H), 5.76 (dd, J1 = 8.0 Hz, J2 = 2.0 Hz, 1H), 4.65 (brs, 1H), 4.18-4.08 (m, 5H), 3.96 (dd, J1 = 4.8 Hz, J2 = 3.2 Hz, 1H), 3.53 (s, 3H), 3.05-2.95 (m, 1H), 2.81-2.41 (m, 2H), 1.34-1.30 (m, 6H).

19. Preparation of Compound **E1-1**

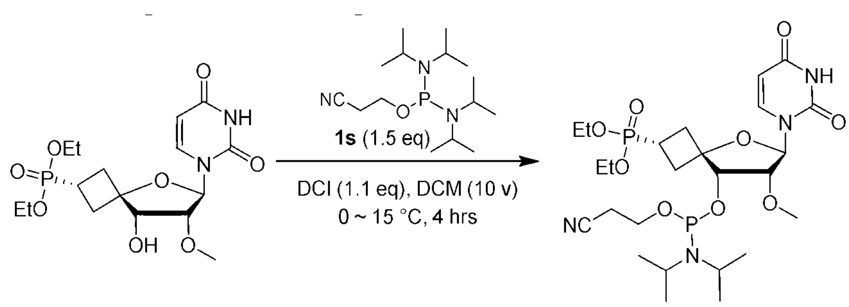


1r-1 E1-1

Compound **1r-1** (500 mg, 1.23 mmol, 1.00 eq) was dissolved in dichloromethane (5.00 mL). Compound **1s** (0.59 mL, 1.85 mmol, 1.50 eq) was added, followed by 4,5-dicyanoimidazole (160 mg, 1.36 mmol, 1.10 eq) after cooling to 0°C, and the mixture was stirred at 15°C for 4 h. The reaction mixture was quenched with saturated sodium bicarbonate (10.0 mL) and extracted twice with dichloromethane (10.0 mL). The crude product was obtained by concentration and purified by silica gel column chromatography (eluent: dichloromethane/acetone) to obtain Compound **E1-1** (530 mg, 70.9%).
**$^1$H NMR:** 400 MHz $CDCl_3$ $\delta$ 9.17 (brs, 1H), 7.24 (d, J = 8.4 Hz, 1H), 5.83-5.79 (m, 1H), 5.64-5.61 (m, 1H), 4.43-4.33 (m, 1H), 4.09-3.62 (m, 9H), 3.43-3.38 (m, 3H), 2.86-2.66 (m, 3H), 2.57-2.21 (m, 4H), 1.28-1.18 (m, 18H).

20. Preparation of Compound **E1-2**

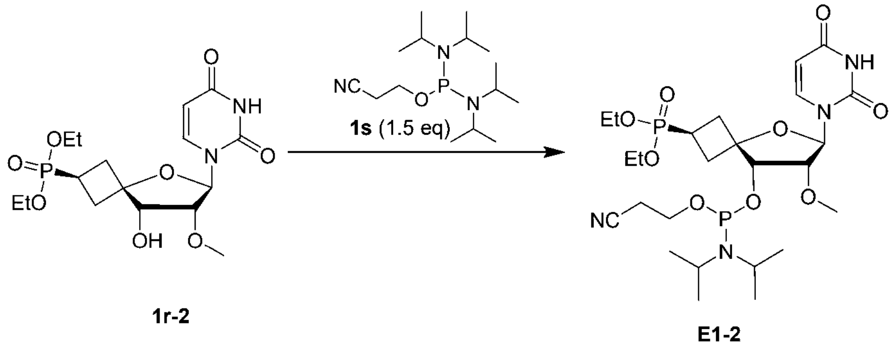


1r-2

E1-2

Compound **1r-2** (500 mg, 1.23 mmol, 1.00 eq) was dissolved in dichloromethane (5.00 mL). Compound **1s** (0.59 mL, 1.85 mmol, 1.50 eq) was added, followed by 4,5-dicyanoimidazole (160 mg, 1.36 mmol, 1.10 eq) after cooling to 0°C, and the mixture was stirred at 15°C for 4 h. The reaction mixture was quenched with saturated sodium bicarbonate (10.0 mL) and extracted twice with dichloromethane (10.0 mL). The crude product was obtained by concentration and purified by silica gel column chromatography (eluent: dichloromethane/acetone) to obtain Compound **E1-2** (480 mg, 64.0%).
**$^1$H NMR:** 400 MHz $CDCl_3$ $\delta$ 9.06 (brs, 1H), 7.33-7.31 (m, 1H), 5.89-5.88 (m, 1H), 5.66-5.64 (m, 1H), 4.49-4.40 (m, 1H), 4.08-3.64 (m, 9H), 3.40-3.36 (m, 3H), 3.00-2.19 (m, 7H), 1.29-1.18 (m, 18H).

**Example 3 Preparation of siRNA**

The siRNA of the present invention was prepared using the solid-phase phosphoramidite method well known in the art. The specific methods can be found, for example, in PCT Publication Nos. WO2016081444 and WO2019105419, and are briefly described below.

**1. Preparation of siRNA without Ligand Conjugated**

*1.1 Synthesis of Sense Strand (SS)*

Using the solid-phase phosphoramidite method, a blank CPG solid support was used as the starting cycle, and nucleoside monomers were linked one by one from the 3' to 5' direction according to the arrangement of sense strand nucleotides. Each linking of a nucleoside monomer involved four steps of deprotection, coupling, capping, and oxidation or thiolation to synthesize 5 μmol oligonucleotide with the synthesis conditions as follows:
Commercially available phosphoramidites with 2'-F, 2'-O-methyl and other modifications introduced were used. Nucleoside monomers were provided as a 0.05 mol/L solution in acetonitrile. The conditions for each step were the same: temperature 25°C; deprotection for 3 times using a 3% trichloroacetic acid-dichloromethane solution; coupling twice using a 0.25 mol/L ETT-acetonitrile solution as an activator; capping twice using 10% acetic anhydride-acetonitrile and pyridine/N-methylimidazole/acetonitrile (10:14:76, v/v/v); oxidation twice using 0.05 mol/L of iodine in tetrahydrofuran/-pyridine/water (70:20:10, v/v/v); thiolation twice using 0.2 mol/L PADS in acetonitrile/3-methylpyridine (1:1, v/v).

*1.2 Synthesis of Antisense Strand (AS)*

Using the solid-phase phosphoramidite method, a blank CPG solid support was used as the starting cycle, and nucleoside monomers were linked one by one from the 3' to 5' direction according to the arrangement of antisense strand nucleotides. Each linkage of a nucleoside monomer involved four steps of deprotection, coupling, capping, and oxidation

or thiolation. The conditions for the synthesis of a 5 μmol oligonucleotide for the antisense strand were the same as for the sense strand.

*1.3 Purification and Annealing of Oligonucleotides*

1.3.1 Ammonolysis

The synthesized solid support (sense or antisense strand) was transferred to a 5 mL centrifuge tube, followed by the addition of 3% diethylamine/ammonia (v/v). The mixture was allowed to react in a thermostatic water bath at 35°C for 16 h (or 55°C for 8 h), and then filtered. The solid support was washed three times with ethanol/water, 1 mL each time. The filtrate was concentrated by centrifugation, and the crude product was purified.

1.3.2 Purification

The methods for purification and desalting are well known to those skilled in the art. For example, a strong anionic packing column and a sodium chloride-sodium hydroxide system may be used for elution and purification. The product can be collected in tubes and desalted using a gel packing purification column, with pure water as the eluent.

1.3.3 Annealing

According to Table 6, the sense strand (SS) was mixed with the antisense strand (AS) at a molar ratio (SS/AS = 1/1.05). The mixture was heated in a water bath to 70-95°C, held for 3-5 min, and then allowed to cool naturally to room temperature. The system was freeze-dried to obtain the product.

**2. Preparation of siRNA with Sense Strand Conjugated to Ligand**

*2.1 Conjugation of ligand to CPG Carrier*

Conjugation of Compound E7 to CPG Carrier

Compound E7 (53 mg, 0.018 mmol) and HBTU (13.3 mg, 0.035 mmol) were mixed and dissolved in acetonitrile (5 mL) by shaking. DIEA (9.0 mg, 0.07 mmol) and DMAP (2.1 mg, 0.018 mmol) were then added and dissolved by shaking until clear. Blank carrier Resin (550 mg, CPG pore size 1000Å) was weighed and added to the reaction mixture, which was then placed on a shaker at 20°C overnight. A sample was taken and monitored until Thin Layer Chromatography (TLC) (developing solvent: DCM/methanol = 4/1; color development: phosphomolybdic acid) showed completion of the reaction. The reaction mixture was filtered through a sand core funnel. The filter cake was washed with anhydrous acetonitrile (20 mL*5), collected, and suction-filtered under reduced pressure using an oil pump for 6 h to obtain 530 mg of an off-white solid.

The above condensed product (530 mg) was transferred to a 50 mL round-bottom flask. CapC (DMAP/acetonitrile), CapB (N-methylimidazole/pyridine/acetonitrile), and CapA (acetic anhydride/acetonitrile) were added sequentially. The mixture was then placed on a shaker at room temperature overnight. After filtration, the filter cake was washed with acetonitrile (20 mL*4), collected, and suction-filtered under reduced pressure using an oil pump for 8 h to obtain 200 mg of an off-white solid for solid-phase synthesis.

*2.2 Synthesis of Sense Strand (SS)*

Using the solid-phase phosphoramidite method, the E7 solid support prepared above was used as the starting cycle, and nucleoside monomers were linked one by one from the 3' to 5' direction according to the arrangement of sense strand nucleotides. Each linking of a nucleoside monomer involved four steps of deprotection, coupling, capping, and oxidation or thiolation to synthesize 5 umol oligonucleotide with the synthesis conditions as follows:
Nucleoside monomers were provided in a 0.05 mol/L acetonitrile solution. The conditions for each step were the same: temperature 25°C; deprotection for 3 times using a 3% trichloroacetic acid-dichloromethane solution; coupling twice using a 0.25 mol/L ETT-acetonitrile solution as an activator; capping twice using 10% acetic anhydride-acetonitrile and pyridine/N-methylimidazole/acetonitrile (10:14:76, v/v/v); oxidation twice using 0.05 mol/L of iodine in tetrahydrofuran/-pyridine/water (70:20:10, v/v/v); thiolation twice using 0.2 mol/L PADS in acetonitrile/3-methylpyridine (1:1, v/v).

*2.3 Synthesis of Antisense Strand (AS)*

Using the solid-phase phosphoramidite method, a blank CPG solid support was used as the starting cycle, and nucleoside monomers were linked one by one from the 3' to 5' direction according to the arrangement of antisense strand nucleotides. Each linkage of a nucleoside monomer involved four steps of deprotection, coupling, capping, and oxidation or thiolation. The conditions for the synthesis of a 5 μmol oligonucleotide for the antisense strand were the same as those for the sense strand.

The antisense strands of the siRNAs listed in Table 5 were finally synthesized. Among them, the modified nucleotide SCP-U in the antisense strands was synthesized using the Compound E1-1 in Example 2 as the nucleoside monomer.

*2.4 Purification and Annealing of Oligonucleotides*

2.4.1 Ammonolysis

The synthesized solid support (sense or antisense strand) was transferred to a 5 mL centrifuge tube, followed by the addition of 3% diethylamine/ammonia (v/v). The mixture was allowed to react in a thermostatic water bath at 35°C for 16 h (or 55°C for 8 h), and then filtered. The solid support was washed three times with ethanol/water, 1 mL each time. The filtrate was concentrated by centrifugation, and the crude product was purified.

2.4.2 Purification

The methods for purification and desalting are well known to those skilled in the art. For example, a strong anionic packing column and a sodium chloride-sodium hydroxide system may be used for elution and purification. The product can be collected in tubes and desalted using a gel packing purification column, with pure water as the eluent.

2.4.3 Annealing

According to Table 6, the sense strand (SS) was mixed with the antisense strand (AS) at a molar ratio (SS/AS = 1/1.05). The mixture was heated in a water bath to 70-95°C, held for 3-5 min, and then allowed to cool naturally to room temperature. The system was freeze-dried to obtain the product.

**Example 4 Activity Screening in Huh7 Cell Line**

*Cell Transfection*

On Day 1, the human hepatocellular carcinoma Huh7 cell line was digested, resuspended, and counted. The cell resuspension was plated into a 96-well plate at 100 μL/well ($1\times10^4$ cells/well) for transfection after 18 hours.

On Day 2, the 20 μM siRNA stock solution was diluted with Opti-MEM. 198 μL of Opti-MEM was added to 2 μL of 20 μM siRNA stock solution with a final siRNA concentration specified below, mixed well by pipetting for later use.

On Day 2, 0.9 μL of RNAiMAX (Thermo, 13778150) was diluted with 14.1 μL of Opti-MEM, mixed gently by pipetting, and allowed to stand at room temperature for 5 min. Then, 15 μL of the prepared RNAi-MAX mixture and 15 μL of the diluted siRNA were mixed gently by pipetting (avoiding bubbles), and allowed to stand at room temperature for 10 min. The mixture was transferred to the 96-well plate at 10 μL/well, and incubated in a 5% $CO_2$ incubator at 37°C for 24 h (no siRNA was added to control group).

*RNA Extraction*

Cellular RNA was extracted using a nucleic acid extractor (Hangzhou Allsheng, Auto-pure96) according to the protocol of the high-throughput cell RNA extraction kit (FireGen, FG0417-L).

*RNA Reverse Transcription*

The denatured reaction mixture was prepared with reference to PrimeScript™ II 1st Strand cDNA Synthesis Kit (Takara, 6210B), with a volume per single well: 1 μL of Oligo dT Primer, 1 μL of dNTP Mixture, and 12.5 μL of template RNA. The mixture was incubated in a conventional PCR instrument at 65°C for 5 min and rapidly cooled on ice for 2 min.

The reverse transcription reaction mixture was prepared with reference to PrimeScript™ II 1st Strand cDNA Synthesis Kit (Takara, 6210B). Each well contained 4 μL of 5×Prime Script II Buffer, 0.5 μL of RNase Inhibitor, and 1 μL of

PrimeScript II RTase.

The denatured reaction mixture (14.5 μL) was mixed gently with the reverse transcription reaction mixture. Reverse transcription was performed by incubation in a conventional PCR instrument at 42°C for 45 min, followed by incubation at 95°C for 5 min to inactivate the enzyme. The reverse transcription product (cDNA) was then cooled at 4°C.

After reverse transcription, 30 μL of DNase/RNase-Free Distilled Water was added to the cDNA sample in each well.

*Fluorescence Quantitative PCR*

Fluorescence quantitative PCR reaction was performed in a 20 μL system (ABI, QuantStudio3) with reference to the procedure of TaqMan™ Fast Advanced Master Mix (ABI, 4444965). The reaction program was: (50°C, 2 min) × 1 Cycle; (95°C, 20 s) × 1 Cycle; (95°C, 1 s; 60°C, 24 s) × 40 Cycles.

Table 7. Primer Information

| Primer Name | Sequence Information (5'-3') | Fluorophore |
|---|---|---|
| hACTB-PF | ACGTGGACATCCGCAAAGAC (SEQ ID NO: 382) | / |
| hACTB-PR | TCTTCATTGTGCTGGGTGCC (SEQ ID NO: 383) | / |
| hACTB-P | AACACAGTGCTGTCTGGCGGCACCA (SEQ ID NO: 384) | 5'TET, 3'BHQ2 |
| hCIDEB-PF | AGCCCTACAAGGAGTGGAGT (SEQ ID NO: 385) | / |
| hCIDEB -PR | GAGCCCGTAGAATGTGGCTT (SEQ ID NO: 386) | / |
| hCIDEB -P | ATGGCCTGGGACGGGAGAGGCCCAAGCA (SEQ ID NO: 387) | 5'6-FAM, 3'BHQ1 |

*Data Statistics*

The $2^{-\triangle\triangle Ct}$ value was calculated and converted into a percentage to obtain the residual inhibition.

$$\triangle\triangle CT = [(Ct_{\text{target gene, experimental group}} - Ct_{\text{reference gene, experimental group}}) - (Ct_{\text{target gene, control group}} - Ct_{\text{reference gene, control group}})].$$

The target gene was hCIDEB, and the reference gene was hACTB.

The siRNA compounds were screened at a final siRNA concentration of 10 nM for activity in the cell line. The experimental screening results are shown in Table 8.

Table 8. Screening Results of Activity in Huh7 Cell Line

| **siRNA No.** | **Residual inhibition** | **SD** | **siRNA No.** | **Residual inhibition** | **SD** |
|---|---|---|---|---|---|
| DR007248 | 25.37% | 0.58% | DR007574 | 18.28% | 3.00% |
| DR007260 | 16.61% | 5.72% | DR007575 | 11.49% | 0.83% |
| DR007261 | 27.14% | 0.75% | DR007576 | 22.36% | 1.90% |
| DR007266 | 26.30% | 1.59% | DR007599 | 28.81% | 1.88% |
| DR007303 | 26.95% | 1.76% | DR007600 | 29.54% | 2.46% |
| DR007435 | 23.78% | 1.79% | DR007601 | 26.90% | 1.19% |
| DR007497 | 26.05% | 2.30% | DR007636 | 25.51% | 6.79% |
| DR007543 | 24.20% | 1.66% | DR007638 | 21.46% | 0.67% |
| DR007559 | 23.62% | 2.12% | DR007640 | 18.91% | 7.63% |
| DR007567 | 24.04% | 0.08% | DR007642 | 19.28% | 2.39% |
| DR007571 | 15.34% | 1.63% | DR007650 | 28.05% | 5.69% |
| DR007572 | 19.83% | 0.81% | DR007652 | 23.81% | 0.39% |
| DR007573 | 9.81% | 1.37% | DR007653 | 15.38% | 3.59% |

**Example 5 Screening of Activity in Primary Human Hepatocytes (PHHs)**

*Cell Transfection*

1.4 mL of rat tail collagen solution (Sigma, C3867) was added to 40.6 mL of DNase/RNase-Free Distilled Water and mixed well. The mixture was added to a 96-well culture plate at 40 μL/well, and coated overnight at 4°C. The coating medium was discarded the next day.

On Day 2, prior to use, the coated cell plate was rinsed with DPBS, which was then aspirated. PHHs (Shanghai Xuanyi Biotechnology Co. Ltd., Cat#: QYLF-HPMC) were recovered at 37°C, transferred into the recovery medium, centrifuged, resuspended, and counted. PHHs were plated in a 96-well plate at 90 μL/well ($2\times10^4$ cells/well). The complete medium was replaced after 4 h, and transfection was performed after 18 h.

On Day 3, the 20 μM siRNA stock solution was diluted with Opti-MEM. 198 μL of Opti-MEM was added to 2 μL of 20 μM siRNA stock solution and mixed well by pipetting, as the first concentration point. Serial dilutions were made as required for the experiment. No siRNA was added to the control group.

On Day 3, 0.9 μL of RNAiMAX (Thermo, 13778150) was diluted with 14.1 μL of Opti-MEM, mixed gently by pipetting, and allowed to stand at room temperature for 5 min. Then, 15 μL of the prepared RNAi-MAX mixture and 15 μL of the diluted compound were mixed gently by pipetting (avoiding bubbles), and allowed to stand at room temperature for 10 min. The mixture was transferred to a 96-well plate at 10 μL/well. The plate was extracted after incubation in a 5% $CO_2$ incubator at 37°C for 24 h before RNA extraction.

*RNA Extraction*

Cellular RNA was extracted using a nucleic acid extractor (Hangzhou Allsheng, Auto-pure96) according to the protocol of the high-throughput cell RNA extraction kit (FireGen, FG0412).

*RNA Reverse Transcription*

The denatured reaction mixture was prepared with reference to PrimeScript™ II 1st Strand cDNA Synthesis Kit (Takara, 6210B). Each well contained 1 μL of Oligo dT Primer, 1 μL of dNTP Mixture, and 12.5 μL of template RNA. The denaturation reaction was performed by incubation in a conventional PCR instrument at 65°C for 5 min. The mixture was rapidly cooled on ice for 2 min.

The reverse transcription reaction mixture was prepared with reference to PrimeScript™ II 1st Strand cDNA Synthesis Kit (Takara, 6210B). Each well contained 4 μL of 5 ×Prime Script II Buffer, 0.5 μL of RNase Inhibitor, and 1 μL of PrimeScript II RTase.

The denatured reaction mixture (14.5 μL) was mixed gently with the reverse transcription reaction mixture. Reverse transcription was performed by incubation in a conventional PCR instrument at 42°C for 45 min, followed by incubation at 95°C for 5 min to inactivate the enzyme. The reverse transcription product (cDNA) was then cooled at 4°C.

After reverse transcription, 30 μL of DNase/RNase-Free Distilled Water was added to the cDNA sample in each well.

*Fluorescence Quantitative PCR*

Fluorescence quantitative PCR reaction was performed in a 20 μL system (ABI, QuantStudio3) with reference to the procedure of TaqMan™ Fast Advanced Master Mix (ABI, 4444965). The reaction program was: (50°C, 2 min) × 1 Cycle; (95°C, 20 s) × 1 Cycle; (95°C, 1 s; 60°C, 24 s) × 40 Cycles.

Table 9. Primer Information

| Primer Name | Sequence Information (5'-3') | Fluorophore |
|---|---|---|
| hACTB-PF | CCTTCCTTCCTGGGCATGG (SEQ ID NO: 388) | / |
| hACTB-PR | TCTTCATTGTGCTGGGTGCC (SEQ ID NO: 383) | / |
| hACTB-P | AACACAGTGCTGTCTGGCGGCACCA (SEQ ID NO: 384) | 5'TET, 3'BHQ2 |
| hCIDEB-PF | AGCCCTACAAGGAGTGGAGT (SEQ ID NO: 385) | / |
| hCIDEB -PR | GAGCCCGTAGAATGTGGCTT (SEQ ID NO: 386) | / |
| hCIDEB -P | ATGGCCTGGGACGGGAGAGGCCCAAGCA (SEQ ID NO: 387) | 5'6-FAM, 3'BHQ1 |

*Data Statistics*

The $2^{-\triangle\triangle Ct}$ value was calculated and converted into a percentage to obtain the residual inhibition.

$\triangle\triangle Ct = [(Ct_{target\ gene,\ experimental\ group} - Ct_{reference\ gene,\ experimental\ group}) - (Ct_{target\ gene,\ control\ group} - Ct_{reference\ gene,\ control\ group})]$.

The target gene was hCIDEB, and the reference gene was hACTB.
47 siRNAs were screened for activity in human primary hepatocytes, using the starting concentration of siRNA as 10 nM and 5 concentration points obtained by 10-fold serial dilution (10nM, 1nM, 0.1 nM, 0.01 nM, 0.001 nM). The experimental results are shown in Table 11.
(10 nM, 1 nM, 0.1 nM, 0.01 nM, 0.001 nM). The screening results are shown in Table 10, in which columns 2 to 6 show the residual inhibition and column 7 shows the IC50 value.

Table 10. Screening Results of siRNA in PHHs at 5 Concentration Points

| Compound No. | 10nM | 1nM | 0.1nM | 0.01nM | 0.001nM | $IC_{50}$ (nM) |
|---|---|---|---|---|---|---|
| DR007248 | 22.63% | 22.38% | 22.59% | 47.34% | 65.34% | 0.0130 |
| DR007261 | 14.91% | 17.97% | 20.44% | 34.10% | 52.83% | 0.0015 |
| DR007266 | 14.47% | 15.02% | 15.93% | 30.98% | 46.96% | 0.0047 |
| DR007497 | 10.43% | 14.34% | 22.65% | 45.59% | 115.18% | 0.0079 |
| DR007571 | 12.28% | 11.46% | 24.22% | 34.58% | 87.37% | 0.0041 |
| DR007572 | 13.48% | 16.39% | 29.33% | 49.51% | 108.61% | 0.0105 |
| DR007573 | 12.98% | 18.53% | 29.54% | 58.39% | 124.53% | 0.0172 |
| DR007574 | 29.26% | 35.40% | 56.61% | 91.70% | 142.94% | 0.1678 |
| DR007575 | 12.65% | 13.06% | 19.25% | 43.86% | 65.31% | 0.0061 |
| DR007576 | 11.71% | 15.59% | 25.38% | 48.97% | 65.05% | 0.0089 |
| DR007599 | 20.72% | 20.29% | 28.67% | 54.07% | 76.07% | 0.0135 |
| DR007600 | 17.76% | 22.09% | 28.40% | 64.55% | 69.21% | 0.0303 |
| DR007601 | 19.50% | 18.81% | 28.14% | 53.70% | 79.88% | 0.0132 |
| DR007636 | 16.71% | 24.29% | 37.75% | 78.06% | 105.68% | 0.0459 |
| DR007638 | 11.64% | 16.83% | 33.73% | 77.52% | 97.35% | 0.0420 |
| DR007650 | 43.89% | 40.07% | 61.76% | 111.52% | 98.18% | 0.1172 |
| DR007652 | 26.32% | 40.32% | 82.77% | 116.58% | 105.96% | 0.4703 |
| DR007653 | 16.97% | 40.30% | 70.50% | 99.20% | 112.25% | 0.4635 |

**Example 6 Screening of Activity in Hep3B Cell Line**

Referring to the protocol of Example 4, the human hepatocellular carcinoma cell line, Hep3B cell line (Nanjing Cobioer Biotechnology Co., Ltd., Cat#: CBP60197) was used. siRNA compounds were screened for activity in the cell line, with a starting concentration of siRNA compounds selected as 10nM and 5 concentration points obtained by 10-fold serial dilution (10nM, 1nM, 0.1 nM, 0.01 nM, 0.001 nM). The experimental results are shown in Table 11.

Table 11. Results of the Screening Experiment of 5-point IC50 Activity in the Hep3B Cell Line

| **Compound No.** | **10nM** | **1nM** | **0.1nM** | **0.01nM** | **0.001nM** | **$IC_{50}$ (nM)** |
|---|---|---|---|---|---|---|
| DR007248 | 14.5% | 15.4% | 31.0% | 76.9% | 145.7% | 0.0326 |
| DR007261 | 3.8% | 3.2% | 5.9% | 25.5% | 83.6% | 0.0026 |
| DR007266 | 5.1% | 3.8% | 5.5% | 16.0% | 88.9% | 0.0015 |

(continued)

| Compound No. | 10nM | 1nM | 0.1nM | 0.01nM | 0.001nM | $IC_{50}$ (nM) |
|---|---|---|---|---|---|---|
| DR007497 | 1.7% | 2.2% | 6.4% | 33.9% | 76.5% | 0.0048 |
| DR007571 | 2.8% | 4.1% | 10.1% | 45.7% | 93.9% | 0.0084 |
| DR007572 | 4.0% | 6.8% | 26.0% | 59.2% | 93.4% | 0.0174 |
| DR007573 | 5.5% | 10.1% | 22.0% | 71.5% | 98.7% | 0.0246 |
| DR007574 | 8.1% | 11.3% | 44.7% | 115.9% | 162.9% | 0.0736 |
| DR007575 | 6.4% | 5.8% | 15.7% | 66.3% | 168.7% | 0.0169 |
| DR007576 | 9.2% | 6.8% | 13.1% | 50.5% | 120.1% | 0.0103 |
| DR007599 | 9.5% | 9.1% | 18.1% | 58.3% | 90.1% | 0.0144 |
| DR007600 | 10.2% | 8.8% | 15.7% | 56.3% | 112.0% | 0.0132 |
| DR007601 | 7.8% | 15.2% | 30.6% | 108.4% | 155.4% | 0.0478 |
| DR007636 | 6.1% | 4.9% | 18.6% | 66.4% | 85.8% | 0.0215 |
| DR007638 | 3.7% | 4.0% | 23.8% | 69.2% | 88.9% | 0.0271 |
| DR007650 | 20.7% | 12.0% | 37.8% | 79.9% | 93.7% | 0.0500 |
| DR007652 | 7.7% | 10.1% | 36.7% | 85.9% | 103.9% | 0.0536 |
| DR007653 | 7.4% | 11.0% | 34.3% | 117.7% | 161.0% | 0.0537 |

**Example 7 Screening of Activity in PCH Cell Line**

Referring to the protocol of Example 4, the monkey primary hepatocyte PCH cell line (Milestone Biotechnologies, Cat# cmTCSC) was used. siRNA compounds were screened for activity in the cell line, with a starting concentration of siRNA compounds selected as 10nM, and 11 concentration points obtained by 3-fold serial dilution (10nM, 3.33nM, 1.11nM, 0.37nM, 0.123nM, 0.041nM, 0.0136nM, 0.0045nM, 0.00152nM, 0.000508nM, 0.000169nM). The experimental results are shown in Table 12.

Table 12. Results of the Screening Experiment of 11-point IC50 Activity in the PCH Cell Line

| Compound No. | 10n M | 3.33 nM | 1.11 nM | 0.37 nM | 0.12 3nM | 0.04 1nM | 0.013 6nM | 0.004 5nM | 0.0015 2nM | 0.0005 08nM | 0.0001 69nM | PCH IC50( nM) |
|---|---|---|---|---|---|---|---|---|---|---|---|---|
| DR00 7248 | 23. 8% | 20.6 % | 31.5 % | 35.8 % | 45.9 % | 55.8 % | 72.7 % | 86.0 % | 89.5% | 91.6% | 88.8% | 0.083 3 |
| DR00 7261 | 19. 7% | 14.7 % | 25.4 % | 25.3 % | 58.2 % | 76.5 % | 88.6 % | 88.2 % | 99.2% | 99.5% | 94.8% | 0.142 8 |
| DR00 7266 | 21. 1% | 13.5 % | 32.1 % | 25.7 % | 33.7 % | 45.7 % | 56.2 % | 66.5 % | 72.6% | 88.1% | 92.0% | 0.028 5 |
| DR00 7497 | 24. 8% | 21.5 % | 22.3 % | 26.9 % | 30.3 % | 48.9 % | 76.6 % | 85.2 % | 95.5% | 94.3% | 92.2% | 0.039 8 |
| DR00 7571 | 17. 2% | 14.0 % | 18.9 % | 17.3 % | 25.1 % | 38.2 % | 64.9 % | 70.7 % | 83.0% | 74.0% | 79.6% | 0.025 4 |
| DR00 7572 | 26. 5% | 18.6 % | 22.4 % | 25.9 % | 55.9 % | 79.1 % | 86.3 % | 90.7 % | 91.8% | 87.7% | 85.2% | 0.140 9 |
| DR00 7573 | 17. 6% | 23.5 % | 25.4 % | 29.7 % | 46.4 % | 61.6 % | 82.1 % | 95.6 % | 86.2% | 76.9% | 80.6% | 0.093 8 |
| DR00 7574 | 34. 1% | 30.6 % | 29.7 % | 34.2 % | 41.8 % | 54.5 % | 66.5 % | 69.5 % | 76.9% | 71.4% | 65.9% | 0.060 9 |
| DR00 7575 | 20. 8% | 17.5 % | 20.8 % | 31.1 % | 44.5 % | 57.7 % | 73.1 % | 88.8 % | 94.4% | 102.1 % | 116.1 % | 0.070 7 |
| DR00 7576 | 34. 6% | 30.3 % | 29.3 % | 33.7 % | 50.8 % | 65.0 % | 87.5 % | 94.1 % | 95.0% | 99.9% | 106.3 % | 0.105 4 |
| DR00 7599 | 28. 0% | 24.9 % | 25.3 % | 34.3 % | 48.7 % | 66.1 % | 81.1 % | 91.5 % | 102.0 % | 93.8% | 102.5 % | 0.104 9 |
| DR00 7600 | 20. 0% | 20.7 % | 22.6 % | 32.3 % | 46.6 % | 66.5 % | 78.3 % | 102.8 % | 107.1 % | 95.8% | 106.6 % | 0.097 3 |
| DR00 7601 | 14. 1% | *15.1* % | 19.3 % | 24.6 % | 34.4 % | 51.8 % | 74.9 % | 91.2 % | 100.0 % | 97.9% | 93.4% | 0.049 5 |
| DR00 7636 | 17. 1% | 15.2 % | 19.2 % | 31.6 % | 52.5 % | 77.5 % | 92.8 % | 103.6 % | 104.7 % | 105.8 % | 96.8% | 0.136 0 |
| DR00 7638 | 20. 6% | 21.0 % | 29.2 % | 39.5 % | 54.9 % | 70.1 % | 85.5 % | 103.3 % | 105.9 % | 103.6 % | 102.8 % | 0.156 7 |
| DR00 7650 | 78. 5% | 69.2 % | 67.6 % | 71.8 % | 83.2 % | 103. 8% | 110.1 % | 109.6 % | 108.3 % | 101.0 % | 97.7% | > 10 |
| DR00 7652 | 45. 2% | 38.6 % | 37.3 % | 53.2 % | 71.1 % | 80.3 % | 84.9 % | 86.8 % | 84.1% | 81.4% | 100.6 % | 0.415 6 |
| DR00 7653 | 66. 6% | 67.2 % | 78.6 % | 83.4 % | 90.2 % | 89.7 % | 90.9 % | 86.1 % | 86.8% | 84.0% | 79.8% | > 10 |

**Example 8. Detection of Activity in HDI Mouse Model**

1. HDI Animal Modeling:

Using the tail vein high-pressure injection method, six- to eight-week-old male Balb/c mice were transfected *in vivo* with a dual-gene stable transfection system. The mice were injected via the tail vein with a TransIT®-QR Delivery Solution (total volume: 10% of animal body weight, Mirusbio-MIR 5240) containing 1:1 mass ratios of a Piggy-Bac transposon plasmid with a cDNA sequence of a target gene encoding cell death-inducing DFFA-like effector B and helper plasmid within 5-7 seconds using a 27-gauge needle. After injection, the mice were returned to their cages and observed for 30 minutes. The day of modeling was designated as Day 0. SEAP expression levels were detected at Day 7 (D7), Day 14 (D14), Day 21 (D21), and Day 28 (D28) after modeling.

2. Detection of SEAP Expression

The standard provided with the kit (Phospha-Light™ SEAP Reporter Gene Assay System, Invitrogen, T1016) was diluted 2-fold starting from 15 mU/mL, resulting in 7 concentration points.

The CSPD substrate was mixed with the Reaction Buffer Diluent at a ratio of 1:20 to prepare the reaction mixture.

The 5×Dilution Buffer was diluted to 1 ×Dilution Buffer with DNase RNase-Free Distilled Water.

The serum was mixed with 1 ×Dilution Buffer in a centrifuge tube to prepare the sample dilution, which was then incubated at 65°C for 30 min and then cooled to room temperature.

50 μL of the sample dilution was added to a 96-well plate, followed by 50 μL of Assay Buffer per well, and incubated at room temperature for 5 min.

50 μL of the reaction solution was added to each well and incubated at room temperature for 20 min. The SEAP chemiluminescence value was read using a microplate reader (Tecan, Infinite 200).

In this experiment, a total of 100 μg of plasmid was used for modeling. On Day 15 after modeling, each mouse was given a single subcutaneous administration of: 200 μl of normal saline containing 3 mg/kg (mpk) siRNA reagent; or 200 μl of normal saline without siRNA reagent used as a blank control. The screening results of the HDI model experiments are shown in Table 13.

Table 13. Experimental Results of Inhibition of siRNA Reagents on CIDEB Expression in the HDI Plasmid Stablely Transfected Mouse Model

| | Blank Control | | DR007886 | | DR007898 | | DR007899 | | DR007900 | |
|---|---|---|---|---|---|---|---|---|---|---|
| | Mean | SD | Mean | SD | Mean | SD | Mean | SD | Mean | SD |
| D7 | 101.7% | 32.8% | 10.9% | 2.9% | 17.8% | 8.9% | 136.2% | 63.9% | 7.9% | 4.2% |
| D14 | 132.4% | 38.1% | 16.1% | 5.4% | 22.7% | 3.8% | 118.0% | 35.0% | 10.6% | 4.3% |
| D21 | 120.7% | 22.0% | 23.3% | 9.9% | 30.2% | 12.7% | ND | ND | 16.2% | 7.1% |
| D28 | 113.9% | 54.8% | 43.8% | 17.2% | 52.6% | 9.8% | ND | ND | 39.3% | 12.9% |
| | DR007901 | | DR007902 | | DR007903 | | DR007904 | | DR007905 | |
| | Mean | SD | Mean | SD | Mean | SD | Mean | SD | Mean | SD |
| D7 | 29.9% | 12.1% | 11.9% | 5.3% | 30.1% | 17.4% | 30.6% | 13.9% | 41.2% | 21.6% |
| D14 | 59.6% | 37.6% | 11.5% | 5.9% | 33.5% | 26.9% | 41.7% | 38.7% | 78.7% | 44.0% |
| D21 | ND | ND | 13.5% | 7.8% | 44.6% | 38.8% | 99.8% | 5.5% | ND | ND |
| D28 | ND | ND | 32.2% | 14.2% | ND | ND | ND | ND | ND | ND |
| | DR007909 | | DR007886 | | DR009202 | | DR009203 | | DR009204 | |
| | Mean | SD | Mean | SD | Mean | SD | Mean | SD | Mean | SD |
| D7 | 21.3% | 8.1% | 21.9% | 3.6% | 25.9% | 7.1% | 19.7% | 15.6% | 24.3% | 6.8% |
| D14 | 30.9% | 13.1% | 19.2% | 5.3% | 22.2% | 6.2% | 18.8% | 20.6% | 17.8% | 5.4% |
| D21 | 36.4% | 4.4% | 31.0% | 15.3% | 32.0% | 9.8% | 33.0% | 40.5% | 27.8% | 11.3% |
| D28 | 57.0% | 14.1% | 55.6% | 17.7% | 45.7% | 18.5% | 41.7% | 9.7% | 52.7% | 23.1% |

(continued)

| | DR009205 | | DR009207 | | DR009208 | | DR009209 | | DR009210 | |
|---|---|---|---|---|---|---|---|---|---|---|
| | Mean | SD | Mean | SD | Mean | SD | Mean | SD | Mean | SD |
| D7 | 27.4% | 9.0% | 16.5% | 6.3% | 20.4% | 5.0% | 23.4% | 8.0% | 32.7% | 8.4% |
| D14 | 32.0% | 7.7% | 16.9% | 4.1% | 16.7% | 5.1% | 22.2% | 10.1% | 34.3% | 20.5% |
| D21 | 47.5% | 23.0% | 27.7% | 6.3% | 24.1% | 10.8% | 32.3% | 15.6% | 54.5% | 41.8% |
| D28 | 74.7% | 21.9% | 33.9% | 18.2% | 29.4% | 17.4% | 51.5% | 21.9% | ND | ND |
| | DR007908 | | DR007886 | | DR009347 | | DR009348 | | DR009349 | |
| | Mean | SD | Mean | SD | Mean | SD | Mean | SD | Mean | SD |
| D7 | 31.3% | 12.8% | 10.8% | 3.1% | 30.5% | 18.9% | 42.7% | 32.4% | 14.9% | 11.2% |
| D14 | 50.8% | 23.8% | 16.7% | 6.1% | 53.0% | 34.9% | 37.6% | 41.0% | 17.3% | 15.2% |
| D21 | ND | ND | 26.8% | 11.3% | 58.7% | 48.5% | 31.0% | 43.3% | 21.3% | 19.9% |
| D28 | ND | ND | 29.3% | 16.2% | ND | ND | ND | ND | 21.6% | 22.1% |
| | DR009350 | | DR009351 | | DR009352 | | DR009353 | | DR009355 | |
| | Mean | SD | Mean | SD | Mean | SD | Mean | SD | Mean | SD |
| D7 | 54.8% | 36.7% | 81.4% | 25.4% | 59.2% | 20.9% | 18.2% | 8.1% | 73.4% | 21.0% |
| D14 | 82.1% | 61.8% | 125.3% | 34.2% | 69.7% | 26.7% | 25.6% | 12.5% | 93.7% | 38.8% |
| D21 | ND | ND | ND | ND | 96.2% | 39.9% | 38.2% | 18.9% | ND | ND |
| D28 | ND | ND | ND | ND | ND | ND | 48.2% | 22.8% | ND | ND |
| | DR007906 | | DR007907 | | DR009211 | | DR007910 | | DR007911 | |
| | Mean | SD | Mean | SD | Mean | SD | Mean | SD | Mean | SD |
| D7 | 9.9% | 2.9% | 13.8% | 6.6% | 52.9% | 11.9% | 16.0% | 5.2% | 23.1% | 10.5% |
| D14 | 13.1% | 7.2% | 15.6% | 8.4% | 62.5% | 21.6% | 28.5% | 9.1% | 38.0% | 18.4% |
| D21 | 30.0% | 27.2% | 18.5% | 10.8% | 101.6% | 54.5% | 34.5% | 10.2% | 57.8% | 33.4% |
| D28 | 55.9% | 32.6% | 28.9% | 19.5% | ND | ND | 71.0% | 18.6% | ND | ND |
| | DR007912 | | DR009356 | | DR009357 | | DR009358 | | DR007913 | |
| | Mean | SD | Mean | SD | Mean | SD | Mean | SD | Mean | SD |
| D7 | 14.4% | 5.5% | 16.6% | 5.0% | 16.0% | 5.9% | 37.7% | 21.3% | 28.5% | 10.5% |
| D14 | 29.6% | 18.4% | 18.0% | 5.4% | 22.8% | 11.9% | 38.9% | 24.5% | 52.0% | 28.6% |
| D21 | 49.6% | 34.7% | 26.6% | 9.5% | 43.7% | 21.7% | 60.7% | 61.6% | ND | ND |
| D28 | ND | ND | 40.7% | 24.9% | 72.0% | 41.1% | ND | ND | ND | ND |
| ND indicates not detected. | | | | | | | | | | |

**Example 9 Screening of Activity in Huh7 Cell Line**

Referring to the protocol of Example 4, the Huh7 cell line was used. siRNA compounds were screened for activity in the cell line, with the concentration of siRNA compounds selected as 10 nM and 1 nM to screen the activity. The experimental results are shown in Table 14.

Table 14. Screening Results of Activity in Huh7 Cell Line

| | **10nM** | | **1nM** | |
|---|---|---|---|---|
| **siRNA No.** | **Residual inhibition** | **SD** | **Residual inhibition** | **SD** |
| DR007886 | 10.9% | 4.6% | 11.1% | 6.4% |

(continued)

| | 10nM | | 1nM | |
|---|---|---|---|---|
| siRNA No. | Residual inhibition | SD | Residual inhibition | SD |
| DR007257 | 14.2% | 1.8% | 8.6% | 3.0% |
| DR007258 | 15.9% | 1.1% | 14.7% | 1.4% |
| DR007263 | 17.9% | 1.5% | 14.3% | 1.5% |
| DR007268 | 18.0% | 3.4% | 13.9% | 2.3% |
| DR007276 | 17.5% | 0.1% | 14.9% | 0.5% |
| DR007334 | 8.9% | 0.4% | 6.9% | 1.2% |
| DR007477 | 11.5% | 1.2% | 9.5% | 0.8% |
| DR007631 | 11.0% | 1.5% | 14.2% | 2.2% |
| DR007632 | 9.9% | 0.4% | 10.2% | 0.8% |
| DR007648 | 9.1% | 0.8% | 10.5% | 0.5% |
| DR009651 | 16.6% | 4.8% | 10.2% | 0.2% |
| DR009653 | 13.0% | 3.6% | 14.0% | 0.0% |
| DR009657 | 11.5% | 1.1% | 13.9% | 0.3% |
| DR009659 | 13.2% | 0.8% | 12.5% | 1.9% |
| DR009660 | 12.3% | 1.6% | 11.4% | 1.4% |
| DR009662 | 27.4% | 13.1% | 7.0% | 1.1% |
| DR009668 | 30.0% | 6.0% | 22.3% | 1.8% |
| DR009672 | 15.5% | 0.6% | 15.0% | 2.0% |
| DR009673 | 10.2% | 0.7% | 17.3% | 2.0% |
| DR009674 | 8.0% | 1.2% | 7.6% | 1.4% |
| DR009675 | 14.0% | 1.6% | 14.8% | 0.5% |
| DR009676 | 12.5% | 3.0% | 13.2% | 0.5% |
| DR009688 | 9.6% | 1.2% | 5.4% | 2.0% |
| DR009690 | 20.9% | 0.1% | 17.0% | 1.3% |
| DR009691 | 11.9% | 5.6% | 5.9% | 1.5% |
| DR009692 | 13.3% | 0.7% | 15.3% | 1.1% |
| DR009693 | 6.3% | 0.9% | 12.3% | 1.2% |
| DR009695 | 9.2% | 1.9% | 8.8% | 0.7% |
| DR009696 | 7.9% | 0.6% | 8.4% | 1.5% |
| DR009697 | 8.9% | 0.3% | 8.8% | 0.5% |
| DR009699 | 7.5% | 0.5% | 10.1% | 0.4% |
| DR009700 | 7.7% | 0.1% | 7.0% | 1.0% |
| DR009701 | 8.8% | 0.4% | 7.3% | 0.4% |
| DR009703 | 15.9% | 3.8% | 8.6% | 4.1% |
| DR009706 | 11.3% | 0.5% | 13.0% | 1.6% |
| DR009707 | 11.3% | 3.3% | 14.3% | 0.4% |
| DR009708 | 11.4% | 3.6% | 6.1% | 0.3% |
| DR009709 | 28.0% | 10.5% | 22.1% | 0.0% |

## Claims

1. A double-stranded nucleotide (dsRNA) for inhibiting the expression of cell death-inducing DFFA-like effector B (CIDEB) in a cell, the dsRNA comprising a sense strand and an antisense strand forming a double-stranded region, wherein the sense strand and the antisense strand are each independently 15-30 nucleotides in length, and the antisense strand comprises a nucleotide sequence of at least 15 contiguous nucleotides from the nucleotide sequence set forth in any one of SEQ ID NOs: 62-122.

2. The dsRNA of claim 1, wherein the sense strand comprises a nucleotide sequence of at least 15 contiguous nucleotides from the nucleotide sequence set forth in any one of SEQ ID NOs: 1-61.

3. The dsRNA of claim 1 or 2, wherein the dsRNA is an siRNA.

4. The dsRNA of any one of claims 1-3, wherein the sense strand and the antisense strand are each independently 15-27 nucleotides, preferably 18-25 nucleotides, more preferably 19-21 nucleotides in length.

5. The dsRNA of any one of claims 1-4, wherein the antisense strand comprises a nucleotide sequence of at least 16 contiguous nucleotides, a nucleotide sequence of at least 17 contiguous nucleotides, a nucleotide sequence of at least 18 contiguous nucleotides, a nucleotide sequence of at least 19 contiguous nucleotides, or a nucleotide sequence of at least 20 contiguous nucleotides from the nucleotide sequence set forth in any one of SEQ ID NOs: 62-122, and preferably the antisense strand comprises the nucleotide sequence set forth in any one of SEQ ID NOs: 62-122.

6. The dsRNA of any one of claims 1-5, wherein the sense strand comprises at least 16 contiguous nucleotides, a nucleotide sequence of at least 17 contiguous nucleotides, or a nucleotide sequence of at least 18 contiguous nucleotides from the nucleotide sequence set forth in any one of SEQ ID NOs: 1-61, and preferably the sense strand comprises the nucleotide sequence set forth in any one of SEQ ID NOs: 1-61.

7. The dsRNA of any one of claims 1-6, wherein the siRNA comprises any pair of the paired sense strand sequences and antisense strand sequences as shown in Table 3 of the description.

8. The dsRNA of any one of claims 1-7, wherein substantially all nucleotides of the sense strand and substantially all nucleotides of the antisense strand are modified nucleotides, or all nucleotides of the sense strand and all nucleotides of the antisense strand are modified nucleotides.

9. The dsRNA of claim 8, wherein the sense strand and the antisense strand each independently comprise one or more nucleotide modifications selected from the group consisting of a 2'-O-methyl modified nucleotide, a 2'-fluoro modified nucleotide, a 2'-deoxy modified nucleotide, an SCP modified nucleotide, and a phosphorothioate internucleotide linkage modification.

10. The dsRNA of claim 8 or 9, wherein the 3' end and/or 5' end of the sense strand and/or the antisense strand comprise 1-5 phosphorothioate internucleotide bondings, preferably 2-3 phosphorothioate internucleotide bondings.

11. The dsRNA of any one of claims 1-10, wherein the antisense strand has a length of 21 nucleotides and has

    (i) 2'-O-methyl modified nucleotides at positions 1, 3, 5, 7, 9, 11, 13, 15, 17, 19, and 21, and 2'-fluoro modified nucleotides at positions 2, 4, 6, 8, 10, 12, 14, 16, 18, and 20 (counting from the 5' end); and/or
    (ii) phosphorothioate internucleotide bondings between nucleotide positions 1 and 2, between nucleotide positions 2 and 3, between nucleotide positions 19 and 20, and between nucleotide positions 20 and 21 (counting from the 5' end).

12. The dsRNA of any one of claims 1-10, wherein the antisense strand has a length of 21 nucleotides and has

    (i) 2'-O-methyl modified nucleotides at positions 1, 3, 5, 7, 9, 11, 13, 15, 17, 19, 20, and 21, and 2'-fluoro modified nucleotides at positions 2, 4, 6, 8, 10, 12, 14, 16, and 18 (counting from the 5' end); and/or
    (ii) phosphorothioate internucleotide bondings between nucleotide positions 1 and 2, between nucleotide positions 2 and 3, between nucleotide positions 19 and 20, and between nucleotide positions 20 and 21 (counting from the 5' end).

**13.** The dsRNA of any one of claims 1-10, wherein the antisense strand has a length of 21 nucleotides and has

(i) a 2'-deoxy modification at one or more nucleotide positions;
(ii) an SCP modification at one or more nucleotide positions;
(iii) a 2'-fluoro modification at one or more nucleotide positions;
(iv) a 2'-O-methyl modification at a position other than the position having the 2'-deoxy modification, SCP modification and 2'-fluoro modification defined in (i), (ii) and (iii); and/or
(v) phosphorothioate internucleotide bondings between nucleotide positions 1 and 2, between nucleotide positions 2 and 3, between nucleotide positions 19 and 20, and between nucleotide positions 20 and 21 (counting from the 5' end).

**14.** The dsRNA of claim 13, wherein the antisense strand has a length of 21 nucleotides and has

(i) 2'-deoxy modifications at positions 2, 5, 7, and 12 (counting from the 5' end);
(ii) an SCP modification at position 1 (counting from the 5' end);
(iii) a 2'-fluoro modification at position 14 (counting from the 5' end);
(iv) 2'-O-methyl modifications at positions 3, 4, 6, 8, 9, 10, 11, 13, 15, 16, 17, 18, 19, 20, and 21 (counting from the 5' end); and/or
(v) phosphorothioate internucleotide bondings between nucleotide positions 1 and 2, between nucleotide positions 2 and 3, between nucleotide positions 19 and 20, and between nucleotide positions 20 and 21 (counting from the 5' end).

**15.** The dsRNA of any one of claims 1-10, wherein the antisense strand has a length of 23 nucleotides and has

(i) an SCP modification at position 1 (counting from the 5' end);
(ii) 2'-fluoro modifications at positions 2, 4, 6, 8, 10, 12, 14, 16, and 18 (counting from the 5' end);
(iii) 2'-O-methyl modifications at positions 3, 5, 7, 9, 11, 13, 15, 17, 19, 20, 21, 22, and 23 (counting from the 5' end); and/or
(iv) phosphorothioate internucleotide bondings between nucleotide positions 1 and 2, between nucleotide positions 2 and 3, between nucleotide positions 21 and 22, and between nucleotide positions 22 and 23 (counting from the 5' end).

**16.** The dsRNA of any one of claims 1-15, wherein the sense strand has a length of 19 nucleotides and has:

(i) 2'-O-methyl modified nucleotides at positions 1 to 6, 10 to 19, and 2'-fluoro modified nucleotides at positions 7 to 9 (counting from the 5' end); and/or
(ii) phosphorothioate internucleotide bondings between nucleotide positions 1 and 2 and between nucleotide positions 2 and 3 (counting from the 5' end).

**17.** The dsRNA of any one of claims 1-15, wherein the sense strand has a length of 19 nucleotides and has:

(i) 2'-O-methyl modified nucleotides at positions 1 to 6, 10 to 19, and 2'-fluoro modified nucleotides at positions 7 to 9 (counting from the 5' end); and/or
(ii) phosphorothioate internucleotide bondings between nucleotide positions 1 and 2, between nucleotide positions 2 and 3, between positions 17 and 18, and between positions 18 and 19 (counting from the 5' end).

**18.** The dsRNA of any one of claims 1-15, wherein the sense strand has a length of 21 nucleotides and has

(i) 2'-fluoro modifications at positions 9, 10, and 11 (counting from the 5' end);
(ii) 2'-O-methyl modifications at positions 1, 2, 3, 4, 5, 6, 7, 8, 12, 13, 14, 15, 16, 17, 18, 19, 20, and 21 (counting from the 5' end); and/or
(iii) phosphorothioate internucleotide bondings between nucleotide positions 1 and 2, between nucleotide positions 2 and 3, and between nucleotide positions 20 and 21 (counting from the 5' end).

**19.** The dsRNA of any one of claims 1-18, wherein the dsRNA is further conjugated to a ligand moiety comprising N-acetylgalactosamine, and preferably the sense strand is conjugated to the ligand moiety.

**20.** The dsRNA of claim 19, wherein the sense strand of the dsRNA comprises a phosphorothioate internucleotide

bonding between nucleotide positions 1 and 2 (counting from the 3' end), and is conjugated to the ligand moiety at the 3' end of the sense strand via a phosphorothioate.

**21.** The dsRNA of claim 19 or 20, wherein the ligand has the following structure:

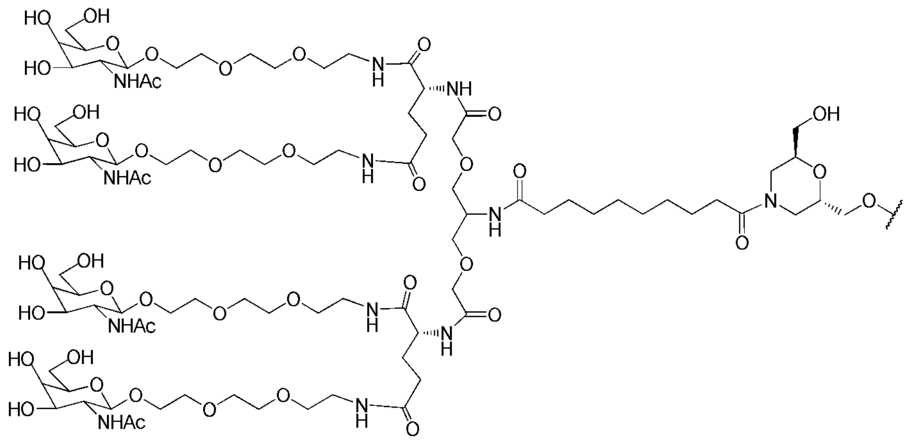

wherein

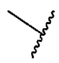

represents the point of attachment to the sense strand (preferably the 3' end of the sense strand) of the dsRNA via a phosphate group or a phosphorothioate group.

**22.** The dsRNA of any one of claims 1-21, wherein the antisense strand comprises any one of the modified nucleotide sequences shown in Table 5 of the description, and/or the sense strand comprises any one of the modified nucleotide sequences shown in Table 4 of the description.

**23.** The dsRNA of any one of claims 1-22, wherein the dsRNA comprises any pair of the paired modified sense strand sequences and modified antisense strand sequences shown in Table 6 of the description.

**24.** The dsRNA of claim 23, wherein:

(a) the sense strand comprises UmsAmsCmUmCmAmGfGfUfCmAmGmUmAmUmCmUmAmsAms-GL6 (SEQ ID NO: 149), and the antisense strand comprises UmsUfsAmGfAmUfAmCfUmGfAmCfCmUfGmAfGmUfAmsAfsGm (SEQ ID NO: 259);
(b) the sense strand comprises CmsAmsCmCmAmUmGfGfAfGmUmAmCmCmUmCmUmCmsAms-GL6 (SEQ ID NO: 150), and the antisense strand comprises UmsGfsAmGfAmGfGmUfAmCfUmCfCmAfUmGfGmUfGmsGfsAm (SEQ ID NO: 257);
(c) the sense strand comprises AmsGmsGmUmCmAmGfUfAfUmCmUmAmAmUmAmUmAmsAms-GL6 (SEQ ID NO: 151), and the antisense strand comprises UmsUfsAmUfAmUfUmAfGmAfUmAfCmUfGmAfCmCfUmsGfsAm (SEQ ID NO: 260);
(d) the sense strand comprises CmsUmsCmUmAmUmGfAfGfUmUmGmUmGmAmCmUmUmsUms-GL6 (SEQ ID NO: 152), and the antisense strand comprises AmsAfsAmGfUmCfAmCfAmAfCmUfCmAfUmAfGmAfGmsUfsAm (SEQ ID NO: 263);
(e) the sense strand comprises AmsGmsAmCmUmAmUfGfAfCmAmGmCmAmUmCmAmAmsAms-GL6 (SEQ ID NO: 153), and the antisense strand comprises UmsUfsUmGfAmUfGmCfUmGfUmCfAmUfAmGfUmCfUmsUfsUm (SEQ ID NO: 267);
(f) the sense strand comprises GmsAmsCmUmAmUmGfAfCfAmGmCmAmUmCmAmAmAmsUms-GL6 (SEQ ID NO: 154), and the antisense strand comprises AmsUfsUmUfGmAfUmGfCmUfGmUfCmAfUmAfGmUfCmsUfsUm (SEQ ID NO: 268);
(g) the sense strand comprises AmsCmsUmAmUmGmAfCfAfGmCmAmUmCmAmAmAmUmsUms-GL6 (SEQ ID NO: 155), and the antisense strand comprises AmsAfsUmUfUmGfAmUfGmCfUmGfUmCfAmUfAmGfUmsCfsUm (SEQ ID NO: 269);

(h) the sense strand comprises CmsUmsAmUmGmAmCfAfGfCmAmUmCmAmAmAmUmUmsUms-GL6 (SEQ ID NO: 156), and the antisense strand comprises AmsAfsAmUfUmUfGmAfUmGfCmUfGmUfCmAfUmAfGmsUfsCm (SEQ ID NO: 270);

(i) the sense strand comprises AmsUmsGmAmCmAmGfCfAfUmCmAmAmAmUmUmUmCmsAms-GL6 (SEQ ID NO: 157), and the antisense strand comprises UmsGfsAmAfAmUfUmUfGmAfUmGfCmUfGmUfCmAfUmsAfsGm (SEQ ID NO: 271);

(j) the sense strand comprises UmsGmsAmCmAmGmCfAfUfCmAmAmAmUmUmUmCmAmsAms-GL6 (SEQ ID NO: 158), and the antisense strand comprises UmsUfsGmAfAmAfUmUfUmGfAmUfGmCfUmGfUmCfAmsUfsAm (SEQ ID NO: 272);

(k) the sense strand comprises CmsAmsGmAmCmAmGfUfAfCmAmGmGmCmUmAmGmAmsUms-GL6 (SEQ ID NO: 159), and the antisense strand comprises AmsUfsCmUfAmGfCmCfUmGfUmAfCmUfGmUfCmUfGmsCfsAm (SEQ ID NO: 273);

(l) the sense strand comprises AmsGmsAmCmAmGmUfAfCfAmGmGmCmUmAmGmAmUmsAms-GL6 (SEQ ID NO: 160), and the antisense strand comprises UmsAfsUmCfUmAfGmCfCmUfGmUfAmCfUmGfUmCfUmsGfsCm (SEQ ID NO: 274);

(m) the sense strand comprises GmsAmsCmAmGmUmAfCfAfGmGmCmUmAmGmAmUmAmsAms-GL6 (SEQ ID NO: 161), and the antisense strand comprises UmsUfsAmUfCmUfAmGfCmCfUmGfUmAfCmUfGmUfCmsUfsGm (SEQ ID NO: 275);

(n) the sense strand comprises AmsAmsAmCmAmUmUfUfCfCmAmAmUmAmAmAmAmAmsUms-GL6 (SEQ ID NO: 162), and the antisense strand comprises AmsUfsUmUfUmUfAmUfUmGfGmAfAmAfUmGfUmUfUmsUfsUm (SEQ ID NO: 276);

(o) the sense strand comprises AmsCmsAmUmUmUmCfCfAfAmUmAmAmAmAmAmUmAmsUms-GL6 (SEQ ID NO: 163), and the antisense strand comprises AmsUfsAmUfUmUfUmUfAmUfUmGfGmAfAmAfUmGfUmsUfsUm (SEQ ID NO: 277);

(p) the sense strand comprises CmsCmsCmUmAmAmAfCfUfCmCmCmCmAmGmCmAmUmsAms-GL6 (SEQ ID NO: 164), and the antisense strand comprises UmsAfsUmGfCmUfGmGfGmGfAmGfUmUfUmAfGmGfGmsAfsCm (SEQ ID NO: 281);

(q) the sense strand comprises UmsAmsCmUmCmAmGfGfUfCmAmGmUmAmUmCmUmAmsAms-GL6 (SEQ ID NO: 149); and the antisense strand comprises (SCP-U)sdTsAmGmdAUmdACmUmGmAmdCCmUfGmAmGmUmAmsAmsGm (SEQ ID NO: 283);

(r) the sense strand comprises AmsGmsGmUmCmAmGfUfAfUmCmUmAmAmUmAmUmAmsAms-GL6 (SEQ ID NO: 151), and the antisense strand comprises (SCP-U)sdTsAmUmdAUmdTAmGmAmUmdACmUfGmAmCmCmUmsGmsAm (SEQ ID NO: 284);

(s) the sense strand comprises AmsGmsAmCmUmAmUfGfAfCmAmGmCmAmUmCmAmAmsAms-GL6 (SEQ ID NO: 153), and the antisense strand comprises (SCP-U)sdTsUmGmdAUmdGCmUmGmUmdCAmUfAmGmUmCmUmsUmsUm (SEQ ID NO: 285);

(t) the sense strand comprises GmsAmsCmUmAmUmGfAfCfAmGmCmAmUmCmAmAmAmsAms-GL6 (SEQ ID NO: 165), and the antisense strand comprises (SCP-U)sdTsUmUmdGAmdTGmCmUmGmdTCmAfUmAmGmUmCmsUmsUm (SEQ ID NO: 286);

(u) the sense strand comprises UmsGmsAmCmAmGmCfAfUfCmAmAmAmUmUmUmCmAmsAms-GL6 (SEQ ID NO: 158), and the antisense strand comprises (SCP-U)sdTsGmAmdAAmdTUmUmGmAmdTGmCfUmGmUmCmAmsUmsAm (SEQ ID NO: 288);

(v) the sense strand comprises AmsGmsAmCmAmGmUfAfCfAmGmGmCmUmAmGmAmUmsAms-GL6 (SEQ ID NO: 160), and the antisense strand comprises (SCP-U)sdAsUmCmdTAmdGCmCmUmGmdTAmCfUmGmUmCmUmsGmsCm (SEQ ID NO: 289);

(w) the sense strand comprises GmsAmsCmAmGmUmAfCfAfGmGmCmUmAmGmAmUmAmsAms-GL6 (SEQ ID NO: 161), and the antisense strand comprises (SCP-U)sdTsAmUmdCUmdAGmCmCmUmdGUmAfCmUmGmUmCmsUmsGm (SEQ ID NO: 290);

(x) the sense strand comprises AmsAmsAmCmAmUmUfUfCfCmAmAmUmAmAmAmAmAmsAms-GL6 (SEQ ID NO: 166), and the antisense strand comprises (SCP-U)sdTsUmUmdTUmdAUmUmGmGmdAAmAfUmGmUmUmUmsUmsUm (SEQ ID NO: 291);

(y) the sense strand comprises AmsCmsAmUmUmUmCfCfAfAmUmAmAmAmAmAmUmAmsAms-GL6 (SEQ ID NO: 167), and the antisense strand comprises (SCP-U)sdTsAmUmdTUmdTUmAmUmUmdGGmAfAmAmUmGmUmsUmsUm (SEQ ID NO: 292);

(z) the sense strand comprises GmsCmsUmCmUmGmAfAfCfCmCmCmAmGmUmGmAmCmsAms-GL6 (SEQ ID NO: 168), and the antisense strand comprises (SCP-U)sdGsUmCmdACmdTGmGmGmGmdTUmCfAmGmAmGmCmsUmsGm (SEQ ID NO: 293);

(ab) the sense strand comprises GmsCmsCmAmGmGmAfGfCfUmGmCmUmAmGmCmCmAmsAms-GL6 (SEQ ID NO: 169), and the antisense strand comprises (SCP-U)sdTsGmGmdCUmdAGmCmAmGmdCUmCfCmUmGmGmCmsUmsGm (SEQ ID NO: 294);
(ac) the sense strand comprises CmsCmsUmUmUmGmAfCfGfUmGmUmAmCmAmAmGmCmsAms-GL6 (SEQ ID NO: 170), and the antisense strand comprises (SCP-U)sdGsCmUmdTGmdTAmCmAmCmdGUmCfAmAmAmGmGmsUmsGm (SEQ ID NO: 295);
(ad) the sense strand comprises GmsCmsCmAmUmAmUfGfUfUmGmCmUmGmGmGmAmAmsAms-GL6 (SEQ ID NO: 171), and the antisense strand comprises (SCP-U)sdTsUmCmdCCmdAGmCmAmAmdCAmUfAmUmGmGmCmsUmsCm (SEQ ID NO: 296);
(ae) the sense strand comprises CmsCmsGmCmCmUmCfCfAfUmUmCmCmUmAmCmUmAmsAms-GL6 (SEQ ID NO: 172), and the antisense strand comprises (SCP-U)sdTsAmGmdTAmdGGmAmAmUmdGGmAfGmGmCmGmGmsUmsCm (SEQ ID NO: 297);
(af) the sense strand comprises GmsCmsAmAmAmGmAfCfUfAmUmGmAmCmAmGmCmAmsAms-GL6 (SEQ ID NO: 173), and the antisense strand comprises (SCP-U)sdTsGmCmdTGmdTCmAmUmAmdGUmCfUmUmUmGmCmsAmsGm (SEQ ID NO: 298);
(ag) the sense strand comprises CmsUmsCmUmGmAmAfCfCfCmCmAmGmUmGmAmCmUmsAms-GL6 (SEQ ID NO: 174), and the antisense strand comprises (SCP-U)sdAsGmUmdCAmdCUmGmGmGmdGUmUfCmAmGmAmGmsCmsUm (SEQ ID NO: 299);
(ah) the sense strand comprises CmsCmsUmCmAmCmAfUfCfCmCmAmAmGmUmCmUmAmsAms-GL6 (SEQ ID NO: 176), and the antisense strand comprises (SCP-U)sdTsAmGmdACmdTUmGmGmGmdAUmGfUmGmAmGmGmsCmsGm (SEQ ID NO: 301);
(ai) the sense strand comprises AmsUmsUmUmCmCmAfAfUfAmAmAmAmAmUmAmUmCmsAms-GL6 (SEQ ID NO: 177), and the antisense strand comprises (SCP-U)sdGsAmUmdAUmdTUmUmUmAmdTUmGfGmAmAmAmUmsGmsUm (SEQ ID NO: 302);
(aj) the sense strand comprises AmsAmsAmAmAmCmAmUmUfUfCfCmAmAmUmAmAmAmAmAmsAms-GL6 (SEQ ID NO: 178), and the antisense strand comprises (SCP-U)sUfsUmUfUmUfAmUfUmGfGmAfAmAfUmGfUmUfUmUmUmsGmsUm (SEQ ID NO: 303);
(ak) the sense strand comprises AmsAmsAmCmAmUmUmUmCfCfAfAmUmAmAmAmAmAmUmAmsAms-GL6 (SEQ ID NO: 179), and the antisense strand comprises (SCP-U)sUfsAmUfUmUfUmUfAmUfUmGfGmAfAmAfUmGfUmUmUmsUmsUm (SEQ ID NO: 304),

wherein GL6 is

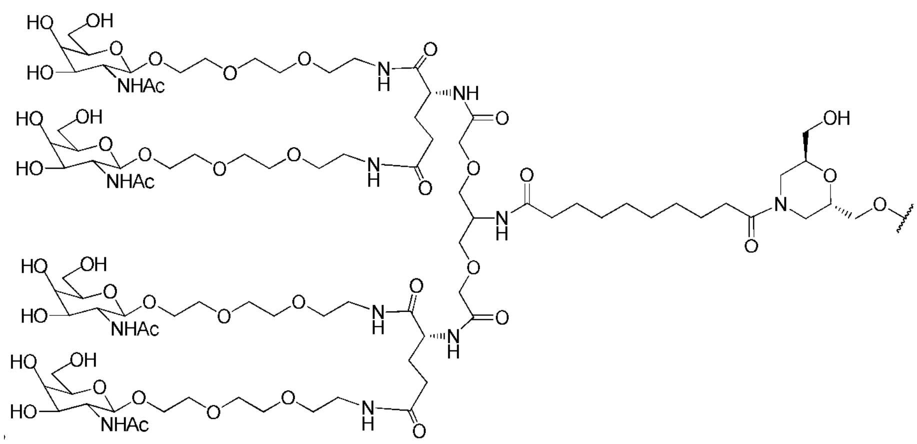

wherein

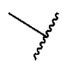

represents the point of attachment to the 3' end of the sense strand of the dsRNA via a phosphate group or a phosphorothioate group.

**25.** A cell comprising the dsRNA of any one of claims 1-24.

**26.** A pharmaceutical composition comprising the dsRNA of any one of claims 1-24, or the cell of claim 25, and optionally a pharmaceutically acceptable carrier or excipient.

**27.** A kit comprising the dsRNA of any one of claims 1-24, the cell of claim 25, or the pharmaceutical composition of claim 26.

**28.** A method of treating a disease or disorder benefiting from reduced expression of cell death-inducing DFFA-like effector B (CIDEB) in a subject, comprising a step of administering to the subject the dsRNA of any one of claims 1-24, the cell of claim 25, or the pharmaceutical composition of claim 26.

**29.** A method of preventing at least one symptom in a subject having a disease or disorder benefiting from reduced expression of cell death-inducing DFFA-like effector B (CIDEB), comprising a step of administering to the subject the dsRNA of any one of claims 1-24, the cell of claim 25, or the pharmaceutical composition of claim 26.

**30.** The method of claim 28 or 29, wherein the disease or disorder benefiting from reduced cell death-inducing DFFA-like effector B (CIDEB) expression is a CIDEB-mediated or CIDEB-related disease, preferably, the CIDEB-mediated or CIDEB-related disease is selected from liver diseases (e.g. fatty liver, hepatitis (e.g. steatohepatitis, non-alcoholic steatohepatitis and viral hepatitis), non-alcoholic fatty liver disease, alcoholic fatty liver disease, hepatic fibrosis, cirrhosis and liver failure), cholangitis (primary biliary cholangitis, primary sclerosing cholangitis), autoimmune diseases, endocrine disorders, urinary diseases, metabolic diseases (e.g. metabolic syndrome), hepatobiliary diseases, fibrotic diseases, cardiovascular diseases (e.g. hypertension, endothelial cell dysfunction, arteriosclerosis, atherosclerosis, coronary artery disease, myocardial infarction, ischemic stroke, and other forms of heart diseases), immunoinflammatory diseases, central nervous diseases, gastrointestinal diseases, hyperproliferative diseases (e.g. cancers), dyslipidemia (e.g. hyperlipidemia, hypercholesterolemia, hypertriglyceridemia, high LDL cholesterol, low HDL cholesterol, and postprandial hypertriglyceridemia), glycemic control disorders (e.g. insulin resistance, type 2 diabetes), adipocyte dysfunction, visceral fat deposition, obesity, eating disorders, and excessive sugar cravings, more preferably, the CIDEB-mediated or CIDEB-related disease is selected from liver diseases (e.g. fatty liver, hepatitis (e.g. steatohepatitis, non-alcoholic steatohepatitis, alcoholic steatohepatitis, and viral hepatitis), non-alcoholic fatty liver disease, alcoholic fatty liver disease, hepatic fibrosis, cirrhosis, and liver failure), cholangitis (e.g. primary biliary cholangitis, primary sclerosing cholangitis), metabolic diseases (e.g. metabolic syndrome), cardiovascular diseases (e.g. hypertension, endothelial cell dysfunction, arteriosclerosis, atherosclerosis, coronary artery disease, myocardial infarction, ischemic stroke, and other forms of cardiac diseases), and dyslipidemia (e.g. hyperlipidemia, hypercholesterolemia, hypertriglyceridemia, high LDL cholesterol, low HDL cholesterol, and postprandial hypertriglyceridemia).

**31.** A method of reducing the level of cell death-inducing DFFA-like effector B (CIDEB) in a subject, comprising a step of administering to the subject the dsRNA of any one of claims 1-24, the cell of claim 25, or the pharmaceutical composition of claim 26.

**INTERNATIONAL SEARCH REPORT**

International application No.
**PCT/CN2024/086382**

**A. CLASSIFICATION OF SUBJECT MATTER**

C12N 15/11(2006.01)i; A61K 31/713(2006.01)i

According to International Patent Classification (IPC) or to both national classification and IPC

**B. FIELDS SEARCHED**

Minimum documentation searched (classification system followed by classification symbols)

IPC: C12N; A61K

Documentation searched other than minimum documentation to the extent that such documents are included in the fields searched

Electronic data base consulted during the international search (name of data base and, where practicable, search terms used)

CJFD; CNTXT; DWPI; VEN; CNABS; USTXT; WOTXT; PUBMED; ISI Web of knowledge; baidu; CNKI; NCBI; 中国专利生物序列检索, China Patent Biological Sequence Search; STN: dsRNA, CIDEB, 细胞死亡诱导DFFA样效应子B, 抑制, 修饰, cell death inducing dffa like effector, SEQ ID NOs: 1-122, siRNA, inhibition, modification

**C. DOCUMENTS CONSIDERED TO BE RELEVANT**

| Category* | Citation of document, with indication, where appropriate, of the relevant passages | Relevant to claim No. |
|---|---|---|
| X | WO 2023034837 A2 (ALNYLAM PHARMACEUTICALS, INC.) 09 March 2023 (2023-03-09)<br>claims 1-117, and tables 3-6 | 1-31 |
| A | WO 2022140624 A1 (REGENERON PHARMACEUTICALS, INC.) 30 June 2022 (2022-06-30)<br>entire document | 1-31 |
| A | VERWEIJ, N. et al. "Germline Mutations in CIDEB and Protection against Liver Disease" *The New England Journal of Medicine*, Vol. vol. 387, 28 July 2022 (2022-07-28),<br>pp. 332-344 | 1-31 |
| A | SU, L. et al. "Cideb Controls Sterol-Regulated ER Export of SREBP/ SCAP by Promoting Cargo Loading at ER Exit Sites" *The EMBO Journal*, Vol. vol. 38, 11 March 2019 (2019-03-11),<br>Document number: e100156, pages 1-19 | 1-31 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 July 2024** | **12 July 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/086382**

**C. DOCUMENTS CONSIDERED TO BE RELEVANT**

| Category* | Citation of document, with indication, where appropriate, of the relevant passages | Relevant to claim No. |
|---|---|---|
| A | SINGARAVELU, R. et al. "Human Serum Activates CIDEB-Mediated Lipid Droplet Enlargement in Hepatoma Cells" *Biochemical and Biophysical Research Communications,* Vol. vol. 441, 24 October 2013 (2013-10-24), pp. 447-452 | 1-31 |
| A | LI, J.Z. et al. "Cideb Regulates Diet-Induced Obesity, Liver Steatosis, and Insulin Sensitivity by Controlling Lipogenesis and Fatty Acid Oxidation" *Diabetes*, Vol. vol. 56, 07 August 2007 (2007-08-07), pp. 2523-2532 | 1-31 |

Form PCT/ISA/210 (second sheet) (July 2022)

**INTERNATIONAL SEARCH REPORT**

International application No.
**PCT/CN2024/086382**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/086382**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **28-31**
because they relate to subject matter not required to be searched by this Authority, namely:

Claims 28-31 relate to a method for preventing or treating a disease or condition, which belongs to a disease treatment method as defined in PCT Rule 39.1(iv). However, a search is still performed on the basis of the pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/086382**

| Patent document cited in search report | Publication date (day/month/year) | Patent family member(s) | Publication date (day/month/year) |
|---|---|---|---|
| WO 2023034837 A2 | 09 March 2023 | CA 3230404 A1 | 09 March 2023 |
| | | KR 20240056561 A | 30 April 2024 |
| | | AU 2022339821 A1 | 07 March 2024 |
| | | TW 202325311 A | 01 July 2023 |
| | | IL 311030 A | 01 April 2024 |
| | | WO 2023034837 A3 | 06 April 2023 |
| WO 2022140624 A1 | 30 June 2022 | EP 4267739 A1 | 01 November 2023 |
| | | JP 2024502282 A | 18 January 2024 |
| | | IL 303536 A | 01 August 2023 |
| | | MX 2023007587 A | 21 September 2023 |
| | | US 2022220474 A1 | 14 July 2022 |
| | | KR 20230124915 A | 28 August 2023 |
| | | US 2022282253 A1 | 08 September 2022 |
| | | US 11773393 B2 | 03 October 2023 |
| | | CA 3201624 A1 | 30 June 2022 |
| | | AU 2021410768 A1 | 22 June 2023 |

Form PCT/ISA/210 (patent family annex) (July 2022)

## REFERENCES CITED IN THE DESCRIPTION

*This list of references cited by the applicant is for the reader’s convenience only. It does not form part of the European patent document. Even though great care has been taken in compiling the references, errors or omissions cannot be excluded and the EPO disclaims all liability in this regard.*

### Patent documents cited in the description


- WO 2011139702 A
- WO 2013033230 A
- WO 2019105419 A
- WO 2009073809 A
- WO 2009082607 A
- WO 2016081444 A


### Non-patent literature cited in the description


- **LI, J.Z. et al.** *Diabetes.*, 2007, vol. 56, 2523-2532
- **YE et al.** *Cell Metab.*, 2009, vol. 9, 177-190
- **YE et al.** *Cell Metab.*, 2009, vol. 9, 177-190
- **LI J.W. et al.** *Biochim. Biophys. Acta*, 2010, vol. 1801, 577-586
- **FRIEDMAN SL et al.** *Nat Med*, 2018, vol. 24, 908-22